# EUROPÄISCHE PATENTSCHRIFT

(11) **EP 0 775 750 B1**
(45) Veröffentlichungstag und Bekanntmachung des Hinweises auf die Patenterteilung: **13.06.2007**
(21) Anmeldenummer: 96890171.0
(22) Anmeldetag: 19.11.1996
(51) Int. Cl.: C12N 15/62, C12N 9/64, C07K 19/00, C12N 5/10, C07K 14/755, C07K 14/16, C07K 14/765, A61K 38/37, A61K 38/48, A61K 38/38

(54) **Herstellung von Proteinen aus Pro-Proteinen durch Fusionsproteine abgeleitet von Furin oder Furinanalogen**
Preparation of proteins from pro-proteins by fusion proteins derived from furin or furin analogs
Préparation de protéines à partir de pro-protéines avec des protéines de fusion dérivés de furin ou d'analogues de furin

(30) Priorität: 24.11.1995 AT 192895
(43) Veröffentlichungstag der Anmeldung: 28.05.1997
(73) Patentinhaber: Baxter Aktiengesellschaft, 1221 Wien (AT)
(72) Erfinder: Schlokat, Uwe, Dr., 2304 Orth/Donau (AT); Fischer, Bernhard, Doz., 1160 Wien (AT); Falkner, Falko-Günter, Dr., 2304 Orth/Donau (AT); Dorner, Friedrich, Prof., 1230 Wien (AT); Eibl, Johann, Dr., 1180 Wien (AT)
(74) Vertreter: Alge, Daniel

(56) Entgegenhaltungen:
- EP-A- 0 282 042
- EP-A- 0 416 890
- EP-A- 0 565 511
- WO-A-91/06314
- WO-A-92/09698
- MOLLOY SET AL: "Intracellular trafficking and activation of the furin proprotein convertase: localization of the TGN and recycling from the cell surface" EMBO JOURNAL, Bd. 13, 1994, Seiten 18-33, XP002091125
- DUIJNHOVEN H VAN: "Development and characterization of a panel of monoclonal antibodies against the novel subtilisin-like proprotein processing enzyme furin" HYBRIDOMA, Bd. 11, Nr. 1, 1992, Seiten 71-86, XP002091126
- FISCHER B E ET AL: "Structural analysis of recombinant von Willebrand factor produced at industrial scale fermentation of transformed CHO cells co-expressing recombinant furin." FEBS LETTERS, (1995 NOV 20) 375 (3) 259-62. JOURNAL CODE: EUH. ISSN: 0014-5793., XP002091127 Netherlands
- JANKNECHT R ET AL: "Rapid and efficient purification of native histidine-tagged protein expressed by recombinant vaccinia virus" PROCEEDINGS OF THE NATIONAL ACADEMY OF SCIENCES OF USA, Bd. 88, 1991, Seiten 8972-8976, XP002091128 WASHINGTON US

## Beschreibung

Die Erfindung betrifft ein neues Fusionsprotein, abgeleitet von Furin oder einem Furinanalogon, sowie ein Verfahren zur Herstellung von Proteinen aus Pro-Proteinen durch das Fusionsprotein, insbesondere von von Willebrand-Faktor aus pro-von Willebrand-Faktor.

Furin, auch PACE genannt, gehört neben PACE4, PC1/PC3, PC2, PC4 und PC5/PC6 zur Gruppe der Subtilisin-ähnlichen Serin-Proteasen, die eine wichtige Rolle bei der Spaltung von Pro-Proteinen, speziell im sekretorischen Syntheseweg, spielen (Van de Ven et al., Crit. Rev. Oncogen., 4:115-136, 1993). Pro-Proteine werden posttranslatorisch, intrazellulär im Golgi-Apparat durch die endogene Protease in ihre reife Form prozessiert. Die Protease-Spaltstelle weist eine Erkennungssequenz auf, die durch die Aminosäuresequenz Arg-X-Lys/Arg-Arg gekennzeichnet ist. Die Protease Furin spaltet Pro-Proteine spezifisch nach dieser Konsensus-Sequenz (Hosaka et al., J.Biol.Chem. 266:12127-12130, 1991).

Die DNA- und Aminosäuresequenz des humanen und des murinen Furins, sowie weitere Proteine mit Subtilisin-ähnlicher Proteasefunktion sind aufgeklärt (Roebroek et al., Mol. Biol. Rep. 11: 117-125, 1986, Roebroek et al., EMBO J. 5:2197-2202, 1986, Barr et al., DNA Cell Biol. 10:319-328,1991, Van den Ouweland et al., Nucleic Acids Res. 17:7101-7102, 1989, Van den Ouweland et al., Nucleic Acids Res. 18:664, 1990, Smeekens et al. 1990, J. Biol. Chem. 265:2997-3000; Smeekens et al 1991, Proc. Natl. Acad. Sci. USA. 88; 340-344; Kiefer et al 1991, DNA Cell. Bio. 10: 757; Nakayama et al 1992, J. Bio. Chem. 267:5897-5900, Hatsuzawa et al. 1990. J. Biol. Chem. 265:22075-22078). Das humane fur-Gen kodiert für ein Protein bestehend aus 794 Aminosäuren, wobei einzelnen, charakteristischen Bereichen bestimmte Funktionen zugeordnet werden können: ein katalytisches Zentrum, eine Mitteldomäne, eine Cystein-reiche Region, eine transmembrane und eine cytoplasmatische Domäne (Van de Ven et al., Crit. Rev. Oncogen., 4:115-136, 1993).

Intaktes Furin wird in das Membransystem des Golgi-Apparates eingebaut und ist dort funktionell aktiv (Bresnahan et al., J. Cell Biol. 111:2851-2859, 1990). Eine trunkierte Form des überexprimierten nativen Furins von 75-80 kD konnte im Zellüberstand als sezerniertes Protein detektiert werden (Wise et al., Proc. Natl. Acad. Sci. USA 87:9373-9332, 1990). Dieses natürlich sekretierte Furin ist als "shed furin" bekannt (Vidricaire et al., Biochem. Biophys. Res. Comm. 195:1011-1018, 1993) und wird N-terminal des transmembranen Teils gespalten (Vey et al. J. Cell Biol. 127:1829-1842, 1994).

Gentechnisch verkürztes Furin, bei dem der kodierende Teil der transmembranen und cytoplasmatischen Domäne deletiert ist, kann ebenfalls exprimiert und entsprechend sezerniert werden. Solche N-terminalen Deletionen wurden für Aminosäuren Δ714-794 (Leduc et al. J. Biol. Chem. 267:14304-14308, 1992, Molloy et al. J. Biol. Chem. 267:16396-16402, 1992) und für Aminosäuren Δ716-794 ("Sol-PACE") erstellt (Wasley et al. 1993. J. Biol. Chem. 268: 8458-8465, Rehemtulla et al. Blood 79:2349-2355, 1992) und für Aminosäure Δ705-794 (Hatsuzawa et al. 1992. J. Biol. Chem. 267: 16094-16099) beschrieben.

Furinmutanten, die zusätzlich eine Deletion der cysteinreichen Region aufweisen wurden ebenfalls beschrieben (Hatsuzawa et al. 1992. J. Biochem. 101:296-301, Creemers et al. 1993. J. Biol. Chem. 268:21826-21834).

Die endoproteolytische Aktivität von Furin und seine Selektivität für basische Aminosäuren wurde erstmals in Experimenten mit pro-von Willebrand-Faktor (pro-vWF) festgestellt. Pro-vWF besteht aus einem Propolypeptid mit 741 Aminosäuren und maturem von Willebrand-Faktor (vWF) mit 2050 Aminosäuren (Verweij et al., EMBO J. 5:1839-1847, 1986). Die Freisetzung von maturem vWF aus pro-vWF resultiert aus einer proteolytischen Spaltung nach Arg763. Transfektion von pro-vWF cDNA in eukaryotischen Expressionsvektoren führt zur Produktion von äquimolaren Mengen des 360 kD pro-vWF und des 260 kD reifen vWF im Zellkulturüberstand. vWF wird in seine reife Form in transfizierten Zellen vermutlich durch endogen vorkommendes Furin prozessiert (Wise et al., Proc. Natl. Acad. Sci. USA 87:9378-9382, 1990, Van de Ven et al., Mol. Biol. Rep. 14:265-275, 1990).

Zu den weiteren Pro-Proteinen, die von Furin, bzw. von Subtilisin-ähnlichen Enzymen gespalten werden, gehören eine Reihe von Hormonen und Wachstumsfaktoren (z.B. Proaktivin A, Hepatozyten-Wachstumsfaktor), Plasmaproteinen (Albumin, Faktor VII, Faktor IX, Faktor X), Rezeptoren (Insulin-Prorezeptor), viralen Proteinen (z. B. HIV-1 gp160, Influenza Virus Hämagglutinin) sowie bakteriellen Proteinen (Diphterie-Toxin, Anthrax-Toxin) (Decroly et al., J. Biol. Chem. 269:12240-12247, 1994, Stieneke-Gröber et al., EMBO J. 11:2407-2414, 1992, Barr, Cell 66:1-3, 1991, Wasley et al., J. Biol. Chem. 268:8458-8465, 1993, Klimpel et al., Proc. Natl. Acad. Sci. USA 89:10277-10281, 1992, Tsuneoka et al., J. Biol. Chem. 268:26461-26465, 1993, Bresnahan et al., J. Cell Biol. 111:2851-2859, 1990, Hosaka et al., J. Biol. Chem. 266:12127-12130, 1991, Vey et al. J. Cell. Biol. 127: 1829-1842, 1994).

Durch Koexpression der für intaktes Furin und für ein Pro-Protein kodierenden Nukleinsäuresequenzen in eukaryotischen Zellkulturen wurde eine erhöhte Prozessierung der Pro-Proteine in vivo erreicht. Dies wurde zum Beispiel für pro-Faktor IX (Wasley et al., J. Biol. Chem. 268:8458-8465, 1993) und pro-vWF (WO 91/06314, Van de Ven et al. Mol. Bio. Rep. 14:265-275, 1990, Rehemtulla et al., Blood 79:2349-2355, 1992) gezeigt.

Neben der Koexpression von intaktem Furin mit Pro-Proteinen gibt es ebenfalls Ansätze, trunkiertes Furin mit Pro-Proteinen gemeinsam zu exprimieren. Deletiertes Furin ist bei Koexpression in vivo enzymatisch aktiv und wird sezerniert; die enzymatische Aktivität solcher Deletionsmutanten konnte unter anderem bei der Prozessierung von pro-Faktor IX (Wasley et al., J. Biol. Chem. 268:8458-8465, 1993) und pro-vWF (Rehemtulla et al., Blood 79: 2349-2355, 1992) nachgewiesen werden. Koexpressionsexperimente mit Furin-Deletionsmutanten zeigten, daß der transmembrane und der cytoplasmatische Teil des Proteins für die katalytische Funktion nicht wesentlich sind (Rehemtulla et al., Proc. Natl. Acad. Sci. USA 89: 8235-8239, 1992).

WO 91/06314 offenbart die rekombinante Expression von Furin in prokaryotischen und eukaryotischen Zellen, die Herstellung von Furin-Fusionsproteinen, -Deletionsmutanten und -Fragmenten, die Reinigung von rekombinant hergestelltem Furin, sowie die mögliche Verwendung von gereinigtem Furin für die Prozessierung von Pro-Proteinen in vitro im allgemeinen.

WO 92/09698 beschreibt die Expression von PACE (Furin), die Koexpression mit inaktiven Vorstufen von Proteinen wie z.B. pro-vWF, sowie die Herstellung von Fusionsproteinen. Zur Anreicherung von PACE wird dabei vorgeschlagen, sekretionsfähiges PACE über konventionelle Methoden zu isolieren.

Stieneke-Gröber et al. (EMBO J. 11:2407-2414, 1992) beschreiben die in vitro-Spaltung von Influenza-Virus-HA-Protein durch gereinigtes Furin. Decroly et al. (J. Biol. Chem. 269: 12240-12247, 1994) beschreiben die in vitro-Spaltung von HIV gp160 durch Furin.

Bei Versuchen mit·C-terminal verkürztem Furin konnte in vitro die Spaltung von Proalbumin und Komplement Pro-C3 (Oda et al., Biochem. Biophys. Res. Commun. 189:1353-1361, 1992), Anthrax-Toxin (Klimpel et al. Proc. Natl. Acad. Sci. USA 89:10277-10281, 1992), Diphtherie-Toxin (Tsuneoka et al., J. Biol. Chem. 268: 26461-26465, 1993) und pro-Faktor IX (Wasley et al. J. Biol. Chem. 268: 8458-8468, 1993, Bristol et al., Biochemistry 33: 14136-14143, 1994) erfolgreich durchgeführt werden.

In vitro-Prozessierung von pro-vWF durch Furin konnte bisher nicht gezeigt werden. Rehemtulla et al. (Blood 79:2349-2355, 1992 und Proc. Natl. Acad. Sci. USA 89:8235-8239, 1992) beschreiben, daß durch Mischen von Überständen von Zellen, transfiziert mit pro-vWF bzw. deletiertem Furin ("PACE SOL"), pro-vWF nicht zu vWF prozessiert wird. Im Gegensatz dazu konnten mittels gereinigtem "PACE SOL" in vitro sowohl synthetische Substrate (Rehemtulla et al., Proc. Natl. Acad. Sci. USA 89:8235-8239, 1992), als auch pro-Faktor IX (Bristol et al., Biochemistry 33:14136-14143, 1994) gespalten werden. Für pro-vWF wurde weiters wiederholt postuliert, daß er in vitro durch trunkiertes Furin nicht in seine reife Form prozessiert wird (Rehemtulla et al., Proc. Natl. Acad. Sci. USA 89:8235-8239, 1992 und Blood 79:2349-2355, 1992), während unter analogen Bedingungen Faktor IX gespalten wird (Wasley et al. 1993. J. Biol. Chem. 268:8458-8465).

Um bei der rekombinanten Herstellung von Proteinen aus Pro-Proteinen hohe Ausbeuten an vollständig prozessierten Proteinen zu erhalten, wurde es gemäß dem Stand der Technik als notwendig angesehen, eine genügend große Menge an Furin zu exprimieren und zu isolieren, oder das Pro-Protein und Furin zu koexprimieren.

Bei der rekombinanten Expression von Furin alleine, aber auch bei der Koexpression von Furin mit einem Pro-Protein im großtechnischen Ansatz in Zellkultur tritt allerdings das Problem auf, daß eine hohe Expression der Protease toxisch für die Zellen ist (Creemers 1994), wodurch nur eine geringe Ausbeute an Furin und an reifem Protein möglich wird. So konnte in Koexpressionsstudien gezeigt werden, daß Zellklone, die Furin hoch exprimieren und das Pro-Protein effizient prozessieren, zu weitaus geringeren Zelldichten wachsen im Vergleich zu Zellen, die kein Furin exprimieren. Daraus resultiert eine schlechtere Gesamtausbeute an prozessiertem Protein. Um eine hohe Ausbeute an reifem Protein zu erhalten, muß daher eine sehr lange Kultivierungszeit in Kauf genommen werden; der Bedarf an Kultivierungsgefäßen und -geräten ist dadurch groß, was in der Folge auch erhöhte Kontaminationsprobleme mit sich bringen kann.

Bisher konnten nach rekombinanter Expression Furin oder Furinderivate nur immunologisch im Westernblot detektiert werden (Molloy et al. 1994. EMBO J. 13:18-33). Versuche, Furin oder Furinderivate hoch zu exprimieren, gelangen bisher nur im Baculovirus-Expressionssystem, wobei eine 20-30fach höhere Expression als in transfizierten Säugerzellen postuliert wurde (Bravo et al., 1994,. J. Biol. Chem. 269:25830-25837). Allerdings ist, trotz der relativ hohen Ausbeute an Furin im Vergleich zu anderen Zellsystemen, das Wachstum dieser virusinfizierten Zellen aufgrund der Zellyse als Folge der Virusvermehrung beschränkt. Eine gute Expression, sowie die Isolierung und Reinigung von Furin oder Furinderivaten, stellt für den immer breiter werdenden Anwendungsbereich von Furin bzw. von Furinderivaten z.B. bei der rekombinanten Herstellung von furin-prozessierten Proteinen aus Pro-Proteinen, eine große Bedeutung und Notwendigkeit dar.

Da eine Überexpression der Protease das Wachstum von kontinuierlich wachsenden Zellkulturen negativ beeinflusst, wurden Lösungsansätze gesucht, um den toxischen Einfluss von Furin auf die Zellen zu reduzieren. Weiter besteht ein Bedarf nach einem verbesserten Verfahren zur Prozessierung von furin-aktivierten Proteinen aus Pro-Proteinen, insbesondere für die großtechnische Herstellung von rekombinanten Blutfaktoren, wie etwa pro-vWF. Die EP 0 416 890 A betrifft ein neues Verfahren zur Aktivierung von Protein C mit Hilfe von immobilisiertem Thrombin. Das Thrombin weist Lysin-Reste auf, die in einem nicht-Amino-Puffer (z.B. HEPES) an die Säule gebunden werden. Das inaktive Protein C wird mit dem immobilisierten Thrombin (z.B. bovines Thrombin) in Kontakt gebracht und anschließend wird das aktivierte Protein aus der Säule eluiert. Als Träger wird dabei z.B. Sepharose oder Agarose verwendet.

Die EP 0 565 511 A offenbart ein Verfahren zur kontrollierten, eingeschränkten, proteolytischen Spaltung von Proteinen, insbesondere Proenzymen, das in Gegenwart eines Detergens oder einer chaotropen Substanz, ausgenommen gerinnungsaktive Salze, durchgeführt wird. Auf diese Weise kann das Spaltungsmuster je nach Art und Konzentration der anwesenden Stoffe gesteuert und nur wenige und ganz bestimmte Proteinfragmente gebildet werden. Bevorzugterweise ist dabei das Proenzym an einen festen Träger, z.B. einem schwerlöslichen Salz oder Chelat eines zweiwertigen Metalls gebunden, so dass eine erleichterte Gewinnung des vom adsorbierten Proenzym abgespaltenen Proteinfragments gewährleistet ist. Das Proenzym (z.B. Prothrombin) wird selektiv über den gamma-Carboxyterminus der zweiwertigen Ionen des Trägers gebunden.

Das Ziel der vorliegenden Erfindung ist daher ein Verfahren zur Herstellung von Furin oder einem Furinderivat aus einer kontinuierlich wachsenden, rekombinanten Zellkultur unter Aufrechterhaltung der enzymatischen Aktivität des Furins oder Furinderivats zur Verfügung zu stellen, ohne dass die Zellkultur wesentlichen Schaden durch die erhöhte proteolytische Aktivität nimmt.

Ein weiteres Ziel der vorliegenden Erfindung besteht darin, ein verbessertes Verfahren zur furin-abhÄngigen proteolytischen Spaltung von Pro-Proteinen zu Proteinen, insbesondere ein neues Verfahren zur Prozessierung von pro-von Willebrand-Faktor zu reifem, aktivem von Willebrand-Faktor, zur Verfügung zu stellen.

Diese Aufgaben werden erfindungsgemäß gelöst durch Zurverfügungstellung von neuen Fusionsproteinen, die aus einem Furin fusioniert mit einer heterologen Sequenz, welche zur Adsorption des Furins an einen festen Träger befähigt, bestehen, bei dem der C-terminale Bereich durch Deletion entfernt ist und durch eine heterologe Sequenz ersetzt ist.

Die erfindungsgemäßen Furin- oder Furinanalogen umfassen eine heterologe Substanz, wie etwa ein heterologes Protein, Polypetid oder funktionell aktives Peptid, insbesondere ein Affinitätspeptid. Erfindungsgemäß ist die heterologe Sequenz so ausgewählt, dass sie eine hohe Affinität oder eine spezifische Bindungseigenschaft für eine funktionelle Gruppe eines Trägers besitzt. Das heterologe Protein oder Polypeptid sollte dabei ein immunologisch gut charakterisiertes Protein sein, gegen das beispielsweise Antikörper zur Kopplung an einen festen Träger zur Verfügung stehen. Erfindungsgemäß kann die Adsorption an den festen Träger z.B. durch kovalente Bindung oder über Affinität erfolgen. Die Proteine oder Polypeptide können dabei abgeleitet sein von z.B. β-Galaktosidase, c-myc-Produkt, Glutathion S-Transferase, Avidin und die Lysin-bindende Kringeldomäne von Plasmaproteinen, wie z.B. von Plasminogen (Evan et al. Mol. Cell. Biol. 5: 3610-3616, 1985; Duijhoven et al., Hybridoma 11:71-86, 1992). Die funktionell aktiven Peptide, welche in der heterologen Sequenz liegen, können aus einer Anreihung von mehreren, gleichen oder verschiedenen Aminosäuren bestehen.

Eine bevorzugte Ausführungsform der vorliegenden Erfindung betrifft ein Fusionsprotein, dessen heterologer Sequenzanteil ein Peptid, das mit einem festen Träger eine kovalente Bindung eingehen kann, oder ein Poly-Histidin, das eine hohe Affinität insbesondere zu Schwermetallionen oder spezifischen anti-Poly-Histidin-Antikörpern besitzt, umfasst.

Durch die C-terminale Deletion der cytoplasmatischen und transmembranen Region wird lösliches Furin exprimiert; das aus den rekombinanten Zellen sekretiert wird. Gegebenenfalls kann bei einem Furinderivat oder dem Derivat eines Analogons zusätzlich die Cystein-reiche Region deletiert sein. Die enzymatische Aktivität des deletierten Proteins ist erfindungsgemäß im Vergleich zum kompletten Protein im Wesentlichen unverändert.

Unter einem Derivat eines Furinanalogen wird im Rahmen der vorliegenden Erfindung jedes Furin-ähnliche Protein verstanden, das gleiche oder ähnliche biologische Aktivität wie Furin aufweist oder Sequenzhomologie zu Furin besitzt. Dies gilt in erster Linie für das mit Furin idente PACE, aber auch PACE4, PC1/PC3, PC2 und PC4 sind in die vorliegende Erfindung miteingeschlossen.

Es sollen aber auch alle weiteren durch Insertion, Deletion oder Austausch von Aminosäuren bzw. Nukleotiden aus Furin bzw. dem Furinanalogen generierten Proteine bzw. Nukleinsäuren, welche Furin-ähnliche biologische Aktivität haben, in den Rahmen der vorliegenden Erfindung fallen.

Fusionsproteine, bestehend aus einem deletierten Furinanteil und einer heterologen Sequenz, sind zwar im Stand der Technik beschrieben, diese bekannten Fusionsproteine eignen sich jedoch nicht zur Lösung der erfindungsgemäßen Aufgabe. So offenbaren Duijhoven et al. (Hybridoma 11:71-86, 1992) N-terminale Furin-deletionsmutanten fusioniert mit Glutathion S-Transferase. Ebenso wurde die Pre-Pro-Sequenz von PACE(Furin) an den N-Terminus der leichten Kette der bovinen Enterokinase fusioniert (LaVallie et al. J. Biol. Chem. 268:23311-23317, 1993). Fusionsproteine enthaltend ein sogenanntes FLAG Epitop-"Tag", inseriert an das N-terminale Ende des katalytischen Zentrums des Furins nach Aminosäure Arg107, sowie murine Furinmutanten, bei denen der transmembrane und cytoplasmatische Bereich C-terminal nach Aminosäure 704 deletiert und durch ein Antikörper-Epitop des HSV Glykoproteins D ersetzt wurde, wurden ebenfalls beschrieben (Molloy et al. EMBO J. 13: 18-33, 1994; Matthews et al. Protein Science 3: 1197-1205, 1994). Diese Furinderivate wurden für die Detektion von Furin während seiner Reifung und Prozessierung im Golgi-Apparat oder zur Detektion von furinhaltigen zellkulturüberständen mittels Immunblot eingesetzt und sind zur biotechnologischen Anwendung für die Zwecke der vorliegenden Erfindung, insbesondere zur Spaltung von pro-vWF in vWF, in vitro ungeeignet. Solche Furinderivate sollen daher nicht in den Rahmen der vorliegenden Erfindung fallen.

Gemäß einem besonderen Aspekt der vorliegenden Erfindung besteht das Fusionsprotein aus einem Furin-Derivat, dessen C-terminale cytoplasmatische und transmembrane Domäne und gegebenenfalls Cystein-reiche Region deletiert und durch ein Affinitätspeptid ersetzt worden ist. Dabei wurde ein Furinderivat oder ein Derivat eines Furinanalogons mit einem funktionellen Peptid, insbesondere aus mehreren Histidin-Resten, vorzugsweise aus 3 bis 20 Histidin-Resten, besonders bevorzugt aus 6 bis 15 aufeinander folgenden Histidin-Resten, fusioniert. Die Verwendung von C-terminal an ein Protein fusionierten Affinitätspeptiden in Form von Poly-Histidin-Resten (sogenanntes "His-Tag") zur Reinigung und/oder zu Funktionsstudien von Proteinen wurde beschrieben (Janknecht et al., Proc. Natl. Acad. Sci. USA 88:8972-8976, 1991, Hoffmann et al., Nucleic Acids Res 19:6337-6338, 1991, EP 0 282 042).

In einer bevorzugten Ausführungsform der vorliegenden Erfindung wurde ein Furinderivat mit deletierter cytoplasmatischer und transmembraner Region mit einem Peptid aus mehreren Histidin-Resten fusioniert. Gemäß einer besonderen Ausführungsform ist Furin derart modifiziert, daß die für den transmembranen und cytoplasmatischen Teil kodierenden Sequenzen (Aminosäure 708 bis 794) deletiert (rFurinΔTM) und nach der Aminosäure 707 die kodierende Sequenz für sechs Histidin-Reste angehängt werden. Das so erhaltene Fusionsprotein wurde mit rFurinΔTM-His bezeichnet.

Eine weitere Ausführungsform der Erfindung betrifft ein Fusionsprotein, bei dem ein Furinderivat oder Derivat eines Furinanalogons mit deletiertem transmembranen und cytoplasmatischen Teil sowie der Cystein-reichen Region mit einem Affinitätspeptid aus mehreren Histidin-Resten fusioniert wurde.

In einer bevorzugten Ausführungsform wurde ein Furinderivat, bei dem neben der transmembranen und cytoplasmatischen Domäne die Cystein-reiche Region nach Aminosäure 585 deletiert war, mit sechs Histidin-Resten fusioniert. Dieses Fusionsprotein wurde mit rFurinΔCys-His bezeichnet.

Die Fusion eines Furinderivats oder eines Derivats eines Furinanalogons mit einer heterologen Sequenz soll gemäß der vorliegenden Erfindung so ausgeführt sein, daß die katalytische Funktion des Furins oder Furinanalogen im wesentlichen nicht beeinträchtigt wird.

Gemäß einem besonderen Aspekt der vorliegenden Erfindung wird daher zwischen die Sequenz des Furinderivats oder eines Derivat eines Furinanalogons und die heterologe Sequenz ein kurzer Peptid-Spacer inseriert, um das katalytische Zentrum des Furinderivats sterisch nicht zu behindern.

Dies ist insbesondere dann von Vorteil, wenn eine direkte Fusion der Cystein-reichen Region des Furinderivats mit einem Peptid die enzymatische Aktivität des Furinderivats beeinträchtigt, die Kopplung an den Träger durch chemische oder sterische Wechselwirkungen behindert oder mit einer effizienten Prozessierung des Pro-Proteins interferiert. Dieser kurze Peptid-Spacer, der vorzugsweise aus 5 bis 15 Aminosäuren besteht, ist im Speziellen aus kleinen, flexiblen Aminosäuren, wie Alanin oder Glycin, zusammengesetzt. In einer besonderen Ausführungsform wird zwischen die Furin-kodierende Sequenz und die heterologe Sequenz von 6 Histidin-Resten ein Spacer bestehend aus Ala-Ala-Gly-Gly-Ala-Ala inseriert. Die so entstandenen Fusionsproteine wurden mit rFurinΔTM-Spacer-His und rFurinΔCys-Spacer-His bezeichnet.

Das erfindungsgemäße Fusionsprotein weist über seinen Protein-, Polypeptid- oder Peptidanteil spezifische Bindungseigenschaften zu einem festen Träger auf. Als feste Träger können dabei Träger mit (Schwer) Metallionen, wie etwa Ni²⁺, Co²⁺, Mg²⁺, Li²⁺ oder mit Antikörpern eingesetzt werden.

Erfindungsgemäß ist das Fusionsprotein durch seine Bindung an den festen Träger immobilisiert. Bevorzugterweise wird das erfindungsgemäße Fusionsprotein mit einem heterologen Sequenzanteil bestehend aus mehreren Histidin-Resten aufgrund dessen Affinität zu (Schwer)Metallionen, insbesondere zu Ni²⁺, oder zu spezifischen anti-Poly-Histidin-Antikörpern, gebunden.

In einer besonderen Ausführungsform werden die Konstrukte rFurinΔTM-His, rFurinΔCys-His, rFurinΔTM-Spacer-His und rFurinΔCys-Spacer-His über ihre Affinität zu Ni²⁺-Ionen, oder zu einem Antikörper an den Träger gebunden. Der feste Träger kann gemäß der vorliegenden Erfindung als Matrix zur Verfügung gestellt werden. Die Bindung an die Matrix erfolgt dabei über die affinen Gruppen des festen Trägers, so dass das Fusionsprotein frei zugänglich ist. Dies ist insbesondere dann von Vorteil, wenn das an den Träger immobilisierte Fusionsprotein für die proteolytische Spaltung von Pro-Proteinen eingesetzt wird und dieser Prozess gebunden an einer Matrix erfolgt.

Als Matrix, an der der Affinitätsträger adsorbiert, können natürliche und synthetische Matrices, wie Sepharose, Agarose, Gelatine, Acrylate etc. verwendet werden. Die feste Matrix trägt je nach Versuchsansatz eine funktionelle Gruppe, die den Träger spezifisch binden kann.

Zur Konstruktion der Fusionsproteine wird die kodierende Nukleotidsequenz von Furin oder einem Furinanalogen derart modifiziert, dass die kodierende Sequenz für den cytoplasmatischen und transmembranen Bereich, und gegebenenfalls für die Cystein-reiche Region deletiert wird (Van der Ven et al. 1993. Crit.Rev.Oncogen 4:115-136). Dies erfolgt durch aus dem Stand der Technik bekannte gentechnische Methoden, wie spezifischen Restriktionsverdau mit Endonukleasen, Ligation oder PCR. Die so hergestellten Deletionsmutanten werden dann mit einer heterologen Sequenz ebenfalls über bekannte Techniken, fusioniert.

Die erfindungsgemäßen Fusionsproteine können ebenfalls durch chemische Synthese hergestellt werden.

Die Fusionsproteine werden vorzugsweise durch rekombinante Expression hergestellt. Die gentechnische Herstellung kann mit allen gängigen eukaryontischen Expressionssystemen, wie z.B. permanenten Zelllinien oder viralen Expressionssystemen, erfolgen. Die permanenten Zelllinien werden hergestellt durch stabile Integration der Fremd-DNA in das Wirtszellchromosom z.B. Vero, MRC5, CHO, BHK, 293, Sk-Hepl, insbesondere Leber- und Nierenzellen, oder durch einen episomalen Vektor, abgeleitet von z.B. Papilloma Virus. Virale Expressionssysteme, wie Vaccinia Virus, Baculovirus oder retrovirale Systeme können ebenfalls eingesetzt werden. Als Zelllinien werden allgemein Vero, MRC5, CHO, BHK, 293, Sk-Hep1, Drüsen-, Leber- und Nierenzellen eingesetzt. Als eukaryotische Expressionssysteme können auch Hefen, endogene Drüsen (z.B. Drüsen transgener Tiere) und andere Zelltypen, die endogen Furin oder Furinanaloge exprimieren, verwendet werden. Natürlich können auch transgene Tiere zur Expression von Furin oder Derivaten davon verwendet werden. Zur Expression der rekombinanten Proteine haben sich im speziellen CHO-DUXS B11 Zellen bewährt (Urlaub et al., Proc. Natl. Acad. Sci. USA 77:4216-4220, 1980).

Zur rekombinanten Herstellung der erfindungsgemäßen Fusionsproteine können auch prokaryontische Expressionssysteme eingesetzt werden. Hierzu eignen sich insbesondere Systeme, die eine Expression in E. coli oder B. subtilis erlauben.

Die Fusionsproteine werden in den entsprechenden Expressionssystemen unter der Kontrolle eines geeigneten Promotors exprimiert. Im Fall der Expression in Eukaryonten eignen sich dazu alle bekannten Promotoren, wie SV40-, CMV-, RSV-, HSV-, EBV-, β-Actin-, hGH oder induzierbare Promotoren wie z.B. hsp- oder Metallothionein-Promotor. Vorzugsweise werden die Fusionsproteine unter Kontrolle des β-Actin-Promotors in CHO-DUXS B11-Zellen exprimiert.

Gemäß einem weiteren Aspekt der vorliegenden Erfindung wird ein Fusionsprotein-Komplex enthaltend ein erfindungsgemäßes Fusionsprotein und einen festen Träger zur Verfügung gestellt. Als feste Träger können dabei Träger mit Metallionen, wie etwa Ni²⁺, Co²+, Mg²+, Li²⁺ oder Antikörper eingesetzt werden. Das Fusionsprotein bildet dabei mit dem Träger einen stabilen Komplex, wobei dieser Komplex gemäß einem weiteren Aspekt der Erfindung aus einer Lösung durch Bindung an eine Matrix entfernt werden kann. Die Bindung an die Matrix erfolgt dabei selektiv, wodurch keine weiteren Bestandteile der Lösung an das Trägermaterial gebunden werden.

In einer besonderen Ausführungsform wird der Fusionsprotein-Komplex dadurch erhalten, daß eine Fusionsprotein-haltige Lösung, vorzugsweise ein Zellkulturüberstand, mit einem festen Träger, gegebenenfalls gebunden an eine Matrix, in Kontakt gebracht wird, wodurch das Fusionsprotein spezifisch an den Träger adsorbiert. Das Fusionsprotein kann so selektiv aus der Lösung entfernt werden und das Fusionsprotein-freie Medium wieder zur Zellkultur zurückgeführt werden. Dies ist insbesondere deshalb von Vorteil, da während des Zellwachstums von den Zellen ausgeschiedene, wichtige Wachstumshormone dem Zellkultursystem wieder zur verfügung gestellt und nicht durch Mediumwechsel ausverdünnt werden. Gleichzeitig wird das mit dem Zellwachstum interferierende Fusionsprotein selektiv aus dem Medium entfernt und kann so das Zellwachstum nicht mehr negativ beeinflussen. Der so gewonnene Fusionsprotein-Komplex kann zur spezifischen in vitro-Spaltung von Pro-Protein zu Protein eingesetzt werden oder aber vom Träger wieder losgelöst und separat aufgearbeitet werden.

Ein anderer Aspekt der vorliegenden Erfindung betrifft ein Verfahren zur Herstellung von Proteinen aus Pro-Proteinen, bei dem ein Pro-Protein durch ein erfindungsgemäßes Fusionsprotein oder einen Fusionsprotein-Komplex proteolytisch gespalten wird.

Unter Pro-Proteinen sind hierbei sämtliche Vorstufen von Proteinen verstanden, welche durch geeignete proteolytische Behandlung in funktionelle Proteine umgewandelt werden können. Insbesondere können Pro-Proteine Pro-Enzyme, Prä-Pro-Enzyme oder andere (inaktive) Vorstufen von biochemisch (physiologisch) oder biotechnologisch verwendbaren Proteinen oder Enzymen sein.

Gemäß einem weiteren Aspekt der vorliegenden Erfindung wird das Pro-Protein durch das erfindungsgemäße Fusionsprotein in vitro in das mature Protein gespalten. Bei der in vitro-Spaltung sind, im Gegensatz zur zuvor dargelegten in vivo-Spaltung keine lebenden Zellen mehr direkt oder indirekt involviert.

Aufgrund der spezifischen Bindungseigenschaften der erfindungsgemäßen Fusionsproteine mit einem festen Träger kann das Fusionsprotein durch In-Kontakt-bringen mit einem festen Träger aus der Fusionsprotein-haltigen Lösung entfernt werden.

Gemäß einem besonderen Aspekt der Erfindung wird der Zellkulturüberstand enthaltend das Fusionsprotein über eine feste Matrix gepumpt, an die der affine Träger spezifisch gebunden ist. Das Fusionsprotein-freie Medium wird anschließend wieder zur Zellkultur zurückgeführt. Das an den festen Träger gebundene Fusionsprotein wird dabei vorzugsweise in einem kontinuierlichen Prozeß aus dem Zellkulturüberstand entfernt. Besonders bevorzugt ist dabei ein kontinuierliches Verfahren, bei dem in vorgegebenen Zeitabständen ein Durchfluß des Zellüberstandes über eine Matrix erfolgt. Dadurch wird gewährleistet, daß bei ständiger Expression das Fusionsprotein in einem Komplex an den festen Träger gebunden wird, und das Fusionsprotein kontinuierlich aus der Lösung entfernt wird. Der toxische Effekt der Protease auf das Zellwachstum wird dadurch stark reduziert und die Zellen können zu größerer Dichte wachsen, was wiederum die Ausbeute an Expressionsprodukt erhöht. Gleichzeitig wird durch das beschriebene Verfahren eine Anreicherung des Fusionsproteins an einer Matrix erreicht, wodurch auch eine hohe Reinheit des Fusionsproteins gewährleistet ist. Ein besonderer Vorteil dieses Verfahrens ist damit die Gewährleistung von verbessertem Zellwachstum verbunden mit einer Anreicherung von reinem Fusionsprotein an einer Matrix. Die Matrix liegt vorzugsweise als Säulenmatrix vor. Die so hergestellte Säulenmatrix kann direkt für die Aktivierung von Pro-Proteinen zu Proteinen eingesetzt werden.

Prinzipiell kann das oben beschriebene Verfahren auch mit "shed furin"-Furin oder Furinanalogen durchgeführt werden, wobei das Furin oder das Furinanaloge über einen Antikörper gebunden wird, der die proteolytische Aktivität nicht beeinträchtigt.

Bei Zellkulturüberständen, die sowohl Fusionsprotein als auch vollständig prozessiertes Protein enthalten, wie etwa bei Koexpression oder Kokultivierung, kann, wie oben erwähnt, das Fusionsprotein durch Bindung an einen ersten festen Träger aus der Lösung entfernt und spezifisch isoliert werden. In einem zusätzlichen Schritt kann das schon prozessierte Protein durch Adsorption an einen zweiten, vom ersten verschiedenen Träger ebenfalls aus der Lösung isoliert werden. Der zweite Träger ist dabei so ausgewählt, daß er spezifische Bindungseigenschaften zum prozessierten Protein besitzt und unprozessiertes Pro-Protein nicht bindet. Als bevorzugte Träger werden Träger mit Antikörpern, Peptiden und Proteinen mit hoher Affinität zum aktiven Protein eingesetzt. Der Träger ist erfindungsgemäß an eine feste Matrix gebunden, die vorzugsweise als Säulenmatrix vorliegt. Die Reihenfolge der an eine Säulenmatrix gebundenen Träger ist gemäß dem beschriebenen Verfahren variabel. Die Kopplung der Säulen erfolgt vorzugsweise sequentiell, wobei die Reihung Fusionsprotein-bindende Säule → Protein-bindende Säule besonders bevorzugt ist. Dieses Verfahren bietet den Vorteil, daß beim Durchlauf der Fusionsprotein-, Pro-Protein-und Protein-haltigen Lösung eine spezifische Bindung an den jeweiligen Träger erfolgt und eine Protein- und Fusionsprotein-freie Lösung zurückgeführt wird. Durch dieses Verfahren können damit auch bei der Koexpression und Kokultivierung von Fusionsprotein-exprimierenden Zellen höhere Zelldichten erreicht werden. Gleichzeitig wird eine Anreicherung sowie Isolierung von zwei verschiedenen, wichtigen Proteinen in einem einzigen Verfahrensschritt erreicht. Es ist zu betonen, daß dieses Verfahren nicht nur mit Furin-Fusionsproteinen durchführbar ist, sondern auch mit Wildtyp-Furin oder bekannten Furin-Mutanten, welche Furin-Aktivität aufweisen, indem diese Proteine an eine feste Matrix gebunden werden.

Die Durchführung der proteolytischen Spaltung des Pro-Proteins zum Protein in vitro kann derart erfolgen, dass neben dem Fusionsprotein dessen Reaktionspartner, das Pro-Protein, immobilisiert ist.

Dabei kann das Pro-Protein an einem festen Träger immobilisiert sein und das erfindungsgemäße Fusionsprotein mit dem Pro-Protein in Kontakt gebracht werden. Gemäß einer Ausführungsform werden für das erfindungsgemäße Verfahren Lösungen enthaltend gereinigtes Fusionsprotein eingesetzt. Dazu werden die Proteine mittels gentechnischer Methoden in transfizierten Zellen separat exprimiert, die Proteine aus dem Überstand gereinigt, in Puffer gelöst, an einem festen Träger immobilisiert und anschließend die proteinhaltigen Pufferlösungen miteinander in Kontakt gebracht. Die Reinigung der Proteine erfolgt dabei mit aus dem Stand der Technik allgemein bekannten Methoden wie Gelfiltrations-, Ionenaustauscher- oder Affinitätschromatographie.

Gemäß der vorliegenden Erfindung ist das Fusionsprotein an einem festen Träger immobilisiert und das Pro-Protein liegt in Lösung vor. Bei diesem Verfahren wird ein an eine Säulenmatrix adsorbierter Fusionsprotein-Komplex, enthaltend ein Fusionsprotein gebunden an einen Träger, mit einer Pro-Protein-haltigen Lösung, in vitro in Kontakt gebracht.

Zur Durchführung des erfindungsgemäßen Verfahrens eignen sich alle inaktiven Vorstufen eines Proteins, das durch die Aktivität von Furin oder eines Furin-ähnlichen Proteins in seine reife oder aktive Form überführt wird. In die vorliegende Erfindung sind daher insbesondere inaktive Vorstufen von Blutfaktoren oder von viralen Proteinen als Pro-Protein eingeschlossen, wobei jedoch keine Einschränkung auf diese erfolgt. Die Plasmaproteine sind insbesondere ausgewählt aus Faktor IX, von Willebrand-Faktor, Faktor VII, Faktor X, Faktor XI, Faktor V, Protein C, Protein S und Albumin oder Derivate davon. Mögliche virale Proteine oder Polypeptide sind solche von CMV, HDV HCV, HSV, HIV wie gp160 oder Influenza-Virus wie HA-Protein (Klenk et al., 1994, Cellular Receptors for Animal Viruses.CSH Laboratory Press. 241-280). Die Proteine sind vorzugsweise durch gentechnische Verfahren hergestellt. Jede Vorstufe eines Polypeptides mit mindestens einer dibasischen Spaltstelle ist jedoch ein Kandidat für das vorliegende Verfahren.

Beim erfindungsgemäßen Verfahren liegt die in vitro-Kontaktdauer zwischen Pro-Protein und Fusionsprotein zwischen einigen Sekunden und mehreren Tagen. Der optimale Kontakt ist abhängig vom verwendeten Fusionsprotein oder Fusionsproteinderivat und der inaktiven Pro-Protein-Vorstufe. Die Bestimmung der optimalen Kontaktdauer, bei der Pro-Protein vollständig zu Protein gespalten wird, kann jedoch von jedem Fachmann mittels einfacher Versuche erfolgen. Die Inkubation erfolgt meist bei einer Temperatur zwischen 4°C und 42°C, vorzugsweise zwischen 20°C und 38°C. Die Reaktion erfolgt bei einem pH-Wert von 5,0 bis 8,0, vorzugsweise bei einem pH-Wert von 6,5 bis 7,9, und insbesondere bei einem pH-Wert von 7,1. Aufgrund der Ca²⁺-Abhängigkeit der Furin- oder Furinanalogen-Aktivität werden üblicherweise zur Durchführung des Verfahrens solche Puffer eingesetzt, die Ca²⁺-Ionen beinhalten.

Die Reaktionsbedingungen für die Aktivierung von Pro-Protein durch das Fusionsprotein können, sofern einer der Reaktionspartner immobilisiert ist, ohne weiteres vom Fachmann je nach Versuchsanordnung innerhalb der gegebenen Rahmenbedingungen optimiert werden. Dabei ist für die Kontaktdauer als Variable die Fließgeschwindigkeit des in Lösung vorliegenden Reaktanden von besonderer Bedeutung. Diese sollte zwischen 0,05 ml/min und 1 ml/min liegen. Als weitere Parameter sind Temperatur, pH-Wert und Salzkonzentration von Bedeutung. Zur vollständigen Aktivierung von Pro-Protein können gemäß dem vorliegenden Verfahren mehrere Säulen hintereinander geschaltet, wobei entweder Pro-Protein oder Fusionsprotein an einen Träger immobilisiert sind. Nach jedem Durchlauf kann das bereits prozessierte Protein von seinem Propeptid abgetrennt und über selektive Chromatographie weiter gereinigt werden.

Die Durchführung des erfindungsgemäßen Verfahrens mit einem an einen Träger gebundenen Reaktionspartner ist deshalb von besonderem Vorteil, da die Reaktionsanordnung durch Verwendung eines Trägers, vorzugsweise einer Chromatographiesäule, einen zusätzlichen Reinigungsschritt ermöglicht.

Das gemäß dem vorliegenden Verfahren prozessierte, aktive Protein wird aus dem Reaktionsgemisch gereinigt und seine Aktivität mit aus dem Stand der Technik bekannten Methoden bestimmt.

Vor der Aufbereitung in eine pharmazeutische Präparation wird isoliertes und gereinigtes prozessiertes Protein den üblichen Qualitätskontrollen unterzogen und in eine therapeutisch verabreichbare Form gebracht.

Daher betrifft die Erfindung auch eine pharmazeutische Präparation enthaltend ein gemäß dem erfindungsgemäßen Verfahren hergestelltes Protein.

In der Fachwelt galt es bisher als unmöglich pro-vWF in vitro mit Furin zu aktivem vWF zu prozessieren (Rehemtulla et al., 1992, Blood 79:2349-2355, Rehemtulla et al., 1992, Proc. Natl. Acad. Sci., USA 89:8235-8239).

Es hat sich überraschenderweise herausgestellt, daß pro-vWFentgegen der Lehrmeinung- unter bestimmten Versuchsbedingungen durch Furin in vitro zu vWF prozessiert wird.

Ein besonderer Aspekt der vorliegenden Erfindung ist daher, daß pro-vWF als Pro-Protein mit dem erfindungsgemäßen Fusionsprotein, vorzugsweise FurinΔTM-Spacer-His, in Kontakt gebracht wird.

Im erfindungsgemäßen Verfahren wird eine Lösung enthaltend gereinigten pro-vWF und an einem Träger immobilisiertes Fusionsprotein eingesetzt.

Pro-vWF kann auch an einem Träger immobilisiert vorliegen. Für pro-vWF eignen sich in besonderer Weise Träger, die entweder pro-vWF über Antikörper oder über spezifische Liganden, wie z.B. Kollagen oder platelet-protein-gpIb, gpIIb/IIIa-Komplex, Faktor VIII-Fragmente, Heparin, Ristocetin oder Botrocetin binden. Die eingesetzten Antikörper können polyklonal oder monoklonal sein und entweder gegen das Propeptid des vWF oder gegen die reife Form des vWF gerichtet sein. Ist der Antikörper gegen das vWF-Propeptid gerichtet, so wird prozessierter vWF nach Kontakt mit dem Fusionsprotein vom Träger eluiert. Ist der Antikörper gegen maturen vWF gerichtet, so wird durch Kontakt mit dem Fusionsprotein das Propeptid abgespalten und aus dem Reaktionsgemisch entfernt. Der am Träger gebundene, prozessierte vWF kann anschließend mit bekannten Methoden von der Säule eluiert werden. Bei dieser Variante des erfindungsgemäßen Verfahrens erreicht man durch den Elutionsschritt zudem eine zusätzliche Reinigung und Anreicherung des vWF.

In einem weiteren Aspekt der vorliegenden Erfindung wird das Fusionsprotein an eine Matrix auf einem Träger mit Antikörpern oder Schwermetallionen gebunden. Die Antikörper können polyklonal oder monoklonal sein. Dabei werden in der Regel nur solche Antikörper eingesetzt, die die proteolytische Aktivität des Fusionsproteins nicht beeinträchtigen.

In einer besonderen Ausführungsform ist das Fusionsprotein über einen Träger mit Metallionen an die Matrix gebunden. Als Metallionen können dabei etwa Ni²⁺, Co²⁺, Mg²⁺ oder Li²⁺ eingesetzt werden.

Die Immobilisierung des pro-vWF oder des Furins oder seiner Derivate erfolgt mit in der Proteinchemie gängigen Verfahren.

Der nach dem erfindungsgemäßen Verfahren erhaltene prozessierte, mature vWF wird aus dem Reaktionsgemisch gereinigt und seine Aktivität mit aus dem Stand der Technik bekannten Methoden bestimmt (Baruch et al., 1989, Bailliere's Clinical Haematology 2:627-672).

Vor der Aufbereitung in eine pharmazeutische Präparation wird isolierter vWF den üblichen Qualitätskontrollen unterzogen, aufkonzentriert und in eine therapeutisch verabreichbare Form gebracht.

Desweiteren betrifft die Erfindung einen rvWF, der nach dem erfindungsgemäßen Verfahren hergestellt wird und eine pharmazeutische Präparation, die rvWF und einen oder mehrere physiologisch akzeptable Träger enthält. Es wurde gefunden, daß der nach dem erfindungsgemäßen Verfahren hergestellte, pro-Peptid-freie rvWF sich durch eine besonders hohe Stabilität und strukturelle Integrität der rvWF-Multimere auszeichnet und keine Satellitenbanden aufweist. Dieser rvWF eignet sich daher für die Stabilisierung von Faktor VIII, rekombinantem Faktor VIII oder funktionellen Deletionsmutanten von Faktor VIII sowohl in vitro als auch in vivo. Die pharmazeutische Präparation enthaltend pro-Peptid-freien rvWF besitzt hohe Stabilität sowie strukturelle Integrität der rvWF-Multimere und eignet sich daher besonders für die Behandlung von Hämophilie A und verschiedener Formen der vWF-Disease.

Ein besonderer Vorteil des erfindungsgemäßen Verfahrens ist, daß pro-vWF fast vollständig in seine reife Form gespalten wird. Erfindungsgemäß wird in vitro-prozessierter vWF in einer Reinheit von 80% bis 100%, vorzugsweise von 90% bis 100%, besonders bevorzugt 95% bis 100% erhalten. Eine Verunreinigung von vWF durch pro-vWF ist vor allem in Hinblick auf den Einsatz in der Therapie zu vermeiden, da ein mit pro-vWF verunreinigter rvWF, eine geringere spezifische Aktivität bzw. eine erhöhte Immunogenität aufweisen könnte. Das erfindungsgemäße Verfahren hat den zusätzlichen Vorteil, daß die proteinchemische Abtrennung von pro-vWF von vWF, insbesondere im Fall der Immobilisierung eines Reaktionspartners an eine Chromatographiesäule, erleichtert wird.

Bei hoher Amplifikation oder zu hoher Expression übt das Furin einen negativen Effekt auf die Zelle aus. Dieser Effekt kann minimiert werden, indem das produzierte Furin oder Fusionsprotein kontinuierlich aus dem Zellüberstand entfernt wird. Dies kann z.B. durch Furin-spezifische Chromatographie entweder im Batch- oder im Säulen-Verfahren erfolgen. Das in den Überstand sekretierte Furin oder Fusionsprotein wird dabei an einen chromatographischen Träger gebunden und der Träger mit immobilisiertem Furin kann dann gegebenenfalls direkt zur Pro-Protein-Spaltung eingesetzt werden.

Wenn eine große Menge von sekretiertem Furin oder Furinderivat gewonnen werden soll, besteht eine weitere Möglichkeit, die Toxizität von Furin auf die exprimierenden Zellen zu minimieren, darin, ein natürliches (Pro-Protein) oder ein synthetisches (Peptid-) Substrat mit Furin oder Fusionsprotein zu koexprimieren oder dem Zellkulturüberstand als Supplement zuzugeben. Ein Substrat kann dabei auch ein an Furin oder das Fusionsprotein reversibel bindendes, jedoch davon nicht spaltbares Peptid oder Protein sein, das die katalytische Aktivität des Furins oder Fusionsproteins verringert oder unterbindet, solange es mit Furin oder Fusionsprotein interagiert. Durch das zusätzliche Pro-Protein bzw. synthetische Substrat kann überschüssiges Furin bzw. Fusionsprotein, das unspezifisch innerhalb oder außerhalb der Zelle endoproteolytisch aktiv ist, abgefangen werden, und die Zellen nicht mehr schädigen. Das durch die hohe Expression synthetisierte Furin oder Fusionsprotein wird mit hoher Effizienz in den Zellüberstand sekretiert und kann anschließend aus dem Zellüberstand gereinigt werden.

Es ist daher ein besonderer Vorteil des Verfahrens, daß durch verschiedene, oben erwähnte Maßnahmen, die für die Zelle hohe Toxizität bedingt durch große Mengen an exprimiertem Furin oder Fusionsprotein reduziert wird, wodurch der Produktionsablauf bei Herstellung von großen Mengen an Furin oder Fusionsprotein und Pro-Protein wesentlich erleichtert bzw. effizienter gestaltet wird, da erstens eine höhere Zelldichte erreicht wird, zweitens in einer geringeren Zeitspanne eine höhere Ausbeute an sekretiertem Furin erreicht wird und drittens eine vollständige Prozessierung von Pro-Protein gewährleistet ist.

Die Durchführung des erfindungsgemäßen Verfahrens wird im folgenden näher beschrieben:

Zur Durchführung der Experimente wurden CHO-Zellen mit einem pro-vWF-kodierenden Plasmid und mit einem Dihydrofolat-Reduktase (DHFR)-cDNA-kodierenden Plasmid kotransfiziert. Das DHFR-kodierende Plasmid diente dabei als Markerplasmid zur Selektion positiver Klone (CHO-vWF). Der daraus resultierende Klon, CHO-vWF, wurde für die Koexpression mit Furin anschließend mit Plasmiden, die die Furin-cDNA bzw. cDNA kodierend für Fusionsproteine und das Neomycin-Phosphotransferase-Gen tragen, kotransfiziert. Das Neomycin-Phosphotransferase-Gen diente ebenfalls als Markergen zur Selektion positiver Klone (CHO-vWF/Furin). Zur Expression von Furin oder Fusionsprotein alleine wurden Zellen mit Plasmiden enthaltend komplette Furin-cDNA oder cDNA der Fusionsproteine (FurinΔTM-His-cDNA, FurinΔTM-Spacer-His-cDNA, FurinΔCys-HiscDNA, FurinΔCys-Spacer-His-cDNA) transfiziert.

Ein anderer Ansatz zum Etablieren pro-vWF/Furin-koexprimierender Zellen war die gleichzeitige Kotransfektion von 3 Plasmiden, die respektive pro-vWF-cDNA, DHFR-cDNA und Furin- oder Fusionsprotein-cDNA enthalten. Durch diesen Ansatz wurde eine Koamplifikation von pro-vWF- und Furin-cDNA ermöglicht, um eine unter den gegebenen Umständen möglichst hohe Ausbeute an vollständig prozessiertem vWF zu erreichen.

Für die Kokultivierung wurden Zellen mit Plasmiden, die entweder die kodierende Sequenz von pro-vWF und DHFR, oder für Furin bzw. Fusionsprotein und DHFR enthalten, kotransfiziert. Die unterschiedlich transfizierten Zellen wurden anschließend gemeinsam kultiviert.

Beispiel 1 beschreibt die Herstellung von pro-vWF- und Furin-exprimierenden Vektoren; Beispiel 2 beschreibt die Etablierung von stabilen Zellinien und zeigt die in vitro-Spaltung von pro-vWF durch Furin; Beispiel 3 skizziert die Klonierung von Furinmutanten und Fusionsproteinen; Beispiel 4 beschreibt den Nachweis der enzymatischen Aktivität von Furin und Fusionsprotein rFurinΔTM-His; Beispiel 5 beschreibt die Immobilisierung eines Fusionsprotein an einem Träger; und Beispiel 6 beschreibt die Aktivierung von pro-vWF an immobilisiertem Fusionsprotein.

Es zeigen:
- Figur 1:: Schematische Darstellung der Expressionskassette von pro-vWF, Furin und der verwendeten Selektionsmarker Dihydrofolat-Reduktase und Neomycin-Phosphotransferase.
- Figur 2:: Western-Blot-Analyse von prozessiertem vWF und Furin in Zellkulturüberständen nach Koexpression von pro-vWF und Furin.
- Figur 3:: Western-Blot-Analyse von Zellkulturüberständen auf rvWF und Nachweis der in vitro-Spaltung von pro-vWF durch Furin.
- Figur 4 :: Nukleotid- und Aminosäuresequenz von Furin.
- Figur 5:: Western-Blot-Analyse von rFurinΔCys-Spacer-10XHis mit anti-Furin monoklonalen Antikörpern.
- Figur 6:: Silberfärbung und Western-Blot-Analyse von gereinigtem rFurin-Fusionsprotein.
- Figur 7:: Western-Blot-Analyse von mittels gereinigtem rFurin-Fusionsprotein prozessiertem vWF.
- Figur 8:: Schematische Zeichnung des Expressionsvektors phAct-rFX.
- Figur 9:: Western-Blot-Analyse von rFaktor X exprimiert in CHO-Zellen vor und nach Amplifikation mit Methotrexat und
- Figur 10:: Western-Blot-Analyse von rFaktor X nach in vitro-Spaltung durch rFurin-Fusionsproteine.

Die Expressionsvektoren wurden mittels Standard-Klonierungs-Methoden (Maniatis et al., "Molecular Cloning" - A Laboratory Manual, Cold Spring Harbor Laboratory, Cold Spring Harbor, New York, USA, 1983) hergestellt. Die Herstellung von DNA-Fragmenten mittels Polymerase-Ketten-Reaktion (PCR) erfolgte durch allgemein bekannte Methoden (Clackson et al., 1991, PCR A practical approach. Ed. McPherson, Quirke, Taylor, S.187-214).

### Beispiel 1:

### Herstellung von pro-vWF und Furin Expressionsvektoren

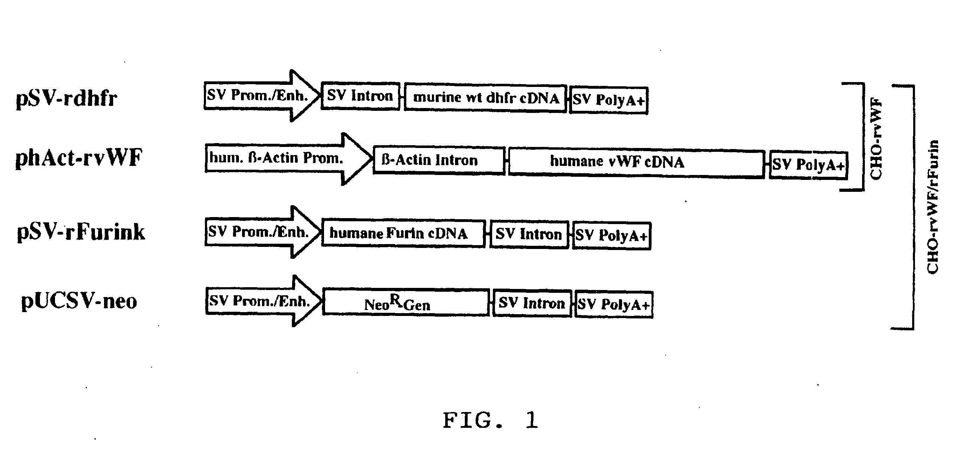

Zur Herstellung von rekombinantem pro-vWF (rpro-vWF) wurde Plasmid phAct-vMF wie in Fischer et. al., (FEBS Lett. 351: 345-348, 1994) beschrieben, konstruiert: Plasmid phAct-vWF enthält die komplette kodierende cDNA für humanen pro-vWF unter transkriptioneller Kontrolle des β-Actin-Promotors. Zur Selektion von positiven Klonen wurde Plasmid pSV-rdhfr, das für die murine DHFR-cDNA kodiert, eingesetzt (Figur 1).

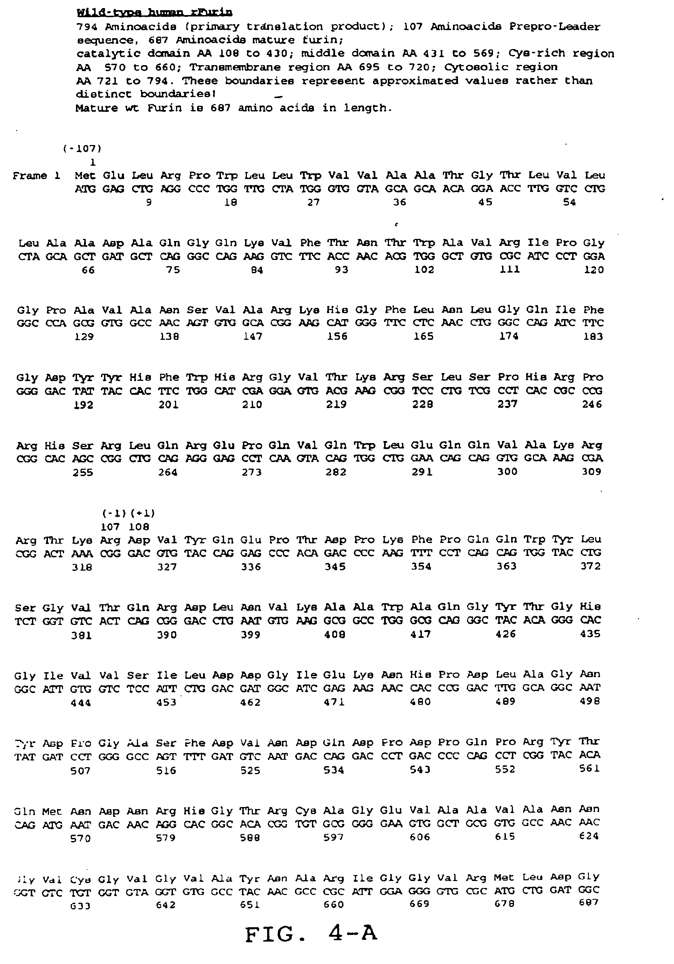

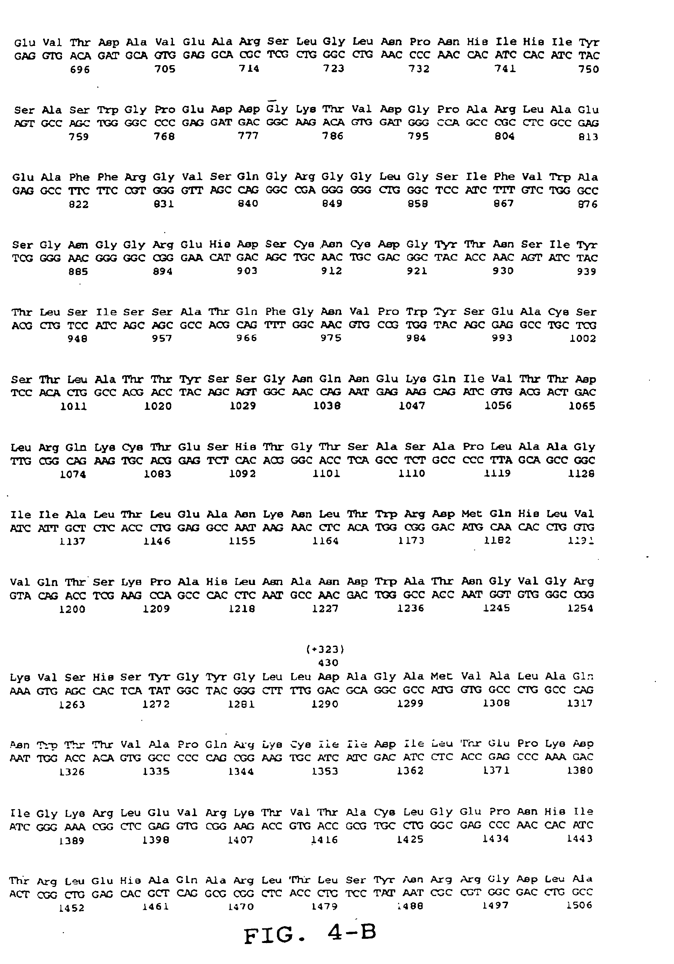

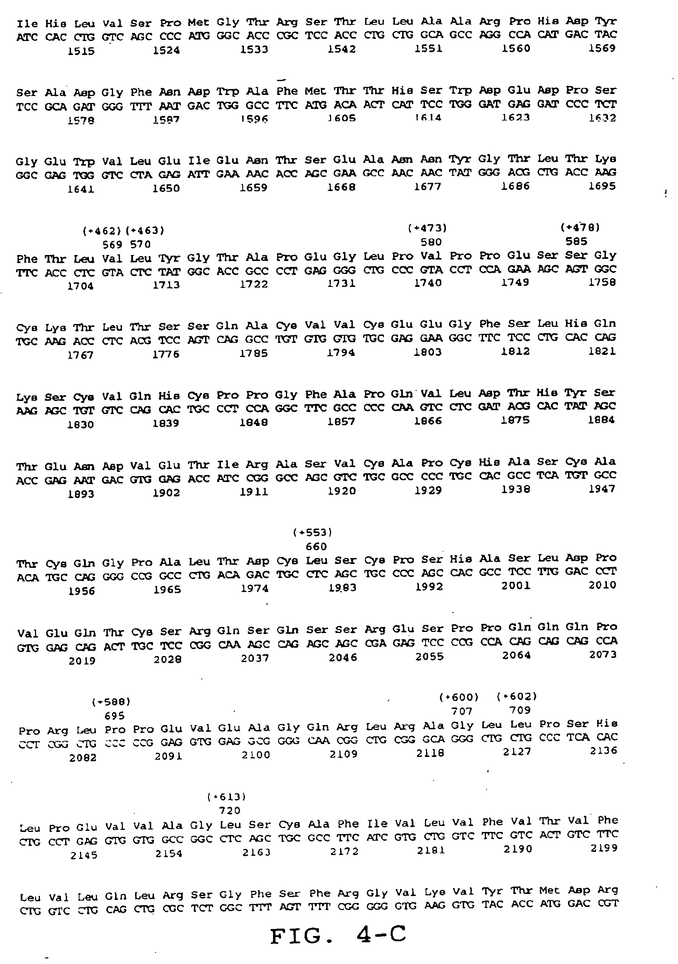

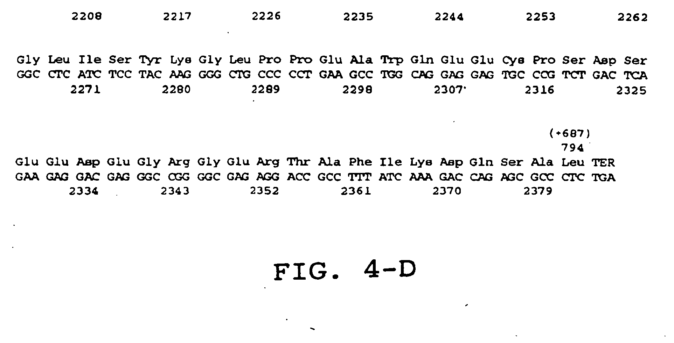

Zur Herstellung von Furin-kodierenden Vektoren wurde die komplette cDNA von humanem Furin (Figur 4), (Van den Ouweland et al., Nucleic Acids Res. 18:664, 1990) als SmaI/AvrII-Fragment isoliert. Dieses Fragment umfaßt die 2,4 kb Furin-kodierende Region, sowie 0,05 kb der 5' nicht-translatierten und 0,4 kb der 3' nicht-translatierten Region und wurde anschließend in den mit SmaI und AvrII geschnittenen Expressionsvektor pSV-MCS VII kloniert. Das resultierende Plasmid wurde mit pSV-rFurink bezeichnet (Figur 1).

Plasmid pSV-MCS VII umfaßt den Promotor/Enhancer der "frühen Gene" von SV40 und 50bp der 5'-UTR, sowie das SV40 16S/19S Intron und eine "Multiple Cloning Site" (MCS), gefolgt von der SV40-Polyadenylierungsstelle. Zur Herstellung von Plasmid pSV-MCS VII wurde Plasmid pSVβ (MacGregor et al., Nucleic Acids Res. 17: 2365, 1989) mit NotI geschnitten und die lacZ-Gensequenz als NotI-Fragment entfernt. Das 3'-seitig der SV40-Polyadenylierungsstelle befindliche XbaI/HindIII-Fragment wurde entfernt, die überstehenden Enden mit Klenow-Polymerase aufgefüllt und das Plasmid religiert. In die singuläre NotI-Restriktionsschnittstelle wurde eine synthetische MCS mittels NotI-kompatibler Enden kloniert und dadurch die NotI-Stelle zerstört. Die zu inserierende MCS wurde durch die beiden synthetischen, komplementären Oligonukleotide #256 (5'-GGCCATCGAT TGAATTCCCC GGGGTCCTCT AGAGTCGACC TGCAGAAGCT TAGTACTAGT AGGCCTAGGG CCCTA-3') (SEQ.ID. NO. 1) und #257 (5'-GGCCTAGGGC CCTAGGCCTA CTAGTACTAA GCTTCTGCAG GTCGACTCTA GAGGACCCCG GGGAATTCAA TCGAT-3') (SEQ.ID.NO. 2) konstituiert.

Plasmid pUCSV-neo wurde hergestellt, indem die SV40-neo-Expressionskassette aus pMAMneo (Lee et al., 1981, Nature 294:228-232) als BamHI-Fragment in die BamHI-Restriktionsstelle von pUC19 (Yanisch-Perron et al., 1985, Gene 33:103-119) inseriert wurde.

### Beispiel 2:

### a. Etablierung stabiler rvWF- und rvWF/rFurin-exprimierender Zellinien und Expression von rvWF und rFurin

Das Expressionsplasmid für rvWF, phAct-vWF (Figur 1), wurde mit dem Selektionsmarkerplasmid pSV-rdhfr (Figur 1) in dhfr-defiziente CHO-Zellen cotransfiziert, unter Selektionsbedingungen der effizient pro-rvWF-exprimierende Klon CHO-rvWF ausgewählt und dieser Klon bis zur Stabilität subkloniert (Fischer et al. 1994. Febs Lett.351:345-349). Zur weiteren Analyse und für rvWF-Expressions- und Funktionsstudien wurden die Zellen zunächst mehrmals mit PBS gewaschen, und anschließend, wenn nicht anders beschrieben, bei regelmässigen 24-Stunden-Medienwechseln in Selektionsmedium ohne Serum inkubiert.

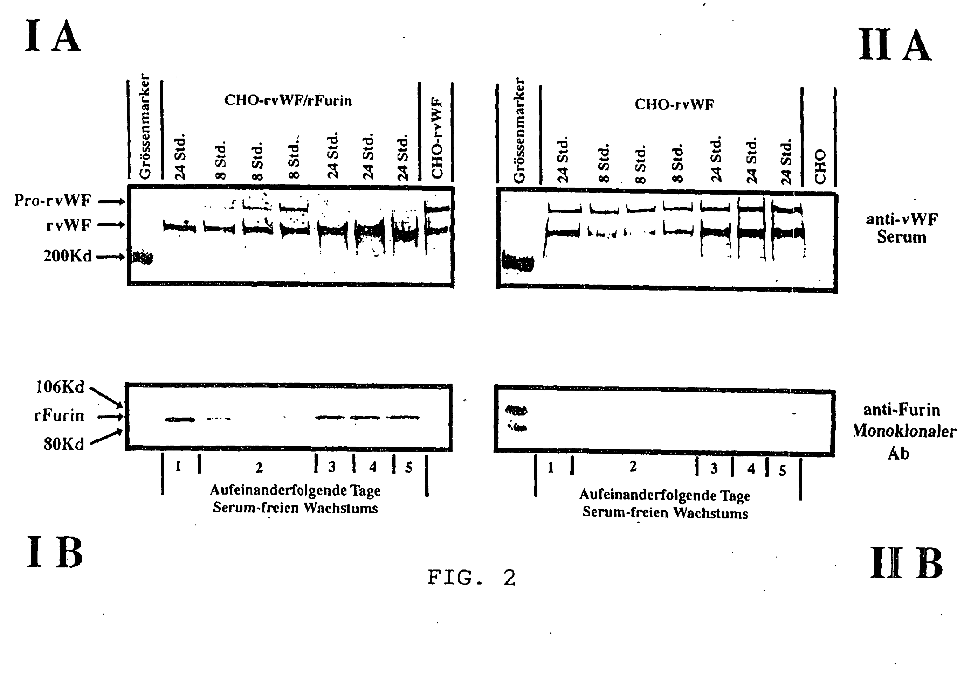

In den Zellüberstand von CHO-rvWF sekretierter rvWF war zu etwa 40% unprozessiert (Figur 2 II A). Um die Effizienz der Propeptid-Abspaltung zu erhöhen, wurde im folgenden der rFurin-Expressionsvektor pSV-rFurink mit dem Selektionsmarker-Plasmid pUCSV-neo in den CHO-rvWF-Zellklon cotransfiziert. Unter Selektionsbedingungen (500µg G418/ml) wurden Klone identifiziert, die neben rvWF auch rFurin exprimieren (CHO-rvWF/rFurin; Figur 2 I A, B).

Der Nachweis von rvWF im Zellkulturüberstand erfolgte mittels Western-Blot Analyse (Figur 2 I A und II A). Dazu wurden 10µl reduzierter Zellkulturüberstand mittels SDS-PAGE (Lämmli, Nature 227:680-685, 1970) aufgetrennt, und die Proteine anschließend mit dem BioRad Mini Trans-Blot System (BioRad Laboratories, Richmond, CA, USA) auf Nitrozellulose-Membranen transferiert. Zur Visualisierung von in den Zellkulturüberstand sekretiertem rvWF wurde das Protoblot-System der Fa. Promega (Madison, WIS, USA) verwendet. Als Antikörper zur vWF-Bindung wurde Kaninchen Anti-vWF Serum (Best. No. A 082) der Fa. Dakopatts (Glostrup, Dänemark) eingesetzt.

Der Nachweis von rFurin im Zellkulturüberstand erfolgte ebenfalls mittels Western-Blot Analyse (Figur 2 I B und II B). Die Visualisierung von rFurin erfolgte unter Verwendung des anti-hFurin Maus monoklonalen Antikörpers MON 148 (van Duijnhoven et al., Hybridoma 11: 71-86, 1992) und als zweitem Antikörper anti-Maus IgG-alkalische Phosphatase-konjugiertes Ziegenserum (Sigma A 4656).

In 24-Stunden-Zellkulturüberständen von CHO-rvWF-Zellen waren noch etwa 40% des sekretierten rvWF Propeptid-haltiger pro-vWF (Figur 2 II A), wogegen unter identischen Bedingungen in 24 Stunden-Überständen des CHO-rvWF/rFurin-Klons kein pro-vWF, sondern nur vollständig prozessierter rvWF detektiert wurde (Figur 2 I A). Hieraus folgt somit, daß in serumfreien 24-Stunden-Zellkulturüberständen kein pro-rvWF mehr nachgewiesen werden konnte, wenn die Zellen ausreichende Mengen rFurin exprimieren (Figur 2 I A, 1, 3, 4, 5). Durch die zusätzliche Expression von rFurin in CHO-rvWF konnte die Prozessierung von pro-rvWF zu vWF deutlich verbessert werden (Figur 2 I A). Dieser Effekt wurde bisher ausschließlich auf die intra-zelluläre Aktion des durch Koexpression hergestellten rFurins zurückgeführt (Wise et al., Proc. Natl. Acad. Sci., USA 87:9378-9382, 1990; Van de Ven et al. Mol. Bio. Rep. 14:265-275, 1990; Rehemtulla et al., Blood 79:2349-2355, 1992).

Erfolgte bei Koexpression von pro-vWF und Furin ein häufigerer Mediumwechsel innerhalb von 24 Stunden (alle 8 Stunden), so konnten jedoch auch in Zellkulturüberständen von serumfrei gezüchteten CHO-rvWF/rFurin-Zellen signifikante Mengen von pro-rvWF nachgewiesen werden (Figur 2 I A; "8 Stunden"). In den Überständen der CHO-rvWF/rFurin-Zellen war rFurin detektierbar (Figur 2 I B); je größer die detektierbare rFurin-Menge im Überstand war, umso geringer war die Menge an pro-vWF (Figur 2 I A).

Eine Erklärungsmöglichkeit - die jedoch im krassen Gegensatz zur vorherrschenden Lehrmeinung (Rehemtulla et al., 1992, Blood 79: 2349-2355, Rehemtulla et al., 1992, Proc. Natl. Acad. Sci. USA 89:8235-8239) steht - ist, daß durch Überexpression in den Zellkulturüberstand gelangtes rFurin erst dort, d.h. in vitro, sekretierten pro-rvWF bis zur vollständigkeit spaltet. Die durch den 8stündigen Medienwechsel kürzere Expositionszeit von pro-rvWF im Überstand zum ebenfalls in den Überstand sekretierten rFurin war daher nicht ausreichend, um alle im Überstand vorhandenen pro-vWF-Moleküle zu prozessieren; dagegen konnte bei längeren Verweilzeiten (24 Stunden) sekretiertes rFurin im Überstand akkumulieren, so daß im Laufe der rFurin-Akkumulation immer mehr der zunächst im Überstand angesammelten pro-rvWF Moleküle prozessiert wurden. In der Folge wurde dann durch die Akkumulation immer größerer Mengen von rFurin weiterhin in den Überstand gelangter pro-rvWF dort sofort nach Ausschleusen aus der Zelle prozessiert. Während bei 8 Stunden-Überständen das Furin:pro-vWF-Verhältnis noch stärker auf Seiten des pro-vWF liegt, hat sich die Situation bei den 24 Stunden-Überständen aufgrund der Akkumulation von rFurin umgekehrt.

Gemäß dem vorliegenden Beispiel wurde rFurin im Überstand von CHO-rvWF/rFurin-Zellen mit Hilfe von Western Blot-Analyse und anti-hFurin monoklonalem Antikörper eindeutig nachgewiesen (Figur 2 I B). Dabei korrelierte der Prozessierungsgrad von pro-rvWF mit der detektierbaren Menge an rFurin im Überstand: je mehr rFurin im Überstand nachweisbar war, umso weniger pro-rvWF war vorhanden (vgl. Figur 2 I B mit 2 I A).

### b. In vitro-Spaltung von pro-rvWF durch rFurin

Zum direkten Nachweis der rFurin-Prozessierungsaktivität für rekombinanten pro-vWF in vitro wurden serumfreie rFurin und pro-vWF-enthaltende Zellkulturüberstände gemischt und inkubiert, sowie entsprechende Kontrollen gemacht. Da vernünftige Expressionsausbeute von rwt-Furin in ausschließlich rFurin exprimierenden CHO-Zellen durch die Interferenz erhöhter rFurin-Konzentration mit der Lebensfähigkeit der Zellen nicht möglich ist, wurde auf rFurin/ vWF-Zellen als Quelle für rFurin zurückgegriffen. Durch die Koexpression konnten detektierbare rFurin-Mengen erzielt werden. Entsprechend wurden CHO-rvWF, CHO-rvWF/rFurin, CHO-rvWF und CHO (im Verhältnis 1:1), sowie CHO-rvWF und CHO-rvWF/rFurin (im Verhältnis 1:1) gemischt und bei 37°C inkubiert.

Aliquote der Reaktionsansätze wurden direkt vor der Inkubation bzw. nach dem Mischen der Zellkulturüberstände mittels Western Blot-Analyse auf prozessierten vWF getestet; weitere Aliquote wurden in Zeitintervallen von jeweils 24 Stunden entnommen und untersucht.

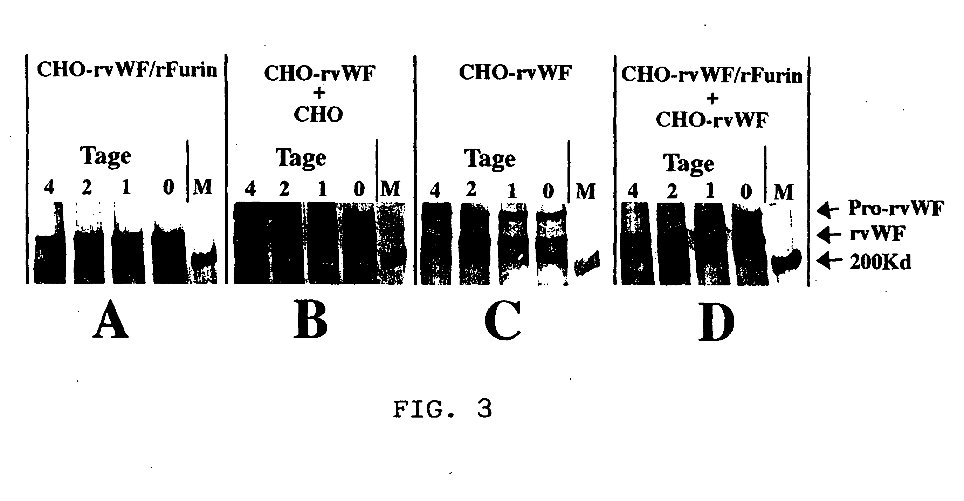

Figur 3 zeigt eine vollständige Prozessierung von pro-rvWF nur in jenen Testansätzen, die entweder rvWF/rFurin koexprimiert hatten oder die CHO-rvWF/rFurin und CHO-rvWF enthielten (Figur 3 A, Spur 0-4, Figur 3 D, Spur 2 und 4). Während pro-rvWF im 24 Stunden-CHO-rvWF/rFurin-Überstand a priori vollständig zu rvWF prozessiert war (Figur 3 A, Spur 0), war in CHO-rvWF-Zellkulturüberständen noch zu 50% unprozessierter vWF im Zellkulturüberstand vorhanden (Figur 3 B und C). In Testansätzen, bei denen die Zellkulturüberstände von CHO-rvWF/rFurin und CHO-rvWF gemischt worden waren, war nach 24 Stunden-nur noch ein geringer Anteil an unprozessiertem pro-vWF nachweisbar (Figur 3 D, Spur 1). Eine Verlängerung der Inkubationsdauer auf 48 h und 96 h (Figur 3 D, Spur 2+4) führte zu vollständig prozessiertem vWF. Damit wurde der Nachweis erbracht, daß in den Überstand von CHO-rvWF/rFurin-Zellen sekretiertes rFurin biologisch aktiv ist und pro-rvWF aus CHO-rvWF-Überständen in vitro vollständig prozessiert.

### Beispiel 3:

### Klonierung und Expression von rFurin-Deletionsmutanten und rFurin-His-Tag-Fusionsproteinen

Zur Herstellung eines sekretionsfähigen rFurins wurden verschiedehe C-terminal-trunkierte Furin-Deletionsmutanten konstruiert und diese zur späteren leichteren Isolierung aus dem Kulturmedium mit zusätzlichen heterologen Sequenzen fusioniert.

Alle rFurin-Mutanten wurden in den Expressionsvektor phAct-ΔEcoRIupstream, der den humanen β-Actin-Promotor enthält, kloniert und, wie in Beispiel 2 beschrieben, stabil in CHO-Zellen exprimiert.

Die Klonierung von Plasmid phAct-ΔEcoRIupstream erfolgte durch partialen EcoRI-Verdau von Plasmid phAct (Fischer et al., FEBS. Lett. 1994, 351:345-348), Auffüllen der überstehenden Enden mit Klenow-Enzym, und Religation. Plasmid phAct-ΔEcoRIupstream unterscheidet sich von phAct durch das Fehlen der EcoRI-Schnittstelle 5'-seitig des Promotors; die EcoRI-Schnittstelle 3' des Promotors, bzw. des Introns, in der MCS ist dagegen noch vorhanden.

Zur Deletion der C-terminalen transmembranen Domäne wurde die rFurin-cDNA nach Position 2127 der Nukleinsäuresequenz (Figur 4) und damit bei Aminosäure 709 deletiert. Mit Hilfe der synthetischen Oligonukleotide #2325 (5'-GATAAGCTTG TCGACCATGG AGCTGAGGCC CTG-3') (SEQ.ID.NO. 3) und #2819 (5'-AAGTCATGAA TTCTTAC AGCAGCCCTG CGCGCAG-3') (SEQ.ID.NO. 4) wurde anhand des "Templates" pSV-rFurink (Beispiel 1) mit der Polymerase-Ketten-Reaktion (PCR) ein DNA-Fragment generiert; dieses Fragment enthielt die Basenpaare analog der ersten 709 Aminosäuren des Furins, gefolgt von einem Translations-Terminations-Triplett. Das entstandene rFurin-ATM-Fragment wurde an den flankierenden SalI- und EcoRI-Restriktionsstellen geschnitten und in den SalI/EcoRI geschnittenen Expressionsvektor phAct-ΔEcoRIupstream inseriert. Das entstandene Plasmid wird mit prFurin-ΔTM bezeichnet. Die Nukleotid- und Aminosäuresequenz von rFurin-ΔTM ist als SEQ.ID.NO. 5 und SEQ.ID.NO 6 wiedergegeben.

Zur Herstellung von FurinΔTM-Fusionsprotein mit einem 3'-seitig fusionierten Affinitätspeptid wurden an das C-terminale Ende von rFurinΔTM an Aminosäure 707(Basenpaar 2121) sechs Histidin-Reste (His-Tag) angehängt:
Mit Hilfe der synthetischen Oligonukleotide #2325 (SEQ.ID.NO. 3) und #2823 (5'-CTAGAATTCAAT GATGATGATG ATGATGCCCT GCGCGCAGCC GTTGCCCC-3') (SEQ.ID.NO. 7) wurde anhand des "Templates" pSV-rFurink (Beispiel 1) mittels PCR ein DNA-Fragment enthaltend die Basenpaare analog der ersten 707 Aminosäuren des Furins, gefolgt von sechs Histidin-Resten und einem Translations-Terminations-Triplett hergestellt. Nach Schneiden der flankierenden SalI/EcoRI-Restriktionsschnittstellen wurde dieses Fragment in die SalI/EcoRI-Schnittstellen des oben beschriebenen Vektors phAct-ΔEcoRIupstream inseriert und so Plasmid prFurihΔTM-His erhalten. Die Nukleotid- und Aminosäuresequenz von rFurinΔTM-His ist als SEQ.ID.NO. 8 und SEQ.ID.NO. 9 wiedergegeben.

Zwischen die kodierende Region des deletierten rFurinΔTM 707 und die His-Tag-Sequenz wurde ein Spacer, bestehend aus Ala-Ala-Gly-Gly-Ala-Ala, inseriert, um eine sterische Behinderung der katalytischen Domäne des Furins zu verhindern und grössere Beweglichkeit und Funktionalität des Fusionsproteins bei Kopplung an eine Säulenmatrix zu gewährleisten. Die Inserierung der Spacer-Sequenz in prFurinΔTM-His erfolgte mittels PCR mit den Oligonukleotiden # 2325 (SEQ.ID.NO. 3) und #2820 (5'-CTAGAATTCAAT GATGATGATG ATGATGTGCAGCTCC ACCAGCTGCC CCTGCGCGCA GCCGTTGCCC C-3') (SEQ.ID.NO. 10). Analog zur Konstruktion von prFurinΔTM-His wurde dieses Fragment in die SalI/EcoRI-Stelle von Plasmid phAct-AEcoRIupstream inseriert. Das entstandene Fusionsprotein wurde prFurinΔTM-Spacer-His genannt. Die Nukleotid- und Aminosäuresequenz von rFurin-ΔTM-Spacer-His ist als SEQ.ID.NO. 11 und SEQ.ID.NO. 12 wiedergegeben.

Da das katalytische Zentrum von Furin am N-terrninalen Ende des Moleküls lokalisiert ist, kann ein noch grösserer Bereich des C-Terminus, die Cys-reiche Region, ohne signifikante Einbussen der katalytischen Funktion deletiert werden.

Es wurde ein Konstrukt hergestellt, bei dem zusätzlich zur transmembranen Domäne auch die Cys-reiche Domäne deletiert ist. Dazu wurde in die Furin kodierende Sequenz nach Nukleotid-(Aminosäure 585) ein Terminations-Triplett inseriert. Zur Konstruktion von prFurinΔCys wurde mittels PCR ein DNA-Fragment mit Oligonukleotid #2325 (SEQ.ID.NO. 3) als 5'-Primer und Oligonukleotid #2821 (5'-CTA GAATTCTAA CTGCTTTCTG GAGGTACGGG CAG-3') (SEQ.ID.NO. 15) als 3'-Primer generiert und analog zur Konstruktion von FurinΔTM in phAct-ΔEcoRIupstream inseriert. Die Nukleotid- und Aminosäuresequenz von rFurin-ΔCys ist als SEQ.ID.NO. 13 und SEQ.ID.NO. 14 wiedergegeben.

Analog zu den FurinΔTM Fusionsprotein-Konstrukten wurden rFurinΔCys-Fusionsproteine mit einer His-Tag-Sequenz nach Aminosäure 585 hergestellt. Dazu wurde ein PCR DNA-Fragment mit Oligonukleotid # 2325 (SEQ.ID.NO 3) als 5'-Primer und Oligonukleotid # 2810 (5'-CTA GAATTCTTAG TGGTGATGGT GATGATGACT GCTTTCTGGA GGTACGGGCA G-3') (SEQ.ID.NO. 16) als 3'-Primer generiert und via SalI/EcoRI-Schnittstelle in Plasmid pAct-ΔEcoRIupstream inseriert. Das entstandene Plasmid wurde prFurinΔCys-His genannt. Die Nukleotid- und Aminosäuresequenz von rFurin-ΔCys-His ist als SEQ.ID.NO. 17 und SEQ.ID.NO.18 wiedergegeben.

Die Konstruktion von rFurinΔCys-Spacer-His erfolgte mittels PCR mit Oligonukleotid # 2325 (SEQ.ID.NO. 3) als 5' Primer und Oligonukleotid # 2822 (5'-CTA GAATTCTTAG TGGTGATGGT GATGATGTGC AGCTCCACCA GCTGCACTGC TTTCTGGAGG TACGGGCAG-3') (SEQ.ID.NO. 19) als 3'-Primer. Das entstandene PCR-Fragment wurde via SalI/EcoRI-Schnittstelle in Plasmid pAct-ΔEcoRIupstream inseriert und prFurinΔCys-Spacer-His genannt. Die Nukleotid- und Aminosäuresequenz von rFurin-ΔCys-Spacer-His ist als SEQ.ID.NO. 20 und SEQ.ID.NO. 21 wiedergegeben.

Es wurde im Rahmen der Untersuchungen gefunden, daß ein Teil der Furin-Moleküle endogen einige, wenige Aminosäuren vor Aminosäure 707 gespalten wird, was zu einem löslichen sogenannten "shed"-Furin führt.

Um ausschließlich Histidin-getagte rFurin-Molekül-Spezies im Überstand stabiler CHO-Zellen zu erhalten, die Ausbeute an trägeraffinem rFurin zu erhöhen, und schließlich bessere und stärkere Interaktion des "His-Tags" mit der Ni²⁺-NTA-Matrix und verbesserte sterische Bewegungsfreiheit des rFurin-Derivates an der Matrix zu ermöglichen, wurde ein verkürztes rFurin-Derivat konstruiert, das C-terminal der Mitteldomäne, d.h.nach Aminosäure 576 deletiert und mit einem Spacer und einem 10xHis-Rest fusioniert wurde. Dazu wurde prFurinΔTM-His partiell mit SauI (an Nukleotid Position 1723-1739 in SEQ.ID.NO. 8) und vollständig mit EcoRI geschnitten und die Spacer-10xHis-Sequenz inseriert, die mittels der annealten synthetischen Oligonukleotide 5'-TGAGGGAGGT GGGGGAGGTC ATCACCACCA TCACCATCAT CATCACCATT-3' (SEQ.ID.NO. 22) und 5'-AATTAATGGTGA TGATGATGGT GATGGTGGTG ATGACCTCCC CCACCTCCC-3' (SEQ.ID.NO. 23) regeneriert wurde. Das resultierende Plasmid wurde prFurinΔCys-Spacer-10xHis genannt.

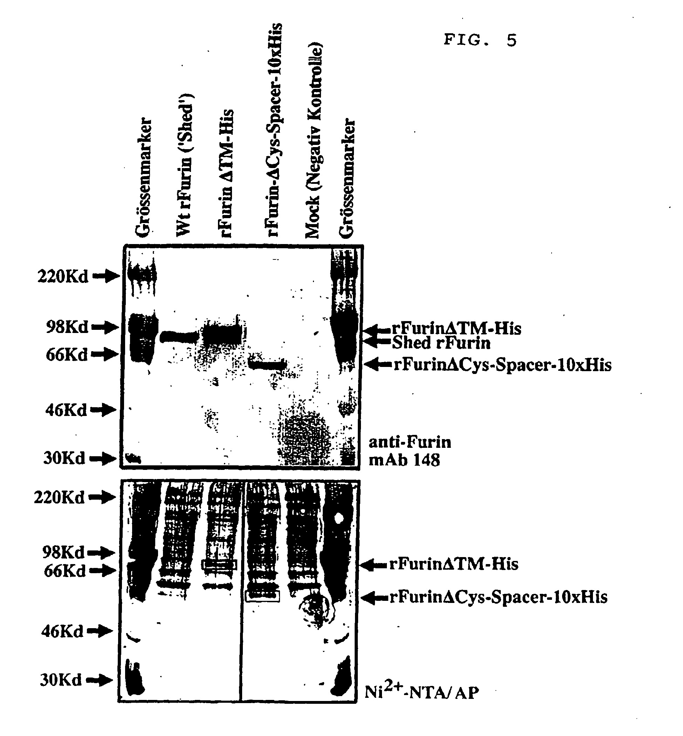

Transiente Expression von rFurinΔCys-Spacer-10xHis in 293 HEK-Zellen (ATCC CRL 1573) zeigt, daß nur eine einzige mit anti-Furin monoklonalen Antikörper reaktive Protein-Bande, und diese in der erwarteten Molekülgröße von etwa 60kD (unter Berücksichtigung der Glycosylierung) zu finden ist. Mit an Ni²⁺ NTA gekoppelter alkalischer Phosphatase wurde zudem die Bindungsfähigkeit der Histidin-getagten Moleküle nachgewiesen (Fig. 5).

Um den C-terminalen Furin-Anteil weiter zu verkürzen, wurde prFurinΔCys-Spacer-10xHis zunächst partiell mit SauI und anschließend vollständig mit MamI geschnitten. In die Schnittstelle wurde ein DNA-Fragment regeneriert aus den annealten Oligonukleotiden #3787 (5'-GGACCCCTCT GGCGAGTGGG TCCTCGAGAT TGAAAACACC AGCGAAGCCA ACAACTATGG GACGCT-3') (SEQ. ID. NO. 24) und #3788 (5'-TCAAGCGTCC CATAGTTGTT GGCTTCGCTG GTGTTTTCAA TCTCGAGGAC CCACTCGCCA GAGGGGTCC-3') (SEQ. ID. NO. 25) inseriert. Das resultierende Plasmid wurde prFurinΔΔCys-Spacer-10xHis genannt. Es kodiert für ein C-terminal deletiertes Furin, das die ersten 563 Aminosäuren von Furin enthält, gefolgt von einem Spacer, bestehend aus einem Glutaminsäure-Rest, fünf Glycin-Resten und zehn Histidin-Resten.

Der Überstand transient mit diesem Konstrukt transfizierter 293 HEK-Zellen wies im Gegensatz zu nicht-transfizierten Zellen im fluorogenen Substrat-Test Prozessierungsaktivität auf.

### Beispiel 4:

### Nachweis der enzymatischen Aktivität von rFurinΔTM-His

Der Nachweis der Furin- oder Fusionsprotein-Aktivität erfolgte mittels niedermolekularem Peptidsubstrat Boc-Arg-Val-Arg-Arg-AMC. Durch Einwirkung von Furin wird vom Substrat AMC (7-Amino-4-Methylcoumarin) abgespalten. Lösliches AMC besitzt dabei gegenüber dem Peptid-AMC fluoreszierende Eigenschaften, die zur Aktivitätsbestimmung von Furin benutzt werden können. Der fluoreszenzspektroskopische Nachweis der Furin- oder Fusionsprotein-Aktivität erfolgte bei 30°C in gerührten Quarzküvetten in einem Testvolumen von 2 ml. 1,7 ml 100mM HEPES-Puffer (pH 7,4, 1 mM CaCl₂ und 1 mM 2-Mercaptoethanol) wurden mit 0,1 ml Substratlösung (Substrat Endkonzentration im Test: 0,1 mM) und 0,2 ml Probe vermischt. Die Fluoreszenz wurde bei einer Wellenlänge von 380 nm angeregt. Nach 2 Stunden Inkubation wurde die Fluoreszenz-Emission bei 438 nm gemessen (Tabelle 1).

### Tabelle 1: Aktivitätsnaähweis von CHO-rFurinΔTM-His

Die Überstände verschiedener permanenter CHO-rFurinΔTM-His-Zellklone und als Kontrolle von CHO-rvWF/rFurin- und CHO-Zellen wurden dem AMC-Peptid-Substrat-Test unterzogen. Die Intensität der Fluoreszenz-Emission bei 438 nm gibt die Aktivität wieder.

**Tabelle 1**

| ZELLKLONE | INTENSITÄT |
|---|---|
| CHO-rFurinΔTM-His | 2524 |
| | |
| CHO-rvWF/rFurin | 444 |
| | |
| CHO | 166 |

### Beispiel 5:

### Immobilisierung von Fusionsprotein rFurinATM-His an einen Träger

Permanente CHO-Zellen, transfiziert mit gemäß Beispiel 3 konstruiertem Expressionsvektor prFurinΔTM-His, wurden in Rollerflaschen im Medium angezüchtet. Das Zellkulturmedium wurde abgezogen, die Zellen sorgfältig mit PBS gewaschen und in serumfreiem Selektionsmedium weiterinkubiert. Der Zellkulturüberstand wurde anschließend alle 24 Stunden abgezogen und durch neues Medium ersetzt. Die Überstände wurde gesammelt und vereinigt. Zur Adsorption von sekretiertem rFurinΔTM-His an Ni²⁺-NTA Agarose wurde 1 1 Zellkulturüberstand mit Imidazol bis zu einer Endkonzentration von 2 mM versetzt, 1 ml Ni²⁺-NTA Agarose zugegeben und die Suspension unter vorsichtigem Schütteln bei 4°C inkubiert. Das träger- und matrixgebundene Fusionsprotein wurde durch einfaches Absitzen oder Zenrifugation vom Überstand getrennt. Die Säulenmatrix mit gebundenem rFurinΔTM-His wurde in 10 ml Puffer A (300mM NaCl, 10% Glycerin, 1mM β-Mercaptoethanol, 2mM Imidazol, 5 mM Hepes pH 7,0, 2 mM CaCl₂, 2 mM MgCl₂) resuspendiert und anschließend für 10 min bei 600 g zentrifugiert. Das Pellet wurde im gleichen Puffervolumen resuspendiert und eine Chromatographie-Säule damit beladen. Die Säule wurde anschließend mit 5 ml Puffer A, enthaltend 2 mM Imidazol, gewaschen und mit 5 ml serumfreiem Selektionsmedium equilibriert.

Die Elution von rFurinΔTM-His erfolgte mit Puffer A enthaltend 200 mM Imidazol. Die enzymatische Aktivität von rFurinΔTM-His erfolgte analog zu Beispiel 4. Das Ergebnis ist in Tabelle 2 zusammengefaßt. Es zeigte sich, daß rFurinΔTM-His an die Säulenmatrix bindet und als aktives Molekül wieder von der Säule eluierbar ist.

### Tabelle 2: Die Aktivität von rFurinΔTM-His vor Bindung an die Ni²⁺-NTA-Matrix sowie nach Elution von der Matrix

**Tabelle 2**

| **ÜBERSTÄNDE** | **INTENSITÄT** |
|---|---|
| Rollerüberstand | |
| CHO-rFurinΔTM-His | 2664 |
| | |
| Equilibrierung | - |
| | |
| 1.Waschen | 340 |
| | |
| 2.Waschen | 280 |
| | |
| 3.Waschen | 121 |
| | |
| Eluat | 2820 |

Die Aktivität von CHO-rFurinΔTM-His-Zellen aus Zellkulturüberständen vor der Bindung an die Säulenmatrix, der einzelnen Waschfraktionen sowie des Eluats wurden mittels AMC-Peptid-Substrat-Test bestimmt.

### Beispiel 6

### Aktivierung von pro-vWF an immobilisiertem Fusionsprotein

Die gemäß Beispiel 5 hergestellte rFurinΔTM-His-Ni²⁺-Säule wurde zur Prozessierung von rpro-vWF zu rvWF eingesetzt. Dazu wurden über die Säule 20 ml auf 2 mM Imidazol eingestellter, serumfreier Zellkulturüberstand von CHO-rvWF-Zellen geleitet. Aliquote des Eluats wurden über Western Blot (gemäß Beispiel 2) analysiert. Proben des Zellkulturüberstandes, die keiner Proteasebehandlung unterzogen wurden, dienten als Kontrolle. Proben von Zellkulturüberständen von CHO-rvWF zeigten einen zu etwa 40% unprozessierten pro-vWF, wogegen in Proben des Säuleneluats nur vollständig prozessierten vWF nachgewiesen wurde.

### Beispiel 7:

### Reinigung von His-getagten rFurin-Fusionsproteinen an Ni²⁺-NTA-Matrix

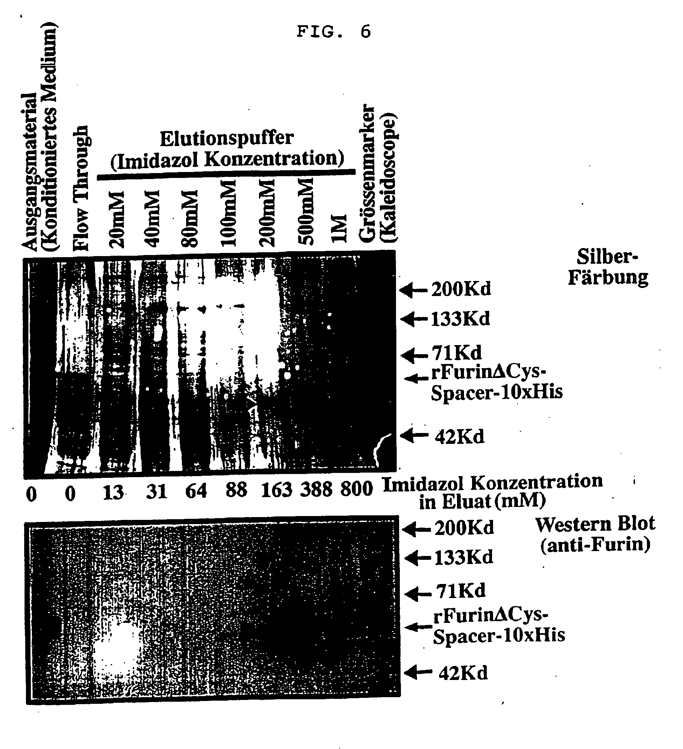

Stabile CHO-Zellklone, die rFurinΔCys-Spacer-10xHis sezernieren, wurden gemäß Beispiel 2 hergestellt und eine mit frischem, serumfreien Kulturmedium äquilibrierte Ni²⁺-NTA-Matrix mit konditioniertem Medium solcher Klone beladen. Adsorbierte Proteine wurden mit Elutionspuffer, enthaltend steigende Konzentrationen an Imidazol, von der Säule eluiert und Aliquote der einzelnen Fraktionen im SDS-PAGE aufgetrennt. Die Proteine wurden anschließend mit Silber gefärbt bzw. mittels Western-Blot-Analyse detektiert (Fig. 6). Die Silberfärbung zeigte, daß eine Vielzahl von Proteinen, enthalten im konditionierten Medium an die Matrix binden können, jedoch bereits bei niedriger Imidazol-Konzentration wieder eluiert werden. Bei einer Imidazol-Konzentration bis etwa 100mM wurden Kontaminanten von der Säule eluiert, wogegen Fraktionen, die bei höherer Imidazol-Konzentration eluiert wurden, nur noch ein Protein enthielten. Dieses Protein wurde im Western-Blot mit anti-Furin-Antikörpern als rFurinΔCys-Spacer-10xHis identifiziert.

Der Einfluß von Imidazol auf die Furin-Aktivität wurde bestimmt. Dazu wurde ein rFurinΔCys-Spacer-10xHis-haltiger CHO-Überstand mit steigenden Mengen von Imidazol versetzt und die Proben anschließend einem fluorogenen Substrat-Test (gemäß Beispiel 4) unterzogen. Tabelle 3 zeigt, daß mit steigender Imidazol-Konzentration die Fähigkeit identischer rFurin-Derivat-Mengen zur Umsetzung des fluorogenen Substrats sukzessive reduziert wird und die Anwesenheit von Imidazol die Furin-Aktivität somit inhibiert.

**TABELLE 3: Inhibition der Furin-Aktivität in Anwesenheit von Imidazol**

| **rFurin haltiger Überstand oder Medium** | **Imidazol Konzentration in Probe (mM)** | **Furin-Aktivität (Gemessene Fluoreszenz Units)** |
|---|---|---|
| CHO-rFurin | 0 | >1000 |
| | 50 | 818 |
| | 100 | 587 |
| | 200 | 469 |
| | 500 | 24 |
| | | |
| CHO (ohne rFurin) | 0 | 43 |
| | | |
| Medium (ohne CHO) | 0 | 32 |

Imidazol-haltige rFurin-Derivat-Fraktionen wurden daher gegen 20mM Hepes pH 7,0, 1mM CaCl₂, 1mM β-Mercaptoethanol über Nacht bei 4°C dialysiert. Bei einem Vergleich der Proben vor und nach Dialyse zeigt sich, daß die Aktivität von rFurinΔCys-Spacer-10xHis nach Entfernen des Imidazols durch Dialyse von 408 auf >1000 Units (Fraktion mit 333 mM Imidazol eluiert) bzw. von 24 auf >1000 Units (Fraktion mit 780 mM Imidazol eluiert) wiederhergestellt werden konnte.

Die schnelle und sehr saubere Reinigung der Histidin-getagten rFurin-Derivate mittels der beschriebenen Methode ermöglicht somit eine großtechnische Prozeßentwicklung, z.B. gereinigte Pro-rvWF Moleküle (oder andere Target-Proteine) durch Epitop-getagte rFurin-Derivate in Lösung zu prozessieren. Nach vollständiger Prozessierung können die beiden Reaktionspartner mittels Ni²⁺-NTA-Matrix selektiv voneinander getrennt werden, wobei das vollständig prozessierte Substrat im Durchfluß entfernt und das rFurin-Derivat an die Matrix gebunden wird. Nach Elution von der Säulenmatrix und Dialyse steht das rFurin-Derivat für eine neuerliche Prozessierungsreaktion zur Verfügung. Auf diese Weise ist es möglich, mittels wiederholter solcher rFurin-Derivat "Recycling"-Schritte mit verhältnismäßig wenig rFurin-Derivat in vitro, eine vergleichsweise große Menge von Pro-rvWF zu prozessieren.

### Beispiel 8:

### Nachweis der enzymatischen Aktivität von immobilisiertem rFurinΔCys-Spacer-10xHis

Der Nachweis der funktionellen Aktivität von immobilisiertem rFurinΔCys-Spacer-10xHis erfolgte durch Prozessierung des Furinspezifischen, fluorogenen Boc-Arg-Val-Arg-Arg-AMC-Substrats (gemäss Beispiel 4).

Dazu wurde 1 ml in Serum-freiem Zellkulturmedium äquilibrierte Ni²⁺-NTA-Matrix (Quiagen) bei 4°C mit 10ml Imidazol-freiem CHO-rFurinΔCys-Spacer-10xHis-Zellkulturüberstand, bzw. als NegativKontrolle mit konditioniertem Medium nicht-manipulierter CHO-Zellen, beladen. Dann wurde die Matrix bei Raumtemperatur dreimal mit Serum-freiem Zellkulturmedium gewaschen und bei 30°C anschließend das fluorogene Substrat mit immobilisiertem rFurinΔCys-Spacer-10xHis in Kontakt gebracht. Als Positiv-Kontrolle wurde CHO-rFurinΔCys-Spacer-10xHis-Zellkulturüberstand auf seine Fähigkeit getestet, fluorogenes Substrat in Lösung umzusetzen.

Äquivalente Mengen (200µl) des Ausgangs-Zellkulturüberstandes, der Durchfluß-Fraktionen und der Waschschritte wurden, wie in Beispiel 4 beschrieben, auf ihren rFurin-Derivat-Inhalt mittels fluorogenem Substrat-Test untersucht. Die ermittelten Substrat-Spaltungskapazitäts-Werte wurden mit den Werten jener Substrathaltigen Lösungen verglichen, die mit der rFurin-Derivat gekoppelten Säulenmatrix exponiert waren.

Es zeigte sich, daß bei entsprechender Exposition Ni²⁺NTA-Matrix gebundenes rFurinΔCys-Spacer-10xHis mehr als 1000 Einheiten fluorogenen Substrats umsetzte und damit vergleichbar der Umsetzung des fluorogenen Substrats durch nicht-immobilisiertes rFurin-Derivat war. Mit konditioniertem Medium unmanipulierter CHO-Zellen "beladene" Säulenmatrix führte zu keiner Spaltung des fluorogenen Substrates.

Dies zeigte, daß rFurinΔCys-Spacer-10xHis nicht nur in Lösung, sondern auch im immobilisierten Zustand proteolytische Aktivität vermittelt. Abhängig vom Substrat (z.B. von Proproteinen rFIX, rFX, rvWF, rPC, rPS, etc.) ist es unter Umständen sinnvoll, die sterische Bindung des rFurin-Derivats an die Matrix durch entsprechende Variation des Spacers (z.B. Verlängerung des Spacers und/oder Wahl der Spacer-Aminosäuren, um z.B. starres Abstehen von der Matrix oder größere Beweglichkeit zu erwirken) zu optimieren und so die Prozessierung noch zu verbessern bzw. für ein gegebenes Substrat zu optimieren. Alternativ können Säulenmatrices verwendet werden, bei denen das gebundene rFurin-Derivat über einen längeren Säulenarm mit der Matrix verbunden ist, wie beispielsweise bei Tentakelgelen (siehe Beispiel 10).

### Beispiel 9:

### In vitro-Prozessierung von gereinigtem rvWF-Vorläufer durch gereinigtes rFurinΔCys-Spacer-10xHis

pro-rvWF-Vorläuferprotein wurde bis zur Homogenität gereinigt (mit einem rvWF-Antigen:Gesamt-Protein-Verhältnis von 2) und 10µg pro-rvWF mit 1340 U gereinigtem rFurinΔCys-Spacer-10xHis (siehe Beispiel 7) in Puffer (0,87mM CaCl₂, 17mM Hepes pH 7,0, 0,87mM Mercaptoethanol) bei 37°C inkubiert. Die Negativ-Kontrolle, ein unmanipulierter CHO-Zellkulturüberstand (ohne rFurinΔCys-Spacer-10xHis) wurde auf der Ni²⁺ NTA-Matrix in analoger Weise aufbereitet.

Aliquote der Reaktionsgemische wurden zu Testbeginn als auch zu unterschiedlichen Zeitpunkten entnommen und weggefroren. Nach Beendigung der Inkubation wurden diese Aliquote unter reduzierten und denaturierten Bedingungen im SDS-PAGE aufgetrennt und im Western-Blot rvWF-reaktives Material (gemäß Beispiel 1) visualisiert.

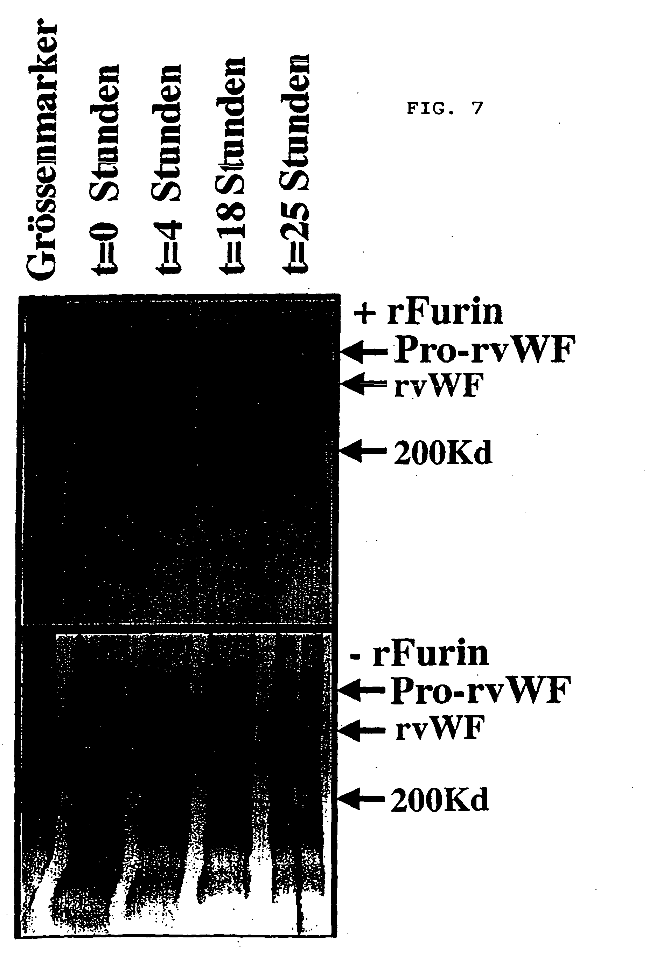

Figur 7 zeigt die Prozessierung von gereinigtem pro-rvWF durch gereinigtes rFurinΔCys-Spacer-10xHis unter definierten Bedingungen. Während in Anwesenheit des rFurin-Derivats pro-vWF bereits nach vier Stunden vollständig prozessiert war (Figur 7, oben), wurde bei Abwesenheit des rFurin-Derivats keine Prozessierung beobachtet (Figur 7, unten). Bei längerer Inkubation mit dem rFurin-Derivat wurden prozessierte rvWF-Moleküle nicht weiter proteolytisch abgebaut und die molekulare Integrität der reifen rvWF-Moleküle blieb über den gesamten Zeitraum der rFurin-Exposition stabil (Figur 7, oben).

### Beispiel 10:

### Prozessierung von Pro-Proteinen mittels an Chelat-Tentakelgelimmobilisiertem rFurinΔCys-Spacer-10xHis

Um die Interaktion von zu spaltendem Substrat mit Säulen-gebundenem rFurin-Derivat gegebenenfalls zu verbessern, wurde untersucht, ob als Säulen-Matrix statt Ni²⁺-NTA-Agarose in einem experimentellen Ansatz Fractogel EMD^{®}-Tentakelgel (Fa. Merck) verwendet werden kann. Da die Metallionen hierbei im Vergleich zur Ni²⁺-NTA-Agarose räumlich weiter von der eigentlichen Säulen-Matrix entfernt sind, könnte eine verbesserte sterische Zugänglichkeit des gebundenen rFurin-Derivats ermöglicht werden. In dem vorliegenden Ansatz wurde Pro-Protein (pro-vWF) durch Tentakelgel gebundenes rFurinΔCys-Spacer-10xHis prozessiert:

Das Fractogel EMD^{®}-Tentakelgel wurde nach Herstellervorschrift mit Ni²⁺-Ionen beladen und mit frischem Serum-freien Zellkulturmedium äquilibriert. Anschließend wurde die Säule mit Serumfreiem CHO-rFurinΔCys-Spacer-10xHis-Überstand beladen. Waschschritte erfolgten durch Serum-freies Zellkulturmedium, enthaltend steigende Imidazol-Konzentrationen bis 40mM. Anschließend wurde das Pro-Protein-Substrat als Serum-freier CHO-Überstand über die Säule geleitet. Mittels Western-Blot-Analyse mit spezifischem vWF-Antiserum wurde die Prozessierung von Pro-Protein zu Protein im Durchfluß der Säule nachgewiesen.

### Beispiel 11:

### Prozessierung von einzelkettigem rFX zu rFX leichte/schwere Kette durch rFurinΔTM-His und rFurinΔCys-Spacer-His

### a. Herstellung des rFX-Expressionsvektors

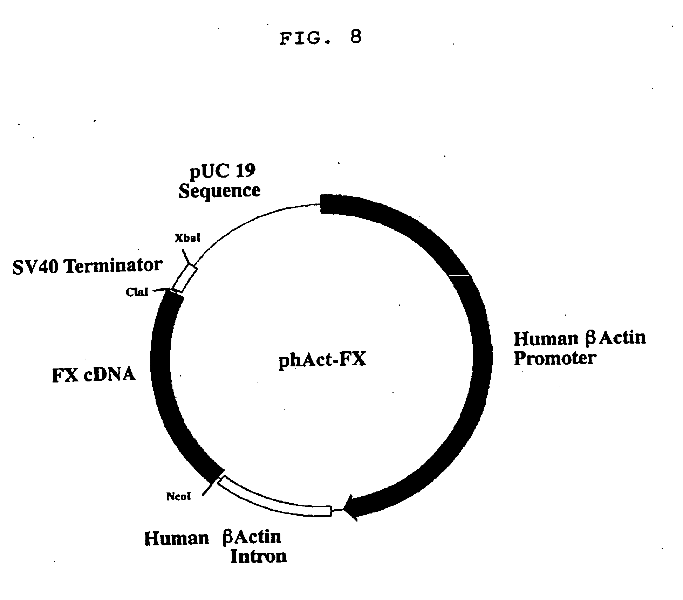

Zur Herstellung von rekombinantem FX (rFX) wurde die cDNA von FX aus einer humanen Leber Lambda-cDNA-Bank, wie von Messier et al. beschrieben (1991, Gene 99:291-294), isoliert. Aus einem positiven Klon wurde mittels PCR mit Oligonukleotid #2911 (5'-ATTACTCGAGAAGCTTACCATGGGGCGCCCACTG-3') (SEQ.ID. NO. 26) als 5-Primer und Oligonukleotid #2912 (5'-ATTACAATTGCTGCAGGGATCCAC-3') (SEQ.ID.N0.27) als 3'-Primer ein DNA-Fragment amplifiziert, das die 1,467kB FX-kodierende Sequenz sowie 39bp der 3'-nicht-translatierten Region, flankiert von einer XhoI-Schnittstelle, am 5'-Ende und einer MfeI-Schnittstelle am 3'-Ende enthält. Zusätzlich wurde durch den Primer #2911 die Sequenz ACC vor das ATG des FX eingebaut, so daß eine optimale Kozak-Transkriptions-Sequenz ensteht. Anschließend wurde dieses PCR-Produkt als XhoI/MfeI-Fragment in den mit SalI und EcoRI geschnittenen Expressionsvektor phAct kloniert. Das resultierende Expressionsplasmid wurde mit phAct-rFX bezeichnet (Figur 8).

Der Expressionsvektor phAct umfaßt den humanen beta-Actin-Promotor, 78bp 5'UTR sowie das Intron, eine multiple Klonierungsschnittstelle und die SV40-Polyadenylierungstelle.

### b. Expression von rFX in CHO-Zellen

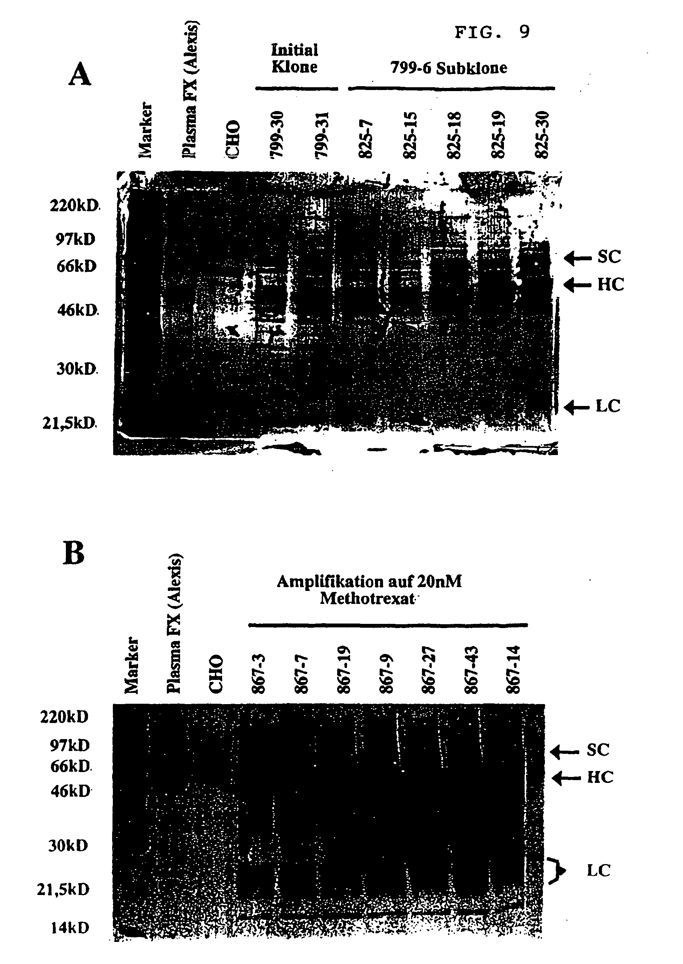

Zur Etablierung einer stabilen rFX-exprimierenden Zellinie wurden, wie in Beispiel 2 beschrieben, dhfr-defiziente CHO-Zellen mit dem Expressionsplasmid phAct-rFX und dem Selektionsmarkerplasmid pSV-dhfr co-transfiziert. Für alle weiteren Expressions- und Funktionsanalysen wurden die Zellkulturen mit serumfreiem Selektionsmedium in Anwesenheit von 10 µg/ml Vitamin K 24 Stunden lang inkubiert. Die Expression von rFX in den resultierenden Zellklonen wurde anhand der Antigenmenge (ELISA) nachgewiesen und das rekombinante Protein anschließend mit SDS-PAGE (wie in Beispiel 2 beschrieben) charakterisiert (Figur 9 A und B). In den Initialklonen und Subklonen liegt, wie im Western-Blot erkennbar (Figur 9 A), das rekombinante FX-Protein in der Form einer leichten Kette (LC) von 22kD und einer schweren Kette (HC) von 45kD vor, die identisch mit dem plasmatischen Faktor X-Protein sind. Zusätzlich ist eine Proteinbande bei 75kD zu erkennen, die dem einzelkettigen (SC) Molekül entspricht und deren Präsenz in FX-transfizierten CHO-Zellen (Wolf et al. J. Biol. Chem. 266:13726-13730, 1991) sowie in humanem Plasma (Fair et al. Blood 64:194-204, 1984) beschrieben wurde. Zur Herstellung von hochexprimierenden Klonen wurden die Initialklone mit steigenden Mengen Methotrexat amplifiziert und anschließend bis zur Stabilisierung subkloniert. Die Expression konnte von ca. 200-500 ng/10E6-Zellen bzw. 1µg/ml auf 30-50µg/10E6-Zellen bzw. 100µg/ml pro 24 Stunden gesteigert werden. Die Western-Blot-Analyse dieser hochexprimierenden Zellklonüberstände (Figur 9 B und Figur 9 A Spur 2) zeigt eine Anreicherung des einzelkettigen rFX-Moleküls sowie die Anwesenheit zusätzlicher Formen der leichten Kette. Neben der 22kD-Form der leichten Kette, die der plasmatischen Form entspricht (vollständig carboxyliert ohne Propeptid) liegen zumindest zwei weitere Varianten der leichten Kette mit ca. 21kD und 20kD vor. Die Heterogenität der leichten Kette in diesen Klonen konnte mittels einer N-terminalen Sequenzierung des rekombinanten Materials auf eine unvollständige Abspaltung des Propeptids (ca. 50% des rFX-Materials) sowie auf Untercarboxylierung (ca. 50% des rFX) zurückgeführt werden. Das 21kD-Protein ist eine untercarboxylierte propeptid-haltige und das 20kD-Protein eine untercarboxylierte pro-peptid-freie Form der leichten Kette.

### c. In vitro-Abspaltung des Propeptides und Prozessierung des einzelkettigen rFX in rFX leichte/schwere Kette durch rFurinΔTM-His oder rFurinACys-Spacer-His.

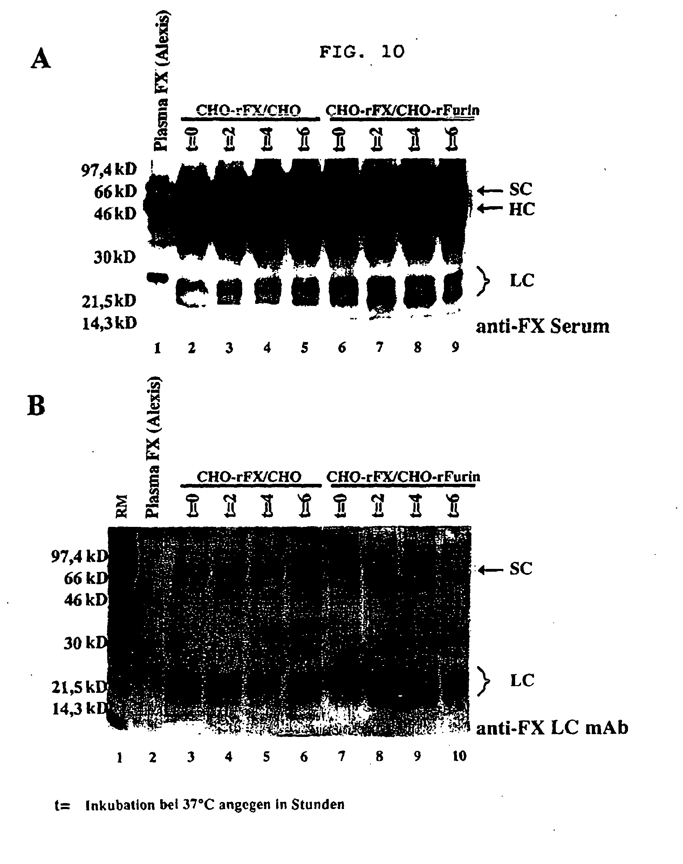

Aufgrund der Sequenzhomologie der Spaltstellen zwischen Faktor X Propeptid/N-Terminus der leichten Kette (RVTR/A) und zwischen leichter/schwerer Kette (RRKR/S) mit der Konsensus-Furin-Erkennungsequenz (RX^{K/R}R/X) bestand die Möglichkeit, die Prozessierung sowohl einzelkettiger als auch Propeptid-haltiger rFX-Moleküle durch rFurin in vitro zu verbessern. Zellkulturüberstände von CHO-rFX und CHO-rFurinΔTM-His (beschrieben in Beispiel 3) sowie CHO-rFX und CHO (als Negativkontrolle) wurden im Verhältnis 1:1 gemischt und bei 37°C inkubiert. Aliquote der Reaktionsansätze wurden vor Inkubation (t=0) und nach verschiedenen Inkubationszeiten (t=2, 4, 6 Stunden) mittels Western-Blot-Analyse auf prozessierten rFX getestet (Figur 10). Der Nachweis von rFX in den Zellkulturüberständen erfolgte mittels eines anti-humanen FX-Antiserums (Figur 10 A) und eines monoklonalen Antikörpers spezifisch für die leichte Kette des FX (Figur 10 B).

Im Gegensatz zu dem CHO-rFX/CHO-Gemisch weist das CHO-rFX/CHOrFurinΔTM-His schon nach zwei Stunden Inkubation bei 37°C (Figur 10 A, Spur 7; Figur 10 B, Spur 8) eine fast vollständige Prozessierung vor. Einzelkettiger rFX ist zum Großteil in die leichte und schwere Kettenform umgesetzt. Im Bereich der leichten Kette wurden nur noch die prozessierten Pro-Peptid-freien Formen von 22kD (carboxylierte Form) und 20kD (untercarboxylierte Form) in einem Verhältnis von ca. 50:50 gefunden. Die korrekte Abspaltung der Pro-Sequenz zwischen Arg-1 und Ala+1 und die Homogenität des N-Terminus der leichten Kette wurde mittels N-terminaler Sequenzierung festgestellt. Im Kontrollexperiment, in dem CHO-rFX mit CHO-Überständen gemischt wurde, ist auch nach einer 6-stündigen Inkubation keine Veränderung des rFX-Bandenmusters zu erkennen. Damit wurde nachgewiesen, daß rFurinΔTM-His im Überstand von CHO-Zellen biologisch aktiv ist und sowohl die Prozessierung des Pro-Peptids als auch der schweren/leichten Kette von rFX durchführen kann. Die Prozessierung von rFX wurde auch mit CHO-rFurinΔCys-Spacer-His-Konstrukten nachgewiesen.

### d. Aktivität des in vitro prozessierten CHO-rFX

Die Überstände aus dem unter c.) angeführten Experiment wurden anschließend mittels FX-Coatest-Kit (Fa. Chromogenix) auf FX-Aktivität getestet. Dazu wurden 50µl von jedem Überstand mit 50µl FX-defizientem, humanen Plasma versetzt und laut Protokoll des Herstellers rFX mit Schlangengift (RVV) in Anwesenheit von CaCl₂ zu rFXa umgesetzt; rFXa hydrolysiert anschließend das chromogene Substrat (S-2337) und führt zur Freisetzung des gelbfarbigen Paranitroanilin. Da die Menge an rFXa und die Farbintensität proportional zueinander sind, kann anhand einer Eichgerade, interpoliert aus Werten einer Plasma-Verdünnungsreihe, die Menge zu rFXa aktivierbarem rFX/ml Zellkulturüberstand bestimmt werden. Mit diesen Ergebnissen und der bekannten rFX-Antigenmenge (ELISA-Daten) kann der Anteil von dem in Faktor Xa aktiviertem rFaktor X in % ausgerechnet werden. Die Ergebnisse sind in Tabelle 4 dargestellt.

**TABELLE 4**

| *Probe* | *0D bei 405nm* | *Aktivität mU*/*ml* | *ELISA µg*/*ml* | % *Funktioneller FX* |
|---|---|---|---|---|
| Plasma 100% | 0,829 | 991,1 | | |
| Plasma 50% | 0,434 | 515,7 | | |
| Plasma 25% | 0,217 | 254,5 | | |
| Plasma 12,5% | 0,108 | 123,3 | | |
| Plasma 6,25% | 0,054 | 58,3 | | |
| Puffer | 0,001 | 0,0 | | |
| CHO/CHO-rFurin | | | | |
| t=0 | 0,008 | 3,0 | 0,0 | |
| t=2 | 0,001 | 0,0 | 0,0 | |
| t=4 | 0,010 | 5,4 | 0,0 | |
| t=6 | 0,006 | 0,6 | 0,0 | |
| CHO-rFX/CHO | | | | |
| t=0 | 0,170 | 197,9 | 19,9 | 24,9 |
| t=2 | 0,131 | 151,0 | 19,9 | 19,0 |
| t=4 | 0,153 | 177,5 | 19,9 | 22,3 |
| t=6 | 0,163 | 189,5 | 19, 9 | 23,8 |
| CHO-rFX/CHO-rFurin | | | | |
| t=0 | 0,151 | 175,1 | 19,9 | 22,0 |
| t=2 | 0,235 | 276,2 | 19,9 | 34,7 |
| t=4 | 0,260 | 306,3 | 19,9 | 38,5 |
| t=6 | 0,292 | 344,8 | 19,9 | 43,3 |

Um unspezifische, proteolytische Aktivität in CHO- und CHOrFurinΔTM-His-Überständen auszuschließen, wurde das Gemisch dieser beiden Zellkulturüberstände ebenso untersucht. Die geringen OD-Werte (weniger als 7% der proteolytischen Aktivität), die in diesen Überständen gefunden wurden, sind photometrische Schwankungen und liegen innerhalb der Standardabweichung. Signifikante unspezifische proteolytische Aktivität in CHO-Überständen, die den Test beeinflussen könnte, wurde somit ausgeschlossen.

CHO-rFX inkubiert mit CHO-Überständen (ohne rFurin) als Kontrolle zeigte auch nach 6 Stunden keinen wesentlichen Anstieg der rFXa-Aktivität, die aufgrund der experimentellen Schwankungen zwischen 150-200mU/ml lag und 19-25% funktionellem rFX entsprach. Wurde im Vergleich dazu CHO-rFX mit CHO-rFurinΔTM-His inkubiert, so entstand schon nach zwei Stunden eine signifikante Steigerung der rFX-Aktivität, die nach 6 Stunden 344mU/ml bzw. 43% funktionellen Anteil des CHO-rFX erreichte. Diese Daten korrelieren sehr gut mit der Anwesenheit von 50% der aktiven bzw. carboxylierten leichten Kette mit 22kD und 50% der inaktiven bzw. untercarboxylierten leichten Kette mit 20kD in diesen behandelten Überständen (Figur 10).

Damit wurde nachgewiesen, daß durch in vitro-Prozessierung von CHO-rFX aus hochexprimierenden Klonen mittels rFurin-Derivat der Anteil von zu funktionellem rFXa aktivierbaren rFX wesentlich verbessert wird.

## Patentansprüche

1. Fusionsprotein, **dadurch gekennzeichnet, dass** es ein C-terminal deletiertes Furin fusioniert mit einer heterologen Sequenz, die die Adsorption des Furins an einen Träger ermöglicht, umfasst, wobei das Fusionsprotein an einem Träger immobilisiert und proteolytisch aktiv ist.

2. Fusionsprotein nach Anspruch 1, **dadurch gekennzeichnet, dass** die C-terminale Deletion die cytoplasmatische und transmembrane Region, und gegebenenfalls die Cys-reiche Region umfasst.

3. Fusionsprotein nach Anspruch 2, **dadurch gekennzeichnet, dass** die heterologe Sequenz ein Protein, ein Polypeptid oder ein Affinitätspeptid ist.

4. Fusionsprotein nach einem der Ansprüche 1 bis 3, **dadurch gekennzeichnet, dass** das Protein oder Polypeptid abgeleitet ist von β-Galaktosidase, Glutathion-S-transferase, c-myc-Produkt, Avidin oder Lysin-bindende Kringeldomäne von Plasmaproteinen, wie Plasminogen.

5. Fusionsprotein nach Anspruch 3, **dadurch gekennzeichnet, dass** das Affinitätspeptid ein Tag-Peptid ist, vorzugsweise ein His-Tag.

6. Fusionsprotein nach Anspruch 5, **dadurch gekennzeichnet, dass** das Affinitätspeptid aus mehreren, vorzugsweise 3 bis 20, besonders bevorzugt 6 bis 15 aufeinanderfolgenden Histidin-Resten besteht.

7. Fusionsprotein nach Anspruch 6, **dadurch gekennzeichnet, dass** es FurinΔTM-His oder FurinΔCys-His, vorzugsweise ein FurinΔTM-His gemäß Seq.ID 9 oder ein FurinΔCys-His gemäß Seq.ID No. 18 ist.

8. Fusionsprotein nach einem der Ansprüche 1 bis 7, **dadurch gekennzeichnet, dass** zwischen der Furin-Sequenz und der heterologen Sequenz ein Spacer insertiert ist.

9. Fusionsprotein nach Anspruch 8, **dadurch gekennzeichnet, dass** es FurinΔTM-Spacer-His oder FurinΔCys-Spacer-His ist.

10. Fusionsprotein nach Anspruch 9, **dadurch gekennzeichnet, dass** es ein FurinΔTM-Spacer-His gemäß Seq.ID No.12 oder FurinΔCys-Spacer-His gemäß Seq.ID. No. 21 ist.

11. Fusionsprotein nach einem der Ansprüche 1 bis 10, **dadurch gekennzeichnet, dass** es Affinität zu einem festen Träger besitzt.

12. Fusionsprotein nach Anspruch 11, **dadurch gekennzeichnet, dass** der Träger einen Antikörper oder ein (Schwer-)Metallion umfasst.

13. Fusionsprotein-Komplex enthaltend ein Fusionsprotein nach einem der Ansprüche 1 bis 12 adsorbiert an einen festen Träger.

14. Verfahren zur Herstellung von Proteinen aus Pro-Proteinen, **dadurch gekennzeichnet, dass** ein Pro-Protein durch ein Fusionsprotein nach einem der Ansprüche 1 bis 12 proteolytisch gespalten ist.

15. Verfahren nach Anspruch 14, **dadurch gekennzeichnet, dass** das Pro-Protein durch ein Fusionsprotein nach einem der Ansprüche 1 bis 12 außerhalb von Zellen gespalten wird.

16. Verfahren nach Anspruch 15, **dadurch gekennzeichnet, dass** das Pro-Protein in Lösung vorliegt.

17. Verfahren nach Anspruch 14, **dadurch gekennzeichnet, dass** der Träger ein Schwermetall oder ein Antikörper ist.

18. Verfahren nach einem der Ansprüche 14 bis 17, **dadurch gekennzeichnet, dass** das Pro-Protein eine inaktive Vorstufe eines Plasmaproteins, vorzugsweise Faktor IX, von Willebrand-Faktor, Faktor VII, Faktor X, Faktor XI, Faktor V, Protein C, Protein S, Albumin oder eines viralen Proteins, vorzugsweise HIV gp160 und Influenzavirus HA, ist.

19. Verfahren zum Herstellen eines Fusionsprotein-Komplexes nach Anspruch 13, **dadurch gekennzeichnet, dass** ein Fusionsprotein wie in einem der Ansprüche 1 bis 12 definiert mit einem festen Träger, welcher das Fusionsprotein binden kann, in Kontakt gebracht wird.

20. Verfahren nach Anspruch 19, **dadurch gekennzeichnet, dass** eine Lösung enthaltend ein Fusionsprotein mit einem Träger in Kontakt gebracht wird.

21. Verfahren nach Anspruch 20, **dadurch gekennzeichnet, dass** die Lösung ein Kulturüberstand von rekombinanten Zelllinien ist.

22. Verfahren nach Anspruch 20, **dadurch gekennzeichnet, dass** die Lösung gereinigtes Fusionsprotein enthält.

23. Verfahren nach einem der Ansprüche 19 bis 22, **dadurch gekennzeichnet, dass** der feste Träger ausgewählt ist aus einem immobilisierten (Schwer-)Metallion, einem immobilisierten Antikörper oder einem immobilisierten Peptid oder Polypeptid.

24. Verwendung eines Fusionsproteins nach einem der Ansprüche 1 bis 12 für ein Verfahren nach einem der Ansprüche 14 bis 18.

25. Verwendung eines Fusionsprotein-Komplexes nach Anspruch 13 für ein Verfahren nach einem der Ansprüche 14 bis 18.

26. Verwendung eines Fusionsproteins nach einem der Ansprüche 1 bis 12 zur Herstellung eines Proteins aus Pro-Protein, insbesondere von Faktor IX aus pro-Faktor IX, von Willebraäd-Faktor aus pro-von Willebrand-Faktor, Faktor X aus pro-Faktor X, Faktor XI aus pro-Faktor XI, Faktor VII aus pro-Faktor VII, Faktor V aus pro-Faktor V, Protein C aus pro-Protein C und Albumin aus pro-Albumin.

27. Verwendung eines Fusionsprotein-Komplexes nach Anspruch 13 zur Herstellung eines Proteins aus Pro-Protein, insbesondere von Faktor IX aus pro-Faktor IX, von Willebrand-Faktor aus pro-von Willebrand-Faktor, Faktor X aus pro-Faktor X, Faktor XI aus pro-Faktor XI, Faktor VII aus pro-Faktor VII, Faktor V aus pro-Faktor V, Protein C aus pro-Protein C und Albumin aus pro-Albumin.

28. Verwendung eines Verfahrens nach einem der Ansprüche 14 bis 18. zur Herstellung eines Proteins aus Pro-Protein, insbesondere von Faktor IX aus pro-Faktor IX, von Willebrand-Faktor aus pro-von Willebrand-Faktor, Faktor X aus pro-Faktor X, Faktor XI aus pro-Faktor XI, Faktor VII aus pro-Faktor VII, Faktor V aus pro-Faktor V, Protein C aus pro-Protein C und Albumin aus pro-Albumin.

## Claims

1. A fusion protein, **characterised in that** it comprises a C-terminally deleted furin fused to a heterologous sequence that enables adsorption of the furin to a carrier, said fusion protein being immobilized to a carrier and proteolytically active.

2. The fusion protein according to claim 1, **characterised in that** the C-terminal deletion comprises the cytoplasmatic and transmembrane regions, and optionally the Cys-rich region.

3. The fusion protein according to claim 2, **characterised in that** the heterologous sequence is a protein, a polypeptide or an affinity peptide.

4. The fusion protein according to any one of claims 1 to 3, **characterised in that** the protein or polypeptide is derived from β-galactosidase, glutathione-S-transferase, c-myc-product, avidine or lysine-binding kringel domain of plasma proteins, such as plasminogen.

5. The fusion protein according to claim 3, **characterised in that** the affinity peptide is a tag-peptide, preferably a His-tag.

6. The fusion protein according to claim 5, **characterised in that** the affinity peptide is comprised of several, preferably 3 to 20, particularly preferably 6 to 15, consecutive histidine residues.

7. The fusion protein according to claim 6, **characterised in that** it is furinΔTM-His or furinΔCys-His, preferably a furinΔTM-His according to Seq. ID No. 9 or a furinΔCys-His according to Seq. ID No. 18.

8. The fusion protein according to any one of claims 1 to 7, **characterised in that** a spacer is inserted between the furin sequence and the heterologous sequence.

9. The fusion protein according to claim 8, **characterised in that** it is furinΔTM-spacer-His or furinΔCys-spacer-His.

10. The fusion protein according to claim 9, **characterised in that** it is a furinΔTM-spacer-His according to Seq. ID No. 12 or furinΔCys-spacer-His according to Seq. ID No. 21.

11. The fusion protein according to any one of claims 1 to 10, **characterised in that** it comprises an affinity to a solid carrier.

12. The fusion protein according to claim 11, **characterised in that** the carrier comprises an antibody or a (heavy) metal ion.

13. A fusion protein complex comprising a fusion protein according to any one of claims 1 to 12 adsorbed to a solid carrier.

14. A method of preparing proteins from proproteins, **characterised in that** a proprotein is proteolytically cleaved by a fusion protein according to any one of claims 1 to 12.

15. The method according to claim 14, **characterised in that** the proprotein is cleaved by a fusion protein according to any one of claims 1 to 12 externally of cells.

16. The method according to claim 15, **characterised in that** the proprotein is present in solution.

17. The method according to claim 14, **characterised in that** the carrier is a heavy metal or an antibody.

18. The method according to any one of claims 14 to 17, **characterised in that** the proprotein is an inactive precursor of a plasma protein, preferably factor IX, von Willebrand factor, factor VII, factor X, factor XI, factor V, protein C, protein S, albumin or a viral protein, preferably HIV gp160 and influenza virus HA.

19. A method of producing a fusion protein complex according to claim 13, **characterised in that** a fusion protein as defined in any one of claims 1 to 12 is contacted with a solid carrier which is capable of binding said fusion protein.

20. The method according to claim 19, **characterised in that** a solution containing a fusion protein is contacted with a carrier.

21. The method according to claim 20, **characterised in that** the solution is a culture supernatant of recombinant cell lines.

22. The method according to claim 20, **characterised in that** the solution contains purified fusion protein.

23. The method according to any one of claims 19 to 22, **characterised in that** the solid carrier is selected from an immobilized (heavy) metal ion, an immobilized antibody or an immobilized peptide or polypeptide.

24. The use of a fusion protein according to any one of claims 1 to 12 for a method according to any one of claims 14 to 18.

25. The use of a fusion protein complex according to claim 13 for a method according to any one of claims 14 to 18.

26. The use of a fusion protein according to any one of claims 1 to 12 for preparing a protein from proprotein, in particular factor IX from pro-factor IX, von Willebrand factor from pro-von Willebrand factor, factor X from pro-factor X, factor XI from pro-factor XI, factor VII from pro-factor VII, factor V from pro-factor V, protein C from pro-protein C and albumin from pro-albumin.

27. The use of a fusion protein complex according to claim 13 for preparing a protein from pro-protein, in particular of factor IX from pro-factor IX, von Willebrand factor from pro-von Willeband factor, factor X from pro-factor X, factor XI from pro-factor XI, factor VII from pro-factor VII, factor V from pro-factor V, protein C from pro-protein C and albumin from pro-albumin.

28. The use of a method according to any one of claims 14 to 18 for preparing a protein from pro-protein, in particular factor IX from pro-factor IX, von Willebrand factor from pro-von Willebrand factor, factor X from pro-factor X, factor XI from pro-factor XI, factor VII from pro-factor VII, factor V from pro-factor V, protein C from pro-protein C and albumin from pro-albumin.

## Revendications

1. Protéine de fusion, **caractérisée en ce qu'**elle comprend une furine avec délétion C-terminale fusionnée à une séquence hétérologue, qui permet l'adsorption de la furine sur un support, la protéine de fusion étant immobilisée sur un support et active au plan protéolytique.

2. Protéine de fusion selon la revendication 1, **caractérisée en ce que** la délétion C-terminale comprend la région cytoplasmique et transmembranaire, et éventuellement la région riche en Cys.

3. Protéine de fusion selon la revendication 2, **caractérisée en ce que** la séquence hétérologue est une protéine, un polypeptide ou un peptide d'affinité.

4. Protéine de fusion selon l'une quelconque des revendications 1 à 3, **caractérisée en ce que** la protéine ou un polypeptide est dérivé de la β-galactosidase, de la glutathion-S-transférase, du produit c-myc, de l'avidine ou de domaines kringels de protéines plasmiques liant la lysine, tel que le plasminogène.

5. Protéine de fusion selon la revendication 3, **caractérisée en ce que** le peptide d'affinité est un peptide tag, de préférence un his-tag.

6. Protéine de fusion selon la revendication 5, **caractérisée en ce que** le peptide d'affinité est constitué de plusieurs, de préférence de 3 à 20, de façon particulièrement préférée de 6 à 15 résidus d'histidine qui se suivent.

7. Protéine de fusion selon la revendication 6, **caractérisée en ce qu'**elle est une furineΔTM-His ou une furineΔCys-His, de préférence une furineΔTM-His selon la séquence ID n°9 ou une furineΔCys-His selon la séquence ID n°18.

8. Protéine de fusion selon l'une quelconque des revendications 1 à 7, **caractérisée en ce qu'**un espaceur est inséré entre la séquence de la furine et la séquence hétérologue.

9. Protéine de fusion selon la revendication 8, **caractérisée en ce qu'**elle est une furineΔTM-espaceur-His ou une furineΔCys-espaceur-His.

10. Protéine de fusion selon la revendication 9, **caractérisée en ce qu'**elle est une furineΔTM-espaceur-His selon la séquence ID n°12 ou une furineΔCys-espaceur-His selon la séquence ID n°21.

11. Protéine de fusion selon l'une quelconque des revendications 1 à 10, **caractérisée en ce qu'**elle possède une affinité envers un support solide.

12. Protéine de fusion selon la revendication 11, **caractérisée en ce que** le support comprend un anticorps ou un ion métallique (lourd).

13. Complexe de protéines de fusion comprenant une protéine de fusion selon l'une quelconque des revendications 1 à 12 adsorbé sur un support solide.

14. Procédé pour la fabrication de protéines à partir de pro-protéines, **caractérisé en ce qu'**une pro-protéine est clivée de façon protéolytique par une protéine de fusion selon l'une quelconque des revendications 1 à 12.

15. Procédé selon la revendication 14, **caractérisé en ce que** la pro-protéine est clivée par une protéine de fusion selon l'une quelconque des revendications 1 à 12 en dehors des cellules.

16. Procédé selon la revendication 15, **caractérisé en ce que** la pro-protéine est sous la forme d'une solution.

17. Procédé selon la revendication 14, **caractérisé en ce que** le support est un métal lourd ou un anticorps.

18. Procédé selon l'une quelconque des revendications 14 à 17, **caractérisé en que** la pro-protéine est un précurseur inactif d'une protéine plasmique, de préférence un facteur IX, un facteur von Willebrand, un facteur VII, un facteur X, un facteur XI, un facteur V, une protéine C, une protéine S, une albumine ou une protéine virale, de préférence gp160 du VIH ou HA du virus de la grippe.

19. Procédé pour la fabrication d'un complexe de protéines de fusion selon la revendication 13, **caractérisé en ce qu'**une protéine de fusion telle que définie dans l'une quelconque des revendications 1 à 12 est mise en contact avec un support solide qui peut lier la protéine de fusion.

20. Procédé selon la revendication 19, **caractérisé en ce qu'**une solution contenant une protéine de fusion est mise en contact avec un support.

21. Procédé selon la revendication 20, **caractérisé en ce que** la solution est un surnageant de culture de lignées cellulaires recombinées.

22. Procédé selon la revendication 20, **caractérisé en ce que** la solution contient une protéine de fusion purifiée.

23. Procédé selon l'une quelconque des revendications 19 à 22, **caractérisé en ce que** le support solide est choisi parmi un ion métallique (lourd) immobilisé, un anticorps immobilisé ou un peptide ou polypeptide immobilisé.

24. Utilisation d'une protéine de fusion selon l'une quelconque des revendications 1 à 12 pour un procédé selon l'une quelconque des revendications 14 à 18.

25. Utilisation d'un complexe de protéines de fusion selon la revendication 13 pour un procédé selon l'une quelconque des revendications 14 à 18.

26. Utilisation d'une protéine de fusion selon l'une quelconque des revendications 1 à 12 pour la fabrication d'une protéine issue d'une pro-protéine, de préférence d'un facteur IX issu d'un pro-facteur IX, d'un facteur von Willebrand issu d'un pro-facteur von Willebrand, d'un facteur X issu du pro-facteur X, d'un facteur XI issu d'un pro-facteur XI, d'un facteur VII issu d'un pro-facteur VII, d'un facteur V issu d'un pro-facteur V, d'une protéine C issue d'une pro-protéine C et d'une albumine issue d'une pro-albumine.

27. Utilisation d'un complexe de protéines de fusion selon la revendication 13 pour la fabrication d'une protéine issue d'une pro-protéine, de préférence d'un facteur IX issu d'un pro-facteur IX, d'un facteur von Willebrand issu d'un pro-facteur von Willebrand, d'un facteur X issu du pro-facteur X, d'un facteur XI issu d'un pro-facteur XI, d'un facteur VII issu d'un pro-facteur VII, d'un facteur V issu d'un pro-facteur V, d'une protéine C issue d'une pro-protéine C et d'une albumine issue d'une pro-albumine.

28. Utilisation d'un procédé selon l'une quelconque des revendications 14 à 18 pour la fabrication d'une protéine issue d'une pro-protéine, de préférence d'un facteur IX issu d'un pro-facteur IX, d'un facteur von Willebrand issu d'un pro-facteur von Willebrand, d'un facteur X issu du pro-facteur X, d'un facteur XI issu d'un pro-facteur XI, d'un facteur VII issu d'un pro-facteur VII, d'un facteur V issu d'un pro-facteur V, d'une protéine C issue d'une pro-protéine C et d'une albumine issue d'une pro-albumine.


### SEQUENZPROTOKOLL

(1) ALLGEMEINE ANGABEN:
   (i) ANMELDER:
      (A) NAME: IMMUNO AG
      (B) STRASSE: Industriestrasse 67
      (C) ORT: Wien
      (D) BUNDESLAND: Austria
      (E) LAND: Austria
      (F) POSTLEITZAHL: 1220
   (ii) BEZEICHNUNG DER ERFINDUNG: Herstellung von Proteinen aus Pro-Proteinen durch Fusionsproteine abgeleitet von Furin oder Furinanalogen
   (iii) ANZAHL DER SEQUENZEN: 27
(2) ANGABEN ZU SEQ ID NO: 1:
   (i) SEQUENZKENNZEICHEN:
      (A) LÄNGE: 75 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 1:
   GGCCATCGAT TGAATTCCCC GGGGTCCTCT AGAGTCGACC TGCAGAAGCT TAGTACTAGT    60
   AGGCCTAGGG CCCTA                                                     75
(2) ANGABEN ZU SEQ ID NO: 2:
   (i) SEQUENZKENNZEICHEN:
      (A) LÄNGE: 75 Basenpaare
      (B) ART: Nucleotid
      (C) STRANGFORM: Einzelstrang
      (D) TOPOLOGIE: linear
   (ii) ART DES MOLEKÜLS: Genom-DNA ,
   (xi) SEQUENZBESCHREIBUNG: SEQ ID NO: 2:
   GGCCTAGGGC CCTAGGCCTA CTAGTACTAA GCTTCTGCAG GTCGACTCTA GAGGACCCCG 60
   GGGAATTCAA TCGAT 75
(2) ANGABEN ZU SEQ ID NO: 3:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 3:
      GATAAGCTTG TCGACCATGG AGCTGAGGCC CTG 33
(2) ANGABEN ZU SEQ ID NO: 4:
   (i) SEQUENZKENNZEICHEN:
      (A) LÄNGE: 34 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 4:
      AAGTCATGAA TTCTTACAGC AGCCCTGCGC GCAG 34
(2) ANGABEN ZU SEQ ID NO: 5:
   (i) SEQUENZKENNZEICHEN:
      (A) LÄNGE: 2130 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 5:
   ATGGAGCTGA GGCCCTGGTT GCTATGGGTG GTAGCAGCAA CAGGAACCTT GGTCCTGCTA 60
   GCAGCTGATG CTCAGGGCCA GAAGGTCTTC ACCAACACGT GGGCTGTGCG CATCCCTGGA 120
   GGCCCAGCGG TGGCCAACAG TGTGGCACGG AAGCATGGGT TCCTCAACCT GGGCCAGATC 180
   TTCGGGGACT ATTACCACTT CTGGCATCGA GGAGTGACGA AGCGGTCCCT GTCGCCTCAC 240
   CGCCCGCGGC ACAGCCGGCT GCAGAGGGAG CCTCAAGTAC AGTGGCTGGA ACAGCAGGTG 300
   GCAAAGCGAC GGACTAAACG GGACGTGTAC CAGGAGCCCA CAGACCCCAA GTTTCCTCAG 360
   CAGTGGTACC TGTCTGGTGT CACTCAGCGG GACCTGAATG TGAAGGCGGC CTGGGCGCAG 420
   GGCTACACAG GGCACGGCAT TGTGGTCTCC ATTCTGGACG ATGGCATCGA GAAGAACCAC 480
   CCGGACTTGG CAGGCAATTA TGATCCTGGG GCCAGTTTTG ATGTCAATGA CCAGGACCCT 540
   GACCCCCAGC CTCGGTACAC ACAGATGAAT GACAACAGGC ACGGCACACG GTGTGCGGGG 600
   GAAGTGGCTG CGGTGGCCAA CAACGGTGTC TGTGGTGTAG GTGTGGCCTA CAACGCCCGC 660
   ATTGGAGGGG TGCGCATGCT GGATGGCGAG GTGACAGATG CAGTGGAGGC ACGCTCGCTG 720
   GGCCTGAACC CCAACCACAT CCACATCTAC AGTGCCAGCT GGGGCCCCGA GGATGACGGC 780
   AAGACAGTGG ATGGGCCAGC CCGCCTCGCC GAGGAGGCCT TCTTCCGTGG GGTTAGCCAG 840
   GGCCGAGGGG GGCTGGGCTC CATCTTTGTC TGGGCCTCGG GGAACGGGGG CCGGGAACAT 900
   GACAGCTGCA ACTGCGACGG CTACACCAAC AGTATCTACA CGCTGTCCAT CAGCAGCGCC 960
   ACGCAGTTTG GCAACGTGCC GTGGTACAGC GAGGCCTGCT CGTCCACACT GGCCACGACC 1020
   TACAGCAGTG GCAACCAGAA TGAGAAGCAG ATCGTGACGA CTGACTTGCG GCAGAAGTGC 1080
   ACGGAGTCTC ACACGGGCAC CTCAGCCTCT GCCCCCTTAG CAGCCGGCAT CATTGCTCTC 1140
   ACCCTGGAGG CCAATAAGAA CCTCACATGG CGGGACATGC AACACCTGGT GGTACAGACC 1200
   TCGAAGCCAG CCCACCTCAA TGCCAACGAC TGGGCCACCA ATGGTGTGGG CCGGAAAGTG   1260
   AGCCACTCAT ATGGCTACGG GCTTTTGGAC GCAGGCGCCA TGGTGGCCCT GGCCCAGAAT   1320
   TGGACCACAG TGGCCCCCCA GCGGAAGTGC ATCATCGACA TCCTCACCGA GCCCAAAGAC   1380
   ATCGGGAAAC GGCTCGAGGT GCGGAAGACC GTGACCGCGT GCCTGGGCGA GCCCAACCAC   1440
   ATCACTCGGC TGGAGCACGC TCAGGCGCGG CTCACCCTGT CCTATAATCG CCGTGGCGAC   1500
   CTGGCCATCC ACCTGGTCAG CCCCATGGGC ACCCGCTCCA CCCTGCTGGC AGCCAGGCCA   1560
   CATGACTACT CCGCAGATGG GTTTAATGAC TGGGCCTTCA TGACAACTCA TTCCTGGGAT   1620
   GAGGATCCCT CTGGCGAGTG GGTCCTAGAG ATTGAAAACA CCAGCGAAGC CAACAACTAT   1680
   GGGACGCTGA CCAAGTTCAC CCTCGTACTC TATGGCACCG CCCCTGAGGG GCTGCCCGTA   1740
   CCTCCAGAAA GCAGTGGCTG CAAGACCCTC ACGTCCAGTC AGGCCTGTGT GGTGTGCGAG   1800
   GAAGGCTTCT CCCTGCACCA GAAGAGCTGT GTCCAGCACT GCCCTCCAGG CTTCGCCCCC   1860
   CAAGTCCTCG ATACGCACTA TAGCACCGAG AATGACGTGG AGACCATCCG GGCCAGCGTC   1920
   TGCGCCCCCT GCCACGCCTC ATGTGCCACA TGCCAGGGGC CGGCCCTGAC AGACTGCCTC   1980
   AGCTGCCCCA GCCACGCCTC CTTGGACCCT GTGGAGCAGA CTTGCTCCCG GCAAAGCCAG   2040
   AGCAGCCGAG AGTCCCCGCC ACAGCAGCAG CCACCTCGGC TGCCCCCGGA GGTGGAGGCG   2100
   GGGCAACGGC TGCGCGCAGG GCTGCTGTAA                                    2130
(2) ANGABEN ZU SEQ ID NO: 6:
   (i) SEQUENZKENNZEICHEN:
      (A) LANGE: 709 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 6:
   Met Glu Leu Arg Pro Trp Leu Leu Trp Val Val Ala Ala Thr Gly Thr
   1               5                   10                  15
   Leu Val Leu Leu Ala Ala Asp Ala Gln Gly Gln Lys Val Phe Thr Asn
               20                  25                  30
   Thr Trp Ala Val Arg Ile Pro Gly Gly Pro Ala Val Ala Asn Ser Val
           35                  40                  45
   Ala Arg Lys His Gly Phe Leu Asn Leu Gly Gln Ile Phe Gly Asp Tyr
       50                  55                  60
   Tyr His Phe Trp His Arg Gly Val Thr Lys Arg Ser Leu Ser Pro His
   65 70 75 80
   Arg Pro Arg His Ser Arg Leu Gln Arg Glu Pro Gln Val Gln Trp Leu
   85 90 95
   Glu Gln Gln Val Ala Lys Arg Arg Thr Lys Arg Asp Val Tyr Gln Glu
   100 105 110
   Pro Thr Asp Pro Lys Phe Pro Gln Gln Trp Tyr Leu Ser Gly Val Thr
   115 120 125
   Gln Arg Asp Leu Asn Val Lys Ala Ala Trp Ala Gln Gly Tyr Thr Gly
   130 135 140
   His Gly Ile Val Val Ser Ile Leu Asp Asp Gly Ile Glu Lys Asn His
   145 150 155 160
   Pro Asp Leu Ala Gly Asn Tyr Asp Pro Gly Ala Ser Phe Asp Val Asn
   165 170 175
   Asp Gln Asp Pro Asp Pro Gln Pro Arg Tyr Thr Gln Met Asn Asp Asn
   180 185 190
   Arg His Gly Thr Arg Cys Ala Gly Glu Val Ala Ala Val Ala Asn Asn
   195 200 205
   Gly Val Cys Gly Val Gly Val Ala Tyr Asn Ala Arg Ile Gly Gly Val
   210 215 220
   Arg Met Leu Asp Gly Glu Val Thr Asp Ala Val Glu Ala Arg Ser Leu
   225 230 235 240
   Gly Leu Asn Pro Asn His Ile His Ile Tyr Ser Ala Ser Trp Gly Pro
   245 250 255
   Glu Asp Asp Gly Lys Thr Val Asp Gly Pro Ala Arg Leu Ala Glu Glu
   260 265 270
   Ala Phe Phe Arg Gly Val Ser Gln Gly Arg Gly Gly Leu Gly Ser Ile
   275 280 285
   Phe Val Trp Ala Ser Gly Asn Gly Gly Arg Glu His Asp Ser Cys Asn
   290 295 300
   Cys Asp Gly Tyr Thr Asn Ser Ile Tyr Thr Leu Ser Ile Ser Ser Ala
   305 310 315 320
   Thr Gln Phe Gly Asn Val Pro Trp Tyr Ser Glu Ala Cys Ser Ser Thr
   325 330 335
   Leu Ala Thr Thr Tyr Ser Ser Gly Asn Gln Asn Glu Lys Gln Ile Val
   340 345 350
   Thr Thr Asp Leu Arg Gln Lys Cys Thr Glu Ser His Thr Gly Thr Ser
   355 360 365
   Ala Ser Ala Pro Leu Ala Ala Gly Ile Ile Ala Leu Thr Leu Glu Ala
   370 375 380
   Asn Lys Asn Leu Thr Trp Arg Asp Met Gln His Leu Val Val Gln Thr
   385 390 395 400
   Ser Lys Pro Ala His Leu Asn Ala Asn Asp Trp Ala Thr Asn Gly Val
   405 410 415
   Gly Arg Lys Val Ser His Ser Tyr Gly Tyr Gly Leu Leu Asp Ala Gly
   420 425 430
   Ala Met Val Ala Leu Ala Gln Asn Trp Thr Thr Val Ala Pro Gln Arg
   435 440 445
   Lys Cys Ile Ile Asp Ile Leu Thr Glu Pro Lys Asp Ile Gly Lys Arg
   450 455 460
   Leu Glu Val Arg Lys Thr Val Thr Ala Cys Leu Gly Glu Pro Asn His
   465 470 475 480
   Ile Thr Arg Leu Glu His Ala Gln Ala Arg Leu Thr Leu Ser Tyr Asn
   485 490 495
   Arg Arg Gly Asp Leu Ala Ile His Leu Val Ser Pro Met Gly Thr Arg
   500 505 510
   Ser Thr Leu Leu Ala Ala Arg Pro His Asp Tyr Ser Ala Asp Gly Phe
   515 520 525
   Asn Asp Trp Ala Phe Met Thr Thr His Ser Trp Asp Glu Asp Pro Ser
   530 535 540
   Gly Glu Trp Val Leu Glu Ile Glu Asn Thr Ser Glu Ala Asn Asn Tyr
   545 550 555 560
   Gly Thr Leu Thr Lys Phe Thr Leu Val Leu Tyr Gly Thr Ala Pro Glu
   565 570 575
   Gly Leu Pro Val Pro Pro Glu Ser Ser Gly Cys Lys Thr Leu Thr Ser
   580 585 590
   Ser Gln Ala Cys Val Val Cys Glu Glu Gly Phe Ser Leu His Gln Lys
   595 600 605
   Ser Cys Val Gln His Cys Pro Pro Gly Phe Ala Pro Gln Val Leu Asp
   610 615 620
   Thr His Tyr Ser Thr Glu Asn Asp Val Glu Thr Ile Arg Ala Ser Val
   625 630 635 640
   Cys Ala Pro Cys His Ala Ser Cys Ala Thr Cys Gln Gly Pro Ala Leu
   645 650 655
   Thr Asp Cys Leu Ser Cys Pro Ser His Ala Ser Leu Asp Pro Val Glu
   660 665 670
   Gln Thr Cys Ser Arg Gln Ser Gln Ser Ser Arg Glu Ser Pro Pro Gln
   675 680 685
   Gln Gln Pro Pro Arg Leu Pro Pro Glu Val Glu Ala Gly Gln Arg Leu
   690 695 700
   Arg Ala Gly Leu Leu
   705
(2) ANGABEN ZU SEQ ID NO: 7:
   (i) SEQUENZKEHNNZEICHEN:
      (A) LÄNGE: 50 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 7:
      CTAGAATTCA ATGATGATGA TGATGATGCC CTGCGCGCAG CCGTTGCCCC 50
(2) ANGABEN ZU SEQ ID NO: 8:
   (i) SEQUENZKENNZEICHEN:
      (A) LÄNGE: 2142 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 8:
   ATGGAGCTGA GGCCCTGGTT GCTATGGGTG GTAGCAGCAA CAGGAACCTT GGTCCTGCTA 60
   GCAGCTGATG CTCAGGGCCA GAAGGTCTTC ACCAACACGT GGGCTGTGCG CATCCCTGGA 120
   GGCCCAGCGG TGGCCAACAG TGTGGCACGG AAGCATGGGT TCCTCAACCT GGGCCAGATC 180
   TTCGGGGACT ATTACCACTT CTGGCATCGA GGAGTGACGA AGCGGTCCCT GTCGCCTCAC 240
   CGCCCGCGGC ACAGCCGGCT GCAGAGGGAG CCTCAAGTAC AGTGGCTGGA ACAGCAGGTG 300
   GCAAAGCGAC GGACTAAACG GGACGTGTAC CAGGAGCCCA CAGACCCCAA GTTTCCTCAG 360
   CAGTGGTACC TGTCTGGTGT CACTCAGCGG GACCTGAATG TGAAGGCGGC CTGGGCGCAG 420
   GGCTACACAG GGCACGGCAT TGTGGTCTCC ATTCTGGACG ATGGCATCGA GAAGAACCAC 480
   CCGGACTTGG CAGGCAATTA TGATCCTGGG GCCAGTTTTG ATGTCAATGA CCAGGACCCT 540
   GACCCCCAGC CTCGGTACAC ACAGATGAAT GACAACAGGC ACGGCACACG GTGTGCGGGG 600
   GAAGTGGCTG CGGTGGCCAA CAACGGTGTC TGTGGTGTAG GTGTGGCCTA CAACGCCCGC 660
   ATTGGAGGGG TGCGCATGCT GGATGGCGAG GTGACAGATG CAGTGGAGGC ACGCTCGCTG 720
   GGCCTGAACC CCAACCACAT CCACATCTAC AGTGCCAGCT GGGGCCCCGA GGATGACGGC 780
   AAGACAGTGG ATGGGCCAGC CCGCCTCGCC GAGGAGGCCT TCTTCCGTGG GGTTAGCCAG 840
   GGCCGAGGGG GGCTGGGCTC CATCTTTGTC TGGGCCTCGG GGAACGGGGG CCGGGAACAT 900
   GACAGCTGCA ACTGCGACGG CTACACCAAC AGTATCTACA CGCTGTCCAT CAGCAGCGCC 960
   ACGCAGTTTG GCAACGTGCC GTGGTACAGC GAGGCCTGCT CGTCCACACT GGCCACGACC 1020
   TACAGCAGTG GCAACCAGAA TGAGAAGCAG ATCGTGACGA CTGACTTGCG GCAGAAGTGC 1080
   ACGGAGTCTC ACACGGGCAC CTCAGCCTCT GCCCCCTTAG CAGCCGGCAT CATTGCTCTC 1140
   ACCCTGGAGG CCAATAAGAA CCTCACATGG CGGGACATGC AACACCTGGT GGTACAGACC 1200
   TCGAAGCCAG CCCACCTCAA TGCCAACGAC TGGGCCACCA ATGGTGTGGG CCGGAAAGTG 1260
   AGCCACTCAT ATGGCTACGG GCTTTTGGAC GCAGGCGCCA TGGTGGCCCT GGCCCAGAAT 1320
   TGGACCACAG TGGCCCCCCA GCGGAAGTGC ATCATCGACA TCCTCACCGA GCCCAAAGAC 1380
   ATCGGGAAAC GGCTCGAGGT GCGGAAGACC GTGACCGCGT GCCTGGGCGA GCCCAACCAC 1440
   ATCACTCGGC TGGAGCACGC TCAGGCGCGG CTCACCCTGT CCTATAATCG CCGTGGCGAC 1500
   CTGGCCATCC ACCTGGTCAG CCCCATGGGC ACCCGCTCCA CCCTGCTGGC AGCCAGGCCA 1560
   CATGACTACT CCGCAGATGG GTTTAATGAC TGGGCCTTCA TGACAACTCA TTCCTGGGAT 1620
   GAGGATCCCT CTGGCGAGTG GGTCCTAGAG ATTGAAAACA CCAGCGAAGC CAACAACTAT 1680
   GGGACGCTGA CCAAGTTCAC CCTCGTACTC TATGGCACCG CCCCTGAGGG GCTGCCCGTA 1740
   CCTCCAGAAA GCAGTGGCTG CAAGACCCTC ACGTCCAGTC AGGCCTGTGT GGTGTGCGAG 1800
   GAAGGCTTCT CCCTGCACCA GAAGAGCTGT GTCCAGCACT GCCCTCCAGG CTTCGCCCCC 1860
   CAAGTCCTCG ATACGCACTA TAGCACCGAG AATGACGTGG AGACCATCCG GGCCAGCGTC 1920
   TGCGCCCCCT GCCACGCCTC ATGTGCCACA TGCCAGGGGC CGGCCCTGAC AGACTGCCTC 1980
   AGCTGCCCCA GCCACGCCTC CTTGGACCCT GTGGAGCAGA CTTGCTCCCG GCAAAGCCAG 2040
   AGCAGCCGAG AGTCCCCGCC ACAGCAGCAG CCACCTCGGC TGCCCCCGGA GGTGGAGGCG 2100
   GGGCAACGGC TGCGCGCAGG GCATCATCAT CATCATCATT GA 2142
(2) ANGABEN ZU SEQ ID NO: 9:
   (i) SEQUENZKENNZEICHEN:
      (A) LÄNGE: 713 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 9:
   Met Glu Leu Arg Pro Trp Leu Leu Trp Val Val Ala Ala Thr Gly Thr
   1               5                   10                  15
   Leu Val Leu Leu Ala Ala Asp Ala Gln Gly Gln Lys Val Phe Thr Asn
               20                  25                  30
   Thr Trp Ala Val Arg Ile Pro Gly Gly Pro Ala Val Ala Asn Ser Val
           35                  40                  45
   Ala Arg Lys His Gly Phe Leu Asn Leu Gly Gln Ile Phe Gly Asp Tyr
       50                  55                  60
   Tyr His Phe Trp His Arg Gly Val Thr Lys Arg Ser Leu Ser Pro His
   65                  70                  75                  80
   Arg Pro Arg His Ser Arg Leu Gln Arg Glu Pro Gln Val Gln Trp Leu
                   85                  90                  95
   Glu Gln Gln Val Ala Lys Arg Arg Thr Lys Arg Asp Val Tyr Gln Glu
               100                 105                 110
   Pro Thr Asp Pro Lys Phe Pro Gln Gln Trp Tyr Leu Ser Gly Val Thr
           115                 120                 125
   Gln Arg Asp Leu Asn Val Lys Ala Ala Trp Ala Gln Gly Tyr Thr Gly
       130                 135                 140
   His Gly Ile Val Val Ser Ile Leu Asp Asp Gly Ile Glu Lys Asn His
   145                 150                 155                 160
   Pro Asp Leu Ala Gly Asn Tyr Asp Pro Gly Ala Ser Phe Asp Val Asn
                   165                 170                 175
   Asp Gln Asp Pro Asp Pro Gln Pro Arg Tyr Thr Gln Met Asn Asp Asn
               180                 185                 190
   Arg His Gly Thr Arg Cys Ala Gly Glu Val Ala Ala Val Ala Asn Asn
           195                 200                 205
   Gly Val Cys Gly Val Gly Val Ala Tyr Asn Ala Arg Ile Gly Gly Val
       210                 215                 220
   Arg Met Leu Asp Gly Glu Val Thr Asp Ala Val Glu Ala Arg Ser Leu
   225                 230                 235                 240
   Gly Leu Asn Pro Asn His Ile His Ile Tyr Ser Ala Ser Trp Gly Pro
                   245                 250                 255
   Glu Asp Asp Gly Lys Thr Val Asp Gly Pro Ala Arg Leu Ala Glu Glu
               260                 265                 270
   Ala Phe Phe Arg Gly Val Ser Gln Gly Arg Gly Gly Leu Gly Ser Ile
           275                 280                 285
   Phe Val Trp Ala Ser Gly Asn Gly Gly Arg Glu His Asp Ser Cys Asn
       290                 295                 300
   Cys Asp Gly Tyr Thr Asn Ser Ile Tyr Thr Leu Ser Ile Ser Ser Ala
   305 310 315 320
   Thr Gln Phe Gly Asn Val Pro Trp Tyr Ser Glu Ala Cys Ser Ser Thr
   325 330 335
   Leu Ala Thr Thr Tyr Ser Ser Gly Asn Gln Asn Glu Lys Gln Ile Val
   340 345 350
   Thr Thr Asp Leu Arg Gln Lys Cys Thr Glu Ser His Thr Gly Thr Ser
   355 360 365
   Ala Ser Ala Pro Leu Ala Ala Gly Ile Ile Ala Leu Thr Leu Glu Ala
   370 375 380
   Asn Lys Asn Leu Thr Trp Arg Asp Met Gln His Leu Val Val Gln Thr
   385 390 395 400
   Ser Lys Pro Ala His Leu Asn Ala Asn Asp Trp Ala Thr Asn Gly Val
   405 410 415
   Gly Arg Lys Val Ser His Ser Tyr Gly Tyr Gly Leu Leu Asp Ala Gly
   420 425 430
   Ala Met Val Ala Leu Ala Gln Asn Trp Thr Thr Val Ala Pro Gln Arg
   435 440 445
   Lys Cys Ile Ile Asp Ile Leu Thr Glu Pro Lys Asp Ile Gly Lys Arg
   450 455 460
   Leu Glu Val Arg Lys Thr Val Thr Ala Cys Leu Gly Glu Pro Asn His
   465 470 475 480
   Ile Thr Arg Leu Glu His Ala Gln Ala Arg Leu Thr Leu Ser Tyr Asn
   485 490 495
   Arg Arg Gly Asp Leu Ala Ile His Leu Val Ser Pro Met Gly Thr Arg
   500 505 510
   Ser Thr Leu Leu Ala Ala Arg Pro His Asp Tyr Ser Ala Asp Gly Phe
   515 520 525
   Asn Asp Trp Ala Phe Met Thr Thr His Ser Trp Asp Glu Asp Pro Ser
   530 535 540
   Gly Glu Trp Val Leu Glu Ile Glu Asn Thr Ser Glu Ala Asn Asn Tyr
   545 550 555 560
   Gly Thr Leu Thr Lys Phe Thr Leu Val Leu Tyr Gly Thr Ala Pro Glu
   565 570 575
   Gly Leu Pro Val Pro Pro Glu Ser Ser Gly Cys Lys Thr Leu Thr Ser
   580 585 590
   Ser Gln Ala Cys Val Val Cys Glu Glu Gly Phe Ser Leu His Gln Lys
   595 600 605
   Ser Cys Val Gln His Cys Pro Pro Gly Phe Ala Pro Gln Val Leu Asp
   610 615 620
   Thr His Tyr Ser Thr Glu Asn Asp Val Glu Thr Ile Arg Ala Ser Val
   625 630 635 640
   Cys Ala Pro Cys His Ala Ser Cys Ala Thr Cys Gln Gly Pro Ala Leu
   645 650 655
   Thr Asp Cys Leu Ser Cys Pro Ser His Ala Ser Leu Asp Pro Val Glu
   660 665 670
   Gln Thr Cys Ser Arg Gln Ser Gln Ser Ser Arg Glu Ser Pro Pro Gln
   675 680 685
   Gln Gln Pro Pro Arg Leu Pro Pro Glu Val Glu Ala Gly Gln Arg Leu
   690 695 700
   Arg Ala Gly His His His His His His
   705 710
(2) ANGABEN ZU SEQ ID NO: 10:
   (i) SEQUENZKENNZEICHEN:
      (A) LÄNGE: 68 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 10:
   CTAGAATTCA ATGATGATGA TGATGATGTG CAGCTCCACC AGCTGCCCCT GCGCGCAGCC 60
   GTTGCCCC 68
(2) ANGABEN ZU SEQ ID NO: 11:
   (i) SEQUENZKENNZEICHEN:
      (A) LÄNGE: 2160 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 11:
   ATGGAGCTGA GGCCCTGGTT GCTATGGGTG GTAGCAGCAA CAGGAACCTT GGTCCTGCTA 60
   GCAGCTGATG CTCAGGGCCA GAAGGTCTTC ACCAACACGT GGGCTGTGCG CATCCCTGGA 120
   CGCCCAGCGG TGGCCAACAG TGTGGCACGG AAGCATGGGT TCCTCAACCT GGGCCAGATC 180
   TTCGGGGACT ATTACCACTT CTGGCATCGA GGAGTGACGA AGCGGTCCCT GTCGCCTCAC 240
   CGCCCGCGGC ACAGCCGGCT GCAGAGGGAG CCTCAAGTAC AGTGGCTGGA ACAGCAGGTG 300
   GCAAAGCGAC GGACTAAACG GGACGTGTAC CAGGAGCCCA CAGACCCCAA GTTTCCTCAG 360
   CAGTGGTACC TGTCTGGTGT CACTCAGCGG GACCTGAATG TGAAGGCGGC CTGGGCGCAG 420
   GGCTACACAG GGCACGGCAT TGTGGTCTCC ATTCTGGACG ATGGCATCGA GAAGAACCAC 480
   CCGGACTTGG CAGGCAATTA TGATCCTGGG GCCAGTTTTG ATGTCAATGA CCAGGACCCT 540
   GACCCCCAGC CTCGGTACAC ACAGATGAAT GACAACAGGC ACGGCACACG GTGTGCGGGG 600
   GAAGTGGCTG CGGTGGCCAA CAACGGTGTC TGTGGTGTAG GTGTGGCCTA CAACGCCCGC 660
   ATTGGAGGGG TGCGCATGCT GGATGGCGAG GTGACAGATG CAGTGGAGGC ACGCTCGCTG 720
   GGCCTGAACC CCAACCACAT CCACATCTAC AGTGCCAGCT GGGGCCCCGA GGATGACGGC 780
   AAGACAGTGG ATGGGCCAGC CCGCCTCGCC GAGGAGGCCT TCTTCCGTGG GGTTAGCCAG 840
   GGCCGAGGGG GGCTGGGCTC CATCTTTGTC TGGGCCTCGG GGAACGGGGG CCGGGAACAT 900
   GACAGCTGCA ACTGCGACGG CTACACCAAC AGTATCTACA CGCTGTCCAT CAGCAGCGCC 960
   ACGCAGTTTG GCAACGTGCC GTGGTACAGC GAGGCCTGCT CGTCCACACT GGCCACGACC 1020
   TACAGCAGTG GCAACCAGAA TGAGAAGCAG ATCGTGACGA CTGACTTGCG GCAGAAGTGC 1080
   ACGGAGTCTC ACACGGGCAC CTCAGCCTCT GCCCCCTTAG CAGCCGGCAT CATTGCTCTC 1140
   ACCCTGGAGG CCAATAAGAA CCTCACATGG CGGGACATGC AACACCTGGT GGTACAGACC 1200
   TCGAAGCCAG CCCACCTCAA TGCCAACGAC TGGGCCACCA ATGGTGTGGG CCGGAAAGTG 1260
   AGCCACTCAT ATGGCTACGG GCTTTTGGAC GCAGGCGCCA TGGTGGCCCT GGCCCAGAAT 1320
   TGGACCACAG TGGCCCCCCA GCGGAAGTGC ATCATCGACA TCCTCACCGA GCCCAAAGAC 1380
   ATCGGGAAAC GGCTCGAGGT GCGGAAGACC GTGACCGCGT GCCTGGGCGA GCCCAACCAC 1440
   ATCACTCGGC TGGAGCACGC TCAGGCGCGG CTCACCCTGT CCTATAATCG CCGTGGCGAC 1500
   CTGGCCATCC ACCTGGTCAG CCCCATGGGC ACCCGCTCCA CCCTGCTGGC AGCCAGGCCA 1560
   CATGACTACT CCGCAGATGG GTTTAATGAC TGGGCCTTCA TGACAACTCA TTCCTGGGAT 1620
   GAGGATCCCT CTGGCGAGTG GGTCCTAGAG ATTGAAAACA CCAGCGAAGC CAACAACTAT 1680
   GGGACGCTGA CCAAGTTCAC CCTCGTACTC TATGGCACCG CCCCTGAGGG GCTGCCCGTA 1740
   CCTCCAGAAA GCAGTGGCTG CAAGACCCTC ACGTCCAGTC AGGCCTGTGT GGTGTGCGAG 1800
   GAAGGCTTCT CCCTGCACCA GAAGAGCTGT GTCCAGCACT GCCCTCCAGG CTTCGCCCCC 1860
   CAAGTCCTCG ATACGCACTA TAGCACCGAG AATGACGTGG AGACCATCCG GGCCAGCGTC 1920
   TGCGCCCCCT GCCACGCCTC ATGTGCCACA TGCCAGGGGC CGGCCCTGAC AGACTGCCTC 1980
   AGCTGCCCCA GCCACGCCTC CTTGGACCCT GTGGAGCAGA CTTGCTCCCG GCAAAGCCAG 2040
   AGCAGCCGAG AGTCCCCGCC ACAGCAGCAG CCACCTCGGC TGCCCCCGGA GGTGGAGGCG 2100
   GGGCAACGGC TGCGCGCAGG GGCAGCTGGT GGAGCTGCAC ATCATCATCA TCATCATTGA 2160
(2) ANGABEN ZU SEQ ID NO: 12:
   (i) SEQUENZKENNZEICHEN:
      (A) LANGE: 719 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 12:
   Met Glu Leu Arg Pro Trp Leu Leu Trp Val Val Ala Ala Thr Gly Thr
   1               5                   10                  15
   Leu Val Leu Leu Ala Ala Asp Ala Gln Gly Gln Lys Val Phe Thr Asn
               20                  25                  30
   Thr Trp Ala Val Arg Ile Pro Gly Gly Pro Ala Val Ala Asn Ser Val
           35                  40                  45
   Ala Arg Lys His Gly Phe Leu Asn Leu Gly Gln Ile Phe Gly Asp Tyr
       50                  55                  60
   Tyr His Phe Trp His Arg Gly Val Thr Lys Arg Ser Leu Ser Pro His
   65                  70                  75                  80
   Arg Pro Arg His Ser Arg Leu Gln Arg Glu Pro Gln Val Gln Trp Leu
                   85                  90                  95
   Glu Gln Gln Val Ala Lys Arg Arg Thr Lys Arg Asp Val Tyr Gln Glu
               100                 105                 110
   Pro Thr Asp Pro Lys Phe Pro Gln Gln Trp Tyr Leu Ser Gly Val Thr
           115                 120                 125
   Gln Arg Asp Leu Asn Val Lys Ala Ala Trp Ala Gln Gly Tyr Thr Gly
       130                 135                 140
   His Gly Ile Val Val Ser Ile Leu Asp Asp Gly Ile Glu Lys Asn His
   145                 150                 155                 160
   Pro Asp Leu Ala Gly Asn Tyr Asp Pro Gly Ala Ser Phe Asp Val Asn
                   165                 170                 175
   Asp Gln Asp Pro Asp Pro Gln Pro Arg Tyr Thr Gln Met Asn Asp Asn
               180                 185                 190
   Arg His Gly Thr Arg Cys Ala Gly Glu Val Ala Ala Val Ala Asn Asn
           195                 200                 205
   Gly Val Cys Gly Val Gly Val Ala Tyr Asn Ala Arg Ile Gly Gly Val
       210                 215                 220
   Arg Met Leu Asp Gly Glu Val Thr Asp Ala Val Glu Ala Arg Ser Leu
   225 230 235 240
   Gly Leu Asn Pro Asn His Ile His Ile Tyr Ser Ala Ser Trp Gly Pro
   245 250 255
   Glu Asp Asp Gly Lys Thr Val Asp Gly Pro Ala Arg Leu Ala Glu Glu
   260 265 270
   Ala Phe Phe Arg Gly Val Ser Gln Gly Arg Gly Gly Leu Gly Ser Ile
   275 280 285
   Phe Val Trp Ala Ser Gly Asn Gly Gly Arg Glu His Asp Ser Cys Asn
   290 295 300
   Cys Asp Gly Tyr Thr Asn Ser Ile Tyr Thr Leu Ser Ile Ser Ser Ala
   305 310 315 320
   Thr Gln Phe Gly Asn Val Pro Trp Tyr Ser Glu Ala Cys Ser Ser Thr
   325 330 335
   Leu Ala Thr Thr Tyr Ser Ser Gly Asn Gln Asn Glu Lys Gln Ile Val
   340 345 350
   Thr Thr Asp Leu Arg Gln Lys Cys Thr Glu Ser His Thr Gly Thr Ser
   355 360 365
   Ala Ser Ala Pro Leu Ala Ala Gly Ile Ile Ala Leu Thr Leu Glu Ala
   370 375 380
   Asn Lys Asn Leu Thr Trp Arg Asp Met Gln His Leu Val Val Gln Thr
   385 390 395 400
   Ser Lys Pro Ala His Leu Asn Ala Asn Asp Trp Ala Thr Asn Gly Val
   405 410 415
   Gly Arg Lys Val Ser His Ser Tyr Gly Tyr Gly Leu Leu Asp Ala Gly
   420 425 430
   Ala Met Val Ala Leu Ala Gln Asn Trp Thr Thr Val Ala Pro Gln Arg
   435 440 445
   Lys Cys Ile Ile Asp Ile Leu Thr Glu Pro Lys Asp Ile Gly Lys Arg
   450 455 460
   Leu Glu Val Arg Lys Thr Val Thr Ala Cys Leu Gly Glu Pro Asn His
   465 470 475 480
   Ile Thr Arg Leu Glu His Ala Gln Ala Arg Leu Thr Leu Ser Tyr Asn
   485 490 495
   Arg Arg Gly Asp Leu Ala Ile His Leu Val Ser Pro Met Gly Thr Arg
   500 505 510
   Ser Thr Leu Leu Ala Ala Arg Pro His Asp Tyr Ser Ala Asp Gly Phe
   515 520 525
   Asn Asp Trp Ala Phe Met Thr Thr His Ser Trp Asp Glu Asp Pro Ser
   530 535 540
   Gly Glu Trp Val Leu Glu Ile Glu Asn Thr Ser Glu Ala Asn Asn Tyr
   545 550 555 560
   Gly Thr Leu Thr Lys Phe Thr Leu Val Leu Tyr Gly Thr Ala Pro Glu
   565 570 575
   Gly Leu Pro Val Pro Pro Glu Ser Ser Gly Cys Lys Thr Leu Thr Ser
   580 585 590
   Ser Gln Ala Cys Val Val Cys Glu Glu Gly Phe Ser Leu His Gln Lys
   595 600 605
   Ser Cys Val Gln His Cys Pro Pro Gly Phe Ala Pro Gln Val Leu Asp
   610 615 620
   Thr His Tyr Ser Thr Glu Asn Asp Val Glu Thr Ile Arg Ala Ser Val
   625 630 635 640
   Cys Ala Pro Cys His Ala Ser Cys Ala Thr Cys Gln Gly Pro Ala Leu
   645 650 655
   Thr Asp Cys Leu Ser Cys Pro Ser His Ala Ser Leu Asp Pro Val Glu
   660 665 670
   Gln Thr Cys Ser Arg Gln Ser Gln Ser Ser Arg Glu Ser Pro Pro Gln
   675 680 685
   Gln Gln Pro Pro Arg Leu Pro Pro Glu Val Glu Ala Gly Gln Arg Leu
   690 695 700
   Arg Ala Gly Ala Ala Gly Gly Ala Ala His His His His His His
   705 710 715
(2) ANGABEN ZU SEQ ID NO: 13:
   (i) SEQUENZKENNZEICHEN:
      (A) LANGE: 1758 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 13:
   ATGGAGCTGA GGCCCTGGTT GCTATGGGTG GTAGCAGCAA CAGGAACCTT GGTCCTGCTA 60
   GCAGCTGATG CTCAGGGCCA GAAGGTCTTC ACCAACACGT GGGCTGTGCG CATCCCTGGA 120
   GGCCCAGCGG TGGCCAACAG TGTGGCACGG AAGCATGGGT TCCTCAACCT GGGCCAGATC 180
   TTCGGGGACT ATTACCACTT CTGGCATCGA GGAGTGACGA AGCGGTCCCT GTCGCCTCAC 240
   CGCCCGCGGC ACAGCCGGCT GCAGAGGGAG CCTCAAGTAC AGTGGCTGGA ACAGCAGGTG 300
   GCAAAGCGAC GGACTAAACG GGACGTGTAC CAGGAGCCCA CAGACCCCAA GTTTCCTCAG 360
   CAGTGGTACC TGTCTGGTGT CACTCAGCGG GACCTGAATG TGAAGGCGGC CTGGGCGCAG 420
   GGCTACACAG GGCACGGCAT TGTGGTCTCC ATTCTGGACG ATGGCATCGA GAAGAACCAC 480
   CCGGACTTGG CAGGCAATTA TGATCCTGGG GCCAGTTTTG ATGTCAATGA CCAGGACCCT 540
   GACCCCCAGC CTCGGTACAC ACAGATGAAT GACAACAGGC ACGGCACACG GTGTGCGGGG 600
   GAAGTGGCTG CGGTGGCCAA CAACGGTGTC TGTGGTGTAG GTGTGGCCTA CAACGCCCGC 660
   ATTGGAGGGG TGCGCATGCT GGATGGCGAG GTGACAGATG CAGTGGAGGC ACGCTCGCTG 720
   GGCCTGAACC CCAACCACAT CCACATCTAC AGTGCCAGCT GGGGCCCCGA GGATGACGGC 780
   AAGACAGTGG ATGGGCCAGC CCGCCTCGCC GAGGAGGCCT TCTTCCGTGG GGTTAGCCAG 840
   GGCCGAGGGG GGCTGGGCTC CATCTTTGTC TGGGCCTCGG GGAACGGGGG CCGGGAACAT 900
   GACAGCTGCA ACTGCGACGG CTACACCAAC AGTATCTACA CGCTGTCCAT CAGCAGCGCC 960
   ACGCAGTTTG GCAACGTGCC GTGGTACAGC GAGGCCTGCT CGTCCACACT GGCCACGACC 1020
   TACAGCAGTG GCAACCAGAA TGAGAAGCAG ATCGTGACGA CTGACTTGCG GCAGAAGTGC 1080
   ACGGAGTCTC ACACGGGCAC CTCAGCCTCT GCCCCCTTAG CAGCCGGCAT CATTGCTCTC 1140
   ACCCTGGAGG CCAATAAGAA CCTCACATGG CGGGACATGC AACACCTGGT GGTACAGACC 1200
   TCGAAGCCAG CCCACCTCAA TGCCAACGAC TGGGCCACCA ATGGTGTGGG CCGGAAAGTG 1260
   AGCCACTCAT ATGGCTACGG GCTTTTGGAC GCAGGCGCCA TGGTGGCCCT GGCCCAGAAT 1320
   TGGACCACAG TGGCCCCCCA GCGGAAGTGC ATCATCGACA TCCTCACCGA GCCCAAAGAC 1380
   ATCGGGAAAC GGCTCGAGGT GCGGAAGACC GTGACCGCGT GCCTGGGCGA GCCCAACCAC 1440
   ATCACTCGGC TGGAGCACGC TCAGGCGCGG CTCACCCTGT CCTATAATCG CCGTGGCGAC 1500
   CTGGCCATCC ACCTGGTCAG CCCCATGGGC ACCCGCTCCA CCCTGCTGGC AGCCAGGCCA 1560
   CATGACTACT CCGCAGATGG GTTTAATGAC TGGGCCTTCA TGACAACTCA TTCCTGGGAT 1620
   GAGGATCCCT CTGGCGAGTG GGTCCTAGAG ATTGAAAACA CCAGCGAAGC CAACAACTAT 1680
   GGGACGCTGA CCAAGTTCAC CCTCGTACTC TATGGCACCG CCCCTGAGGG GCTGCCCGTA 1740
   CCTCCAGAAA GCAGTTAG 1758
(2) ANGABEN ZU SEQ ID NO: 14:
   (i) SEQUENZKENNZEICHEN:
      (A) LÄNGE: 585 Aminosauren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 14:
   Met Glu Leu Arg Pro Trp Leu Leu Trp Val Val Ala Ala Thr Gly Thr
   1 5 10 15
   Leu Val Leu Leu Ala Ala Asp Ala Gln Gly Gln Lys Val Phe Thr Asn
   20 25 30
   Thr Trp Ala Val Arg Ile Pro Gly Gly Pro Ala Val Ala Asn Ser Val
   35 40 45
   Ala Arg Lys His Gly Phe Leu Asn Leu Gly Gln Ile Phe Gly Asp Tyr
   50 55 60
   Tyr His Phe Trp His Arg Gly Val Thr Lys Arg Ser Leu Ser Pro His
   65 70 75 80
   Arg Pro Arg His Ser Arg Leu Gln Arg Glu Pro Gln Val Gln Trp Leu
   85 90 95
   Glu Gln Gln Val Ala Lys Arg Arg Thr Lys Arg Asp Val Tyr Gln Glu
   100 105 110
   Pro Thr Asp Pro Lys Phe Pro Gln Gln Trp Tyr Leu Ser Gly Val Thr
   115 120 125
   Gln Arg Asp Leu Asn Val Lys Ala Ala Trp Ala Gln Gly Tyr Thr Gly
   130 135 140
   His Gly Ile Val Val Ser Ile Leu Asp Asp Gly Ile Glu Lys Asn His
   145 150 155 160
   Pro Asp Leu Ala Gly Asn Tyr Asp Pro Gly Ala Ser Phe Asp Val Asn
   165 170 175
   Asp Gln Asp Pro Asp Pro Gln Pro Arg Tyr Thr Gln Met Asn Asp Asn
   180 185 190
   Arg His Gly Thr Arg Cys Ala Gly Glu Val Ala Ala Val Ala Asn Asn
   195 200 205
   Gly Val Cys Gly Val Gly Val Ala Tyr Asn Ala Arg Ile Gly Gly Val
   210 215 220
   Arg Met Leu Asp Gly Glu Val Thr Asp Ala Val Glu Ala Arg Ser Leu
   225 230 235 240
   Gly Leu Asn Pro Asn His Ile His Ile Tyr Ser Ala Ser Trp Gly Pro
   245 250 255
   Glu Asp Asp Gly Lys Thr Val Asp Gly Pro Ala Arg Leu Ala Glu Glu
   260 265 270
   Ala Phe Phe Arg Gly Val Ser Gln Gly Arg Gly Gly Leu Gly Ser Ile
   275 280 285
   Phe Val Trp Ala Ser Gly Asn Gly Gly Arg Glu His Asp Ser Cys Asn
   290 295 300
   Cys Asp Gly Tyr Thr Asn Ser Ile Tyr Thr Leu Ser Ile Ser Ser Ala
   305                 310                 315                 320
   Thr Gln Phe Gly Asn Val Pro Trp Tyr Ser Glu Ala Cys Ser Ser Thr
                   325                 330                 335
   Leu Ala Thr Thr Tyr Ser Ser Gly Asn Gln Asn Glu Lys Gln Ile Val
               340                 345                 350
   Thr Thr Asp Leu Arg Gln Lys Cys Thr Glu Ser His Thr Gly Thr Ser
           355                 360                 365
   Ala Ser Ala Pro Leu Ala Ala Gly Ile Ile Ala Leu Thr Leu Glu Ala
       370                 375                 380
   Asn Lys Asn Leu Thr Trp Arg Asp Met Gln His Leu Val Val Gln Thr
   385                 390                 395                 400
   Ser Lys Pro Ala His Leu Asn Ala Asn Asp Trp Ala Thr Asn Gly Val
                   405                 410                 415
   Gly Arg Lys Val Ser His Ser Tyr Gly Tyr Gly Leu Leu Asp Ala Gly
               420                 425                 430
   Ala Met Val Ala Leu Ala Gln Asn Trp Thr Thr Val Ala Pro Gln Arg
           435                 440                 445
   Lys Cys Ile Ile Asp Ile Leu Thr Glu Pro Lys Asp Ile Gly Lys Arg
       450                 455                 460
   Leu Glu Val Arg Lys Thr Val Thr Ala Cys Leu Gly Glu Pro Asn His
   465                 470                 475                 480
   Ile Thr Arg Leu Glu His Ala Gln Ala Arg Leu Thr Leu Ser Tyr Asn
                   485                 490                 495
   Arg Arg Gly Asp Leu Ala Ile His Leu Val Ser Pro Met Gly Thr Arg
               500                 505                 510
   Ser Thr Leu Leu Ala Ala Arg Pro His Asp Tyr Ser Ala Asp Gly Phe
           515                 520                 525
   Asn Asp Trp Ala Phe Met Thr Thr His Ser Trp Asp Glu Asp Pro Ser
       530                 535                 540
   Gly Glu Trp Val Leu Glu Ile Glu Asn Thr Ser Glu Ala Asn Asn Tyr
   545                 550                 555                 560
   Gly Thr Leu Thr Lys Phe Thr Leu Val Leu Tyr Gly Thr Ala Pro Glu
                   565                 570                 575
   Gly Leu Pro Val Pro Pro Glu Ser Ser
               580                 585
(2) ANGABEN ZU SEQ ID NO: 15:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 15: CTAGAATTCT AACTGCTTTC TGGAGGTACG GGCAG 35
(2) ANGABEN ZU SEQ ID NO: 16:
   (i) SEQUENZKENNZEICHEN:
      (A) LÄNGE: 54 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 16:
      CTAGAATTCT TAGTGGTGAT GGTGATGATG ACTGCTTTCT GGAGGTACGG GCAG 54
(2) ANGABEN ZU SEQ ID NO: 17:
   (i) SEQUENZKENNZEICHEN:
      (A) LÄNGE: 1776 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 17:
   ATGGAGCTGA GGCCCTGGTT GCTATGGGTG GTAGCAGCAA CAGGAACCTT GGTCCTGCTA 60
   GCAGCTGATG CTCAGGGCCA GAAGGTCTTC ACCAACACGT GGGCTGTGCG CATCCCTGGA 120
   GGCCCAGCGG TGGCCAACAG TGTGGCACGG AAGCATGGGT TCCTCAACCT GGGCCAGATC 180
   TTCGGGGACT ATTACCACTT CTGGCATCGA GGAGTGACGA AGCGGTCCCT GTCGCCTCAC 240
   CGCCCGCGGC ACAGCCGGCT GCAGAGGGAG CCTCAAGTAC AGTGGCTGGA ACAGCAGGTG 300
   GCAAAGCGAC GGACTAAACG GGACGTGTAC CAGGAGCCCA CAGACCCCAA GTTTCCTCAG 360
   CAGTGGTACC TGTCTGGTGT CACTCAGCGG GACCTGAATG TGAAGGCGGC CTGGGCGCAG 420
   GGCTACACAG GGCACGGCAT TGTGGTCTCC ATTCTGGACG ATGGCATCGA GAAGAACCAC 480
   CCGGACTTGG CAGGCAATTA TGATCCTGGG GCCAGTTTTG ATGTCAATGA CCAGGACCCT    540
   GACCCCCAGC CTCGGTACAC ACAGATGAAT GACAACAGGC ACGGCACACG GTGTGCGGGG    600
   GAAGTGGCTG CGGTGGCCAA CAACGGTGTC TGTGGTGTAG GTGTGGCCTA CAACGCCCGC    660
   ATTGGAGGGG TGCGCATGCT GGATGGCGAG GTGACAGATG CAGTGGAGGC ACGCTCGCTG    720
   GGCCTGAACC CCAACCACAT CCACATCTAC AGTGCCAGCT GGGGCCCCGA GGATGACGGC    780
   AAGACAGTGG ATGGGCCAGC CCGCCTCGCC GAGGAGGCCT TCTTCCGTGG GGTTAGCCAG    840
   GGCCGAGGGG GGCTGGGCTC CATCTTTGTC TGGGCCTCGG GGAACGGGGG CCGGGAACAT    900
   GACAGCTGCA ACTGCGACGG CTACACCAAC AGTATCTACA CGCTGTCCAT CAGCAGCGCC    960
   ACGCAGTTTG GCAACGTGCC GTGGTACAGC GAGGCCTGCT CGTCCACACT GGCCACGACC   1020
   TACAGCAGTG GCAACCAGAA TGAGAAGCAG ATCGTGACGA CTGACTTGCG GCAGAAGTGC   1080
   ACGGAGTCTC ACACGGGCAC CTCAGCCTCT GCCCCCTTAG CAGCCGGCAT CATTGCTCTC   1140
   ACCCTGGAGG CCAATAAGAA CCTCACATGG CGGGACATGC AACACCTGGT GGTACAGACC   1200
   TCGAAGCCAG CCCACCTCAA TGCCAACGAC TGGGCCACCA ATGGTGTGGG CCGGAAAGTG   1260
   AGCCACTCAT ATGGCTACGG GCTTTTGGAC GCAGGCGCCA TGGTGGCCCT GGCCCAGAAT   1320
   TGGACCACAG TGGCCCCCCA GCGGAAGTGC ATCATCGACA TCCTCACCGA GCCCAAAGAC   1380
   ATCGGGAAAC GGCTCGAGGT GCGGAAGACC GTGACCGCGT GCCTGGGCGA GCCCAACCAC   1440
   ATCACTCGGC TGGAGCACGC TCAGGCGCGG CTCACCCTGT CCTATAATCG CCGTGGCGAC   1500
   CTGGCCATCC ACCTGGTCAG CCCCATGGGC ACCCGCTCCA CCCTGCTGGC AGCCAGGCCA   1560
   CATGACTACT CCGCAGATGG GTTTAATGAC TGGGCCTTCA TGACAACTCA TTCCTGGGAT   1620
   GAGGATCCCT CTGGCGAGTG GGTCCTAGAG ATTGAAAACA CCAGCGAAGC CAACAACTAT   1680
   GGGACGCTGA CCAAGTTCAC CCTCGTACTC TATGGCACCG CCCCTGAGGG GCTGCCCGTA   1740
   CCTCCAGAAA GCAGTCATCA TCACCATCAC CACTAA                             1776
(2) ANGABEN ZU SEQ ID NO: 18:
   (i) SEQUENZKENNZEICHEN:
      (A) LÄNGE: 591 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 18:
   Met Glu Leu Arg Pro Trp Leu Leu Trp Val Val Ala Ala Thr Gly Thr
   1 5 10 15
   Leu Val Leu Leu Ala Ala Asp Ala Gln Gly Gln Lys Val Phe Thr Asn
   20 25 30
   Thr Trp Ala Val Arg Ile Pro Gly Gly Pro Ala Val Ala Asn Ser Val
   35 40 45
   Ala Arg Lys His Gly Phe Leu Asn Leu Gly Gln Ile Phe Gly Asp Tyr
   50 55 60
   Tyr His Phe Trp His Arg Gly Val Thr Lys Arg Ser Leu Ser Pro His
   65 70 75 80
   Arg Pro Arg His Ser Arg Leu Gln Arg Glu Pro Gln Val Gln Trp Leu
   85 90 95
   Glu Gln Gln Val Ala Lys Arg Arg Thr Lys Arg Asp Val Tyr Gln Glu
   100 105 110
   Pro Thr Asp Pro Lys Phe Pro Gln Gln Trp Tyr Leu Ser Gly Val Thr
   115 120 125
   Gln Arg Asp Leu Asn Val Lys Ala Ala Trp Ala Gln Gly Tyr Thr Gly
   130 135 140
   His Gly Ile Val Val Ser Ile Leu Asp Asp Gly Ile Glu Lys Asn His
   145 150 155 160
   Pro Asp Leu Ala Gly Asn Tyr Asp Pro Gly Ala Ser Phe Asp Val Asn
   165 170 175
   Asp Gln Asp Pro Asp Pro Gln Pro Arg Tyr Thr Gln Met Asn Asp Asn
   180 185 190
   Arg His Gly Thr Arg Cys Ala Gly Glu Val Ala Ala Val Ala Asn Asn
   195 200 205
   Gly Val Cys Gly Val Gly Val Ala Tyr Asn Ala Arg Ile Gly Gly Val
   210 215 220
   Arg Met Leu Asp Gly Glu Val Thr Asp Ala Val Glu Ala Arg Ser Leu
   225 230 235 240
   Gly Leu Asn Pro Asn His Ile His Ile Tyr Ser Ala Ser Trp Gly Pro
   245 250 255
   Glu Asp Asp Gly Lys Thr Val Asp Gly Pro Ala Arg Leu Ala Glu Glu
   260 265 270
   Ala Phe Phe Arg Gly Val Ser Gln Gly Arg Gly Gly Leu Gly Ser Ile
   275 280 285
   Phe Val Trp Ala Ser Gly Asn Gly Gly Arg Glu His Asp Ser Cys Asn
   290 295 300
   Cys Asp Gly Tyr Thr Asn Ser Ile Tyr Thr Leu Ser Ile Ser Ser Ala
   305                 310                 315                 320
   Thr Gln Phe Gly Asn Val Pro Trp Tyr Ser Glu Ala Cys Ser Ser Thr
                   325                 330                 335
   Leu Ala Thr Thr Tyr Ser Ser Gly Asn Gln Asn Glu Lys Gln Ile Val
               340                 345                 350
   Thr Thr Asp Leu Arg Gln Lys Cys Thr Glu Ser His Thr Gly Thr Ser
           355                 360                 365
   Ala Ser Ala Pro Leu Ala Ala Gly Ile Ile Ala Leu Thr Leu Glu Ala
       370                 375                 380
   Asn Lys Asn Leu Thr Trp Arg Asp Met Gln His Leu Val Val Gln Thr
   385                 390                 395                 400
   Ser Lys Pro Ala His Leu Asn Ala Asn Asp Trp Ala Thr Asn Gly Val
                   405                 410                 415
   Gly Arg Lys Val Ser His Ser Tyr Gly Tyr Gly Leu Leu Asp Ala Gly
               420                 425                 430
   Ala Met Val Ala Leu Ala Gln Asn Trp Thr Thr Val Ala Pro Gln Arg
           435                 440                 445
   Lys Cys Ile Ile Asp Ile Leu Thr Glu Pro Lys Asp Ile Gly Lys Arg
       450                 455                 460
   Leu Glu Val Arg Lys Thr Val Thr Ala Cys Leu Gly Glu Pro Asn His
   465                 470                 475                 480
   Ile Thr Arg Leu Glu His Ala Gln Ala Arg Leu Thr Leu Ser Tyr Asn
                   485                 490                 495
   Arg Arg Gly Asp Leu Ala Ile His Leu Val Ser Pro Met Gly Thr Arg
               500                 505                 510
   Ser Thr Leu Leu Ala Ala Arg Pro His Asp Tyr Ser Ala Asp Gly Phe
           515                 520                 525
   Asn Asp Trp Ala Phe Met Thr Thr His Ser Trp Asp Glu Asp Pro Ser
       530                 535                 540
   Gly Glu Trp Val Leu Glu Ile Glu Asn Thr Ser Glu Ala Asn Asn Tyr
   545                 550                 555                 560
   Gly Thr Leu Thr Lys Phe Thr Leu Val Leu Tyr Gly Thr Ala Pro Glu
                   565                 570                 575
   Gly Leu Pro Val Pro Pro Glu Ser Ser His His His His His His
               580                 585                 590
(2) ANGABEN ZU SEQ ID NO: 19:
   (i) SEQUENZKENNZEICHEN:
      (A) LÄNGE: 72 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 19:
   CTAGAATTCT TAGTGGTGAT GGTGATGATG TGCAGCTCCA CCAGCTGCAC TGCTTTCTGG 60
   AGGTACGGGC AG 72
(2) ANGABEN ZU SEQ ID NO: 20:
   (i) SEQUENZKENNZEICHEN:
      (A) LÄNGE: 1794 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 20:
   ATGGAGCTGA GGCCCTGGTT GCTATGGGTG GTAGCAGCAA CAGGAACCTT GGTCCTGCTA 60
   GCAGCTGATG CTCAGGGCCA GAAGGTCTTC ACCAACACGT GGGCTGTGCG CATCCCTGGA 120
   GGCCCAGCGG TGGCCAACAG TGTGGCACGG AAGCATGGGT TCCTCAACCT GGGCCAGATC 180
   TTCGGGGACT ATTACCACTT CTGGCATCGA GGAGTGACGA AGCGGTCCCT GTCGCCTCAC 240
   CGCCCGCGGC ACAGCCGGCT GCAGAGGGAG CCTCAAGTAC AGTGGCTGGA ACAGCAGGTG 300
   GCAAAGCGAC GGACTAAACG GGACGTGTAC CAGGAGCCCA CAGACCCCAA GTTTCCTCAG 360
   CAGTGGTACC TGTCTGGTGT CACTCAGCGG GACCTGAATG TGAAGGCGGC CTGGGCGCAG 420
   GGCTACACAG GGCACGGCAT TGTGGTCTCC ATTCTGGACG ATGGCATCGA GAAGAACCAC 480
   CCGGACTTGG CAGGCAATTA TGATCCTGGG GCCAGTTTTG ATGTCAATGA CCAGGACCCT 540
   GACCCCCAGC CTCGGTACAC ACAGATGAAT GACAACAGGC ACGGCACACG GTGTGCGGGG 600
   GAAGTGGCTG CGGTGGCCAA CAACGGTGTC TGTGGTGTAG GTGTGGCCTA CAACGCCCGC 660
   ATTGGAGGGG TGCGCATGCT GGATGGCGAG GTGACAGATG CAGTGGAGGC ACGCTCGCTG 720
   GGCCTGAACC CCAACCACAT CCACATCTAC AGTGCCAGCT GGGGCCCCGA GGATGACGGC 780
   AAGACAGTGG ATGGGCCAGC CCGCCTCGCC GAGGAGGCCT TCTTCCGTGG GGTTAGCCAG 840
   GGCCGAGGGG GGCTGGGCTC CATCTTTGTC TGGGCCTCGG GGAACGGGGG CCGGGAACAT 900
   GACAGCTGCA ACTGCGACGG CTACACCAAC AGTATCTACA CGCTGTCCAT CAGCAGCGCC 960
   ACGCAGTTTG GCAACGTGCC GTGGTACAGC GAGGCCTGCT CGTCCACACT GGCCACGACC 1020
   TACAGCAGTG GCAACCAGAA TGAGAAGCAG ATCGTGACGA CTGACTTGCG GCAGAAGTGC 1080
   ACGGAGTCTC ACACGGGCAC CTCAGCCTCT GCCCCCTTAG CAGCCGGCAT CATTGCTCTC 1140
   ACCCTGGAGG CCAATAAGAA CCTCACATGG CGGGACATGC AACACCTGGT GGTACAGACC 1200
   TCGAAGCCAG CCCACCTCAA TGCCAACGAC TGGGCCACCA ATGGTGTGGG CCGGAAAGTG 1260
   AGCCACTCAT ATGGCTACGG GCTTTTGGAC GCAGGCGCCA TGGTGGCCCT GGCCCAGAAT 1320
   TGGACCACAG TGGCCCCCCA GCGGAAGTGC ATCATCGACA TCCTCACCGA GCCCAAAGAC 1380
   ATCGGGAAAC GGCTCGAGGT GCGGAAGACC GTGACCGCGT GCCTGGGCGA GCCCAACCAC 1440
   ATCACTCGGC TGGAGCACGC TCAGGCGCGG CTCACCCTGT CCTATAATCG CCGTGGCGAC 1500
   CTGGCCATCC ACCTGGTCAG CCCCATGGGC ACCCGCTCCA CCCTGCTGGC AGCCAGGCCA 1560
   CATGACTACT CCGCAGATGG GTTTAATGAC TGGGCCTTCA TGACAACTCA TTCCTGGGAT 1620
   GAGGATCCCT CTGGCGAGTG GGTCCTAGAG ATTGAAAACA CCAGCGAAGC CAACAACTAT 1680
   GGGACGCTGA CCAAGTTCAC CCTCGTACTC TATGGCACCG CCCCTGAGGG GCTGCCCGTA 1740
   CCTCCAGAAA GCAGTGCAGC TGGTGGAGCT GCACATCATC ACCATCACCA CTAA 1794
(2) ANGABEN ZU SEQ ID NO: 21:
   (i) SEQUENZKENNZEICHEN:
      (A) LÄNGE: 597 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 21:
   Met Glu Leu Arg Pro Trp Leu Leu Trp Val Val Ala Ala Thr Gly Thr
   1               5                   10                  15
   Leu Val Leu Leu Ala Ala Asp Ala Gln Gly Gln Lys Val Phe Thr Asn
               20                  25                  30
   Thr Trp Ala Val Arg Ile Pro Gly Gly Pro Ala Val Ala Asn Ser Val
           35                  40                  45
   Ala Arg Lys His Gly Phe Leu Asn Leu Gly Gln Ile Phe Gly Asp Tyr
       50                  55                  60
   Tyr His Phe Trp His Arg Gly Val Thr Lys Arg Ser Leu Ser Pro His
   65                  70                  75                  80
   Arg Pro Arg His Ser Arg Leu Gln Arg Glu Pro Gln Val Gln Trp Leu
   85 90 95
   Glu Gln Gln Val Ala Lys Arg Arg Thr Lys Arg Asp Val Tyr Gln Glu
   100 105 110
   Pro Thr Asp Pro Lys Phe Pro Gln Gln Trp Tyr Leu Ser Gly Val Thr
   115 120 125
   Gln Arg Asp Leu Asn Val Lys Ala Ala Trp Ala Gln Gly Tyr Thr Gly
   130 135 140
   His Gly Ile Val Val Ser Ile Leu Asp Asp Gly Ile Glu Lys Asn His
   145 150 155 160
   Pro Asp Leu Ala Gly Asn Tyr Asp Pro Gly Ala Ser Phe Asp Val Asn
   165 170 175
   Asp Gln Asp Pro Asp Pro Gln Pro Arg Tyr Thr Gln Met Asn Asp Asn
   180 185 190
   Arg His Gly Thr Arg Cys Ala Gly Glu Val Ala Ala Val Ala Asn Asn
   195 200 205
   Gly Val Cys Gly Val Gly Val Ala Tyr Asn Ala Arg Ile Gly Gly Val
   210 215 220
   Arg Met Leu Asp Gly Glu Val Thr Asp Ala Val Glu Ala Arg Ser Leu
   225 230 235 240
   Gly Leu Asn Pro Asn His Ile His Ile Tyr Ser Ala Ser Trp Gly Pro
   245 250 255
   Glu Asp Asp Gly Lys Thr Val Asp Gly Pro Ala Arg Leu Ala Glu Glu
   260 265 270
   Ala Phe Phe Arg Gly Val Ser Gln Gly Arg Gly Gly Leu Gly Ser Ile
   275 280 285
   Phe Val Trp Ala Ser Gly Asn Gly Gly Arg Glu His Asp Ser Cys Asn
   290 295 300
   Cys Asp Gly Tyr Thr Asn Ser Ile Tyr Thr Leu Ser Ile Ser Ser Ala
   305 310 315 320
   Thr Gln Phe Gly Asn Val Pro Trp Tyr Ser Glu Ala Cys Ser Ser Thr
   325 330 335
   Leu Ala Thr Thr Tyr Ser Ser Gly Asn Gln Asn Glu Lys Gln Ile Val
   340 345 350
   Thr Thr Asp Leu Arg Gln Lys Cys Thr Glu Ser His Thr Gly Thr Ser
   355 360 365
   Ala Ser Ala Pro Leu Ala Ala Gly Ile Ile Ala Leu Thr Leu Glu Ala
   370 375 380
   Asn Lys Asn Leu Thr Trp Arg Asp Met Gln His Leu Val Val Gln Thr
   385 390 395 400
   Ser Lys Pro Ala His Leu Asn Ala Asn Asp Trp Ala Thr Asn Gly Val
   405 410 415
   Gly Arg Lys Val Ser His Ser Tyr Gly Tyr Gly Leu Leu Asp Ala Gly
   420 425 430
   Ala Met Val Ala Leu Ala Gln Asn Trp Thr Thr Val Ala Pro Gln Arg
   435 440 445
   Lys Cys Ile Ile Asp Ile Leu Thr Glu Pro Lys Asp Ile Gly Lys Arg
   450 455 460
   Leu Glu Val Arg Lys Thr Val Thr Ala Cys Leu Gly Glu Pro Asn His
   465 470 475 480
   Ile Thr Arg Leu Glu His Ala Gln Ala Arg Leu Thr Leu Ser Tyr Asn
   485 490 495
   Arg Arg Gly Asp Leu Ala Ile His Leu Val Ser Pro Met Gly Thr Arg
   500 505 510
   Ser Thr Leu Leu Ala Ala Arg Pro His Asp Tyr Ser Ala Asp Gly Phe
   515 520 525
   Asn Asp Trp Ala Phe Met Thr Thr His Ser Trp Asp Glu Asp Pro Ser
   530 535 540
   Gly Glu Trp Val Leu Glu Ile Glu Asn Thr Ser Glu Ala Asn Asn Tyr
   545 550 555 560
   Gly Thr Leu Thr Lys Phe Thr Leu Val Leu Tyr Gly Thr Ala Pro Glu
   565 570 575
   Gly Leu Pro Val Pro Pro Glu Ser Ser Ala Ala Gly Gly Ala Ala His
   580 585 590
   His His His His His
   595
(2) ANGABEN ZU SEQ ID NO: 22:
   (i) SEQUENZKENNZEICHEN:
      (A) LÄNGE: 50 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 22:
      TGAGGGAGGT GGGGGAGGTC ATCACCACCA TCACCATCAT CATCACCATT 50
(2) ANGABEN ZU SEQ ID NO: 23:
   (i) SEQUENZKENNZEICHEN:
      (A) LÄNGE: 51 Basenpaare
      (B) ART: Nucleotid
      (C) STRANGFORM: Binzelstrang
      (D) TOPOLOGIE: linear
   (ii) ART DES MOLEKÜLS: Genom-DNA
   (xi) SEQUENZBESCHREIBUNG: SEQ ID NO: 23:
      AATTAATGGT GATGATGATG GTGATGGTGG TGATGACCTC CCCCACCTCC C 51
(2) ANGABEN ZU SEQ ID NO: 24:
   (i) SEQUENZKENNZEICHEN:
      (A) LÄNGE: 66 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 24:
   GGACCCCTCT GGCGAGTGGG TCCTCGAGAT TGAAAACACC AGCGAAGCCA ACAACTATGG 60
   GACGCT 66
(2) ANGABEN ZU SEQ ID NO: 25:
   (i) SEQUENZKENNZEICHEN:
      (A) LÄNGE: 69 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 25:
   TCAAGCGTCC CATAGTTGTT GGCTTCGCTG GTGTTTTCAA TCTCGAGGAC CCACTCGCCA 60
   GAGGGGTCC 69
(2) ANGABEN ZU SEQ ID NO: 26:
   (i) SEQUENZKENNZEICHEN:
      (A) LÄNGE: 69 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 26:
   TCAAGCGTCC CATAGTTGTT GGCTTCGCTG GTGTTTTCAA TCTCGAGGAC CCACTCGCCA 60
   GAGGGGTCC 69
(2) ANGABEN ZU SEQ ID NO: 27:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 27:
      ATTACAATTG CTGCAGGGAT CCAC 24